# EUROPEAN PATENT SPECIFICATION

(11) **EP 3 013 945 B1**
(45) Date of publication and mention of the grant of the patent: **27.05.2020**
(21) Application number: 14818265.2
(22) Date of filing: 25.06.2014
(51) Int. Cl.: C12N 5/0775, A61K 35/28, A61P 9/00

(54) **CORTICAL BONE-DERIVED STEM CELLS**
AUS KORTIKALEM KNOCHEN GEWONNENE STAMMZELLEN
CELLULES SOUCHES DÉRIVÉES D'OS CORTICAL

(30) Priority: 25.06.2013 US 201361839168 P
(43) Date of publication of application: 04.05.2016
(73) Proprietor: Temple University Of The Commonwealth System Of Higher Education, Philadelphia, PA 19122 (US)
(72) Inventor: HOUSER, Steven R., Fort Washington, PA 19034 (US); KUBO, Hajime, North Wales, PA 19454 (US); DURAN, Jason Mathew, Philadelphia, PA 19147 (US); SHARP, Thomas E., Toms River, NJ 08753 (US)
(74) Representative: Schiweck Weinzierl Koch Patentanwälte Partnerschaft mbB
(86) International application number: PCT/US2014/044077
(87) International publication number: WO 2014/210142

(56) References cited:
- SUNG J H ET AL: "Isolation and Characterization of Mouse Mesenchymal Stem Cells", TRANSPLANTATION PROCEEDINGS, ELSEVIER INC, ORLANDO, FL; US, vol. 40, no. 8, 1 October 2008 (2008-10-01), pages 2649-2654, XP025841375, ISSN: 0041-1345, DOI: 10.1016/J.TRANSPROCEED.2008.08.009 [retrieved on 2008-10-14]
- ZHU ET AL.: 'A protocol for isolation and culture of mesenchymal stem cells from mouse compact bone' NATURE PROTOCOLS vol. 5, no. 3, 01 March 2010, pages 550 - 560, XP009172805
- ORLIC ET AL.: 'Bone marrow cells regenerate infarcted myocardium' NATURE vol. 410, 05 April 2010, pages 701 - 705, XP002965971
- MAFI ET AL.: 'Adult mesenchymal stem cells and cell surface characterization - a systematic review of the literature' THE OPEN ORTHOPAEDICS JOURNAL vol. 5, no. 1, 28 July 2011, pages 253 - 260, XP055026032
- FAZEL ET AL.: 'Cardioprotective c-kit+ cells are from the bone marrow and regulate the myocardial balance of angiogenic cytokines' THE JOURNAL OF CLINICAL INVESTIGATION vol. 116, no. 7, 01 July 2006, pages 1865 - 1877, XP055302853
- JIANG ET AL.: 'Pluripotency of mesenchymal stem cells derived from adult marrow' NATURE vol. 418, no. 6893, 04 July 2002, pages 41 - 49, XP002559664
- GNECCHI ET AL.: 'Evidence supporting paracrine hypothesis for Akt-modified mesenchymal stem cell -mediated cardiac protection and functional improvement' FASEB J. vol. 20, 01 April 2006, pages 661 - 669, XP002514370
- FAZEL ET AL.: 'Current status of cellular therapy for ischemic heart disease' ANN. THORAC. SURG. vol. 79, no. 6, 01 June 2005, pages S2238 - S2247, XP055302856
- FUKATA ET AL.: 'Contribution of Bone Marrow-Derived Hematopoietic Stem/Progenitor Cells to the Generation of Donor-Marker+ Cardiomyocytes In Vivo' PLOS ONE vol. 8, no. 5, 07 May 2013, pages 1 - 10, XP055302858
- DURAN ET AL.: 'Bone-Derived Stem Cells Repair the Heart After Myocardial Infarction Through Transdifferentiation and Paracrine Signaling Mechanisms' CIRCULATION RESEARCH vol. 113, no. 5, 25 June 2013, pages 539 - 552, XP055302859
- ONG ET AL.: 'Cortical Bone-Derived Stem Cells: A Novel Class of Cells for Myocardial Protection' CIRCULATION RESEARCH vol. 113, no. 5, 16 August 2013, pages 480 - 483, XP055302860

## Description

### CROSS-REFERENCE TO RELATED APPLICATION

This application claims priority to U.S. Provisional Application Serial No. 61/839,168, filed June 25, 2013.

### BACKGROUND OF THE INVENTION

Ischemic injury of the heart, including myocardial infarction (MI), is a major health problem that leads to structural and functional remodeling (Pfeffer et al., 1990, Circulation 81:1161-1172) and often culminates in heart failure (Gomez et al., 2001, Circulation 104:688-693). Novel therapies to repair or replace damaged cardiac tissue are needed to improve the prognosis of MI patients. Stem cell therapy has the potential to repair hearts after ischemic injury. A variety of adult stem cell types that might repair the injured heart have been tested in animal models. These studies have shown that transplantation of autologous cardiac (Beltrami et al., 2003, Cell 114:763-776; Tang et al., 2010, Circulation 121:293-305; Rota et al., 2008, Circ Res 103:107-116; Smith et al., 2007, Circulation 115:896-908) or bone marrow-derived (Orlic et al., 2001, Nature 410:701-705; Rota et al., 2007, Proc Natl Acad Sci USA 104:17783-17788) stem cells, induced pluripotent stem cells (Mauritz et al., 2008, Circulation 118:507-517; Zhang et al., 2009, Circ Res 104:e30-41; Zwi et al., 2009, Circulation 120:1513-1523), and direct reprogramming of endogenous non-stem cells into cardiogenic phenotypes (Qian et al., 2012, Nature 485:593-598; Song et al, 2012, Nature 485:599-604) can improve cardiac function after injury. Early stage clinical trials have largely focused on autologous stem cells due to their ease of isolation and lack of immunogenicity. These trials have demonstrated the ability of both bone marrow (Schachinger et al., 2006, N Engl J Med 355:1210-1221; Yousef et al., 2009, J Am Coll Cardiol 53:2262-2269; Chen et al., 2004, Am J Cardiol 94:92-95) and cardiac-derived (Bolli et al., 2011, Lancet 378:1847-1857; Chugh et al., 2012, Circulation 126:S54-64; Makkar et al., 2012, Lancet 379:895-904) cells to offer moderate functional benefits when transplanted after cardiac injury. While the outcomes of these trials continue to improve, the overall beneficial effects of autologous stem cell therapies are still relatively modest and the fundamental mechanisms of stem cell mediated repair are largely unknown and controversial.

Sung et al., 2008, Transplantation Proceedings 40(8):2649-2654 disclose the isolation and characterization of mouse mesenchymal stem cells. Fazel et al., 2006, J Clin Invest 116(7):1865-1877 disclose that cardioprotective c-kit⁺ cells are from the bone marrow and regulate the myocardial balance of angiogenic cytokines. Further, Fazel et al., 2005, Ann. Thorac. Surg. 79(6):S2238-S2247 disclose the current status of cellular therapy for ischemic heart disease.

A consensus regarding stem cell repair mechanisms is lacking, most cardiac stem cell researchers currently believe that bone marrow-derived stem cells act primarily through paracrine stimulation of new blood vessel formation or through differentiation into vascular cells rather than transforming to myocytes, while cardiac-derived stem cells (CDCs) may differentiate into adult myocytes (Segers and Lee, 2008, Nature 451:937-942). Although some studies have demonstrated that bone marrow-derived stem cells can regenerate into adult myocytes (Orlic et al., 2001, Nature 410:701-705; Rota et al., 2007, Proc Natl Acad Sci USA 104:17783-17788), others have suggested that these new myocytes are mostly formed through stimulation of endogenous cardiac stem cells rather than through direct differentiation of the transplanted cells (Williams et al., 2011, Circ Res 109:923-940; Hatzistergos et al., 2010, Circ Res 107:913-922).

The mechanisms of stem cell-mediated cardiac repair are unknown. Many preclinical studies in animal models have shown that differentiation (Smith et al., 2007, Circulation 115:896-908; Orlic et al., 2001, Nature 410:701-705; Rota et al., 2007, Proc Natl Acad Sci USA 104:17783-17788) of injected cells into new cardiac myocytes is one potential mechanism for this repair. Secretion of paracrine factors that enhance cardioprotection of the endogenous myocardium, neovascularization, and recruitment of endogenous stem cells that promote repair are other major mechanisms (Tang et al., 2010, Circulation 121:293-305; Rota et al., 2008, Circ Res 103:107-116; Zeng et al., 2007, Circulation 115:1866-1875; Li et al., 2012, J Am Coll Cardiol 59:942-953). Well-characterized sources of stem cells shown to enhance cardiac repair of the diseased heart should lead to effective cell therapies. Furthermore, certain stem cell types may have a greater capacity to transdifferentiate, while others may produce more paracrine factors and have a greater potential to stimulate neovascularization and other endogenous repair mechanisms.

Thus, there is a need in the art for compositions and methods for using stem cells to repair cardiac tissue. The present invention satisfies this unmet need.

### SUMMARY OF THE INVENTION

The invention is defined by the appended claims and the following embodiments.

The invention provides an isolated cortical bone-derived stem cell (CBSC) capable of differentiating into a cardiomyocyte, wherein the cell expresses CD29, Sca-1, CD105, CD44, CD106, CD73, CD271, and CD90.

In one embodiment, the cell expresses c-kit (CD117), Sca-1, and β₁-Integrin (CD29) at the time of cell isolation.

In one embodiment, the cell does not express a hematopoietic biomarker protein or a nucleic acid encoding the biomarker protein.

In one embodiment, the hematopoietic biomarker is selected from the group consisting of CD34, CD45, CD5, CD11b, CD45R, antigen 7-4, Gr-1, Ly6G/C, Terr-119, and any combination thereof.

In one embodiment, the cell is pluripotent. In one embodiment, the cell retains the ability to differentiate into a germ layer selected from the group consisting of mesoderm, ectoderm, endoderm, and any combination thereof.

In one embodiment, the cell differentiates into cardiomyocytes and secretes a pro-angiogenic paracrine factor when the cells are administered to the heart.

The invention provides a cortical bone-derived stem cell (CBSC) for use in a method of treating a cardiovascular disease in a subject, the use comprising administering to a subject in need thereof an effective amount of cortical bone-derived stem cells (CBSCs), wherein the cell expresses CD29, Sca-1, CD105, CD44, CD106, CD73, CD271, and CD90.

In one embodiment, the cardiovascular disease is myocardial injury. In one embodiment, the myocardial injury comprises at least one of arterial disease, atheroma, atherosclerosis, arteriosclerosis, coronary artery disease, arrhythmia, angina pectoris, congestive heart disease, ischemic cardiomyopathy, myocardial infarction, stroke, transient ischemic attack, aortic aneurysm, cardiopericarditis, infection, inflammation, valvular insufficiency, vascular clotting defects, and combinations thereof.

In one embodiment, the CBSCs are treated prior to administration to the subject. In one embodiment, the CBSCs are treated during or after administration to the subject. In one embodiment, the CBSCs are administered to the subject by at least one of direct injection, venous infusion, and arterial infusion.

In one embodiment, the CBSCs differentiate into cardiac myocytes in the subject. In one embodiment, the CBSCs secrete a pro-angiogenic paracrine factor in the subject. In one embodiment, the pro-angiogenic paracrine factor is selected from the group consisting of bFGF, VEGF, and a combination thereof.

In one embodiment, the method further comprises modifying the CBSCs by gene transfer.

In one embodiment the subject is a human.

### BRIEF DESCRIPTION OF THE DRAWINGS

The following detailed description of preferred embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities of the embodiments shown in the drawings.

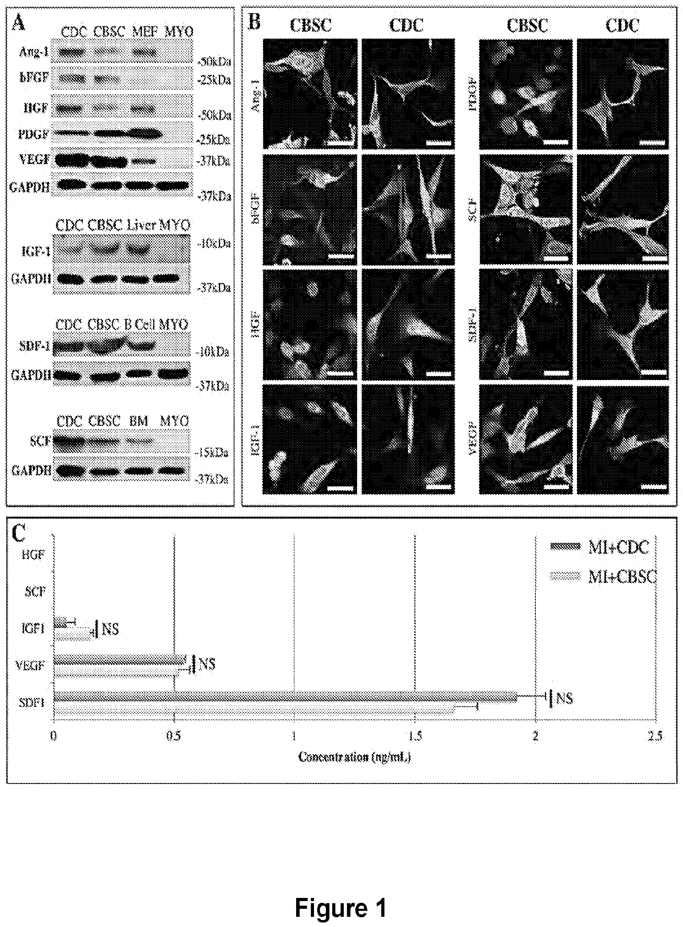

Figure 1, comprising Figure 1A through Figure 1C, depicts the results of experiments demonstrating the *in vitro* characterization of stem cells. Figure 1A: CBSC or CDC lysates were analyzed by Western analysis. Positive controls include mouse endothelial fibroblasts (MEF), liver, bone marrow (BM), and B lymphocytes (B Cell). Myocyte (MYO) lysates were used as negative controls for all samples. Figure 1B: CBSCs were fixed *in vitro* and immunostained against each paracrine factor. Nuclei are labeled with DAPI and scale bars = 20 µm. Figure 1C: CBSCs or CDCs were allowed to proliferate over 72 hours and their culture media was analyzed by ELISA for the presence of soluble HGF, IGF, SCF, SDF-1, and VEGF. Samples were analyzed in triplicate and background signal was subtracted using unconditioned media blanks. NS = No Significant difference (p > 0.05).
Figure 2 is a graph depicting the results of experiments demonstrating the six-week survival of treated and untreated mice. Mice underwent sham, MI+Saline, MI+CDC or MI+CBSC surgery. Data was analyzed using a Kaplan-Meier regression and significance was determined using the Log-Rank test.
Figure 3, comprising Figure 3A and Figure 3B, depicts the results of experiments investigating cardiac function measured by echocardiography. Animals underwent sham, MI+Saline, MI+CBSC or MI+CDC surgeries and received follow-up serial echocardiography at 1, 2, 4 and 6 weeks post-MI. Figure 3A: Representative M-mode tracings from animals at baseline, 6 weeks post-MI+CBSC, 6 weeks post-MI+CDC, or 6 weeks post-MI+Saline. Figure 3B: Structural and functional parameters derived from echocardiography measurements are shown. */**/***= CBSC v. Saline, */**/*** = CBSC v. CDC
Figure 4, comprising Figure 4A through Figure 4C, depicts the results of experiments investigating left ventricular endomyocardial strain. Figure 4A: Three-dimensional regional wall velocity diagrams showing contraction (positive values) or relaxation (negative values) of 3 consecutive cardiac cycles. Figure 4B: Vector diagrams showing the direction and magnitude of endocardial contraction at mid-systole. Figure 4C: Global averages of strain and strain rate measured in the radial or longitudinal axes across the LV endocardium. */**/***= CBSC v. Saline, */**/*** = CBSC v. CDC
Figure 5 is a set of images demonstrating the characterization of paracrine factors secreted by stem cells after 24 hours post-MI *in vivo.* Animals receiving MI+CBSCs or MI+CDCs were sacrificed 24 hours post-MI for analysis of paracrine factor production. EGFP+ CBSCs stained positive for bFGF, and VEGF, but negative for HGF, IGF-1, PDGF, SCF, and SDF-1. EGFP+ CDCs stained positive for Ang-1 in addition to bFGF and VEGF, but also stained negative for HGF, IGF-1, PDGF, SCF, and SDF-1. Nuclei are labeled with DAPI and injected CBSCs are shaded.
Figure 6 is a set of images demonstrating that expansion of EGFP+ CBSCs as stem cells engraft, align, and elongate over 6 weeks. Samples were immunostained against α-sarcomeric actin, nuclei are labeled with DAPI and injected EGPF+ CBSCs are shaded.
Figure 7, comprising Figure 7A through Figure 7D are a set of images demonstrating that cortical bone stem cells grow and differentiate over 6 weeks. Figure 7A and Figure 7B: 1 week post-MI (Figure 7A) and 2 weeks post-MI (Figure 7B) samples were immunostained for α-sarcomeric actin and EGFP. 6 weeks post-MI samples were immunostained for (Figure 7C) α-sarcomeric actin (white), connexin43 and EGFP or (Figure 7D) α-smooth muscle actin (white), von Willebrand Factor and EGFP. Nuclei were labeled with DAPI in all images.

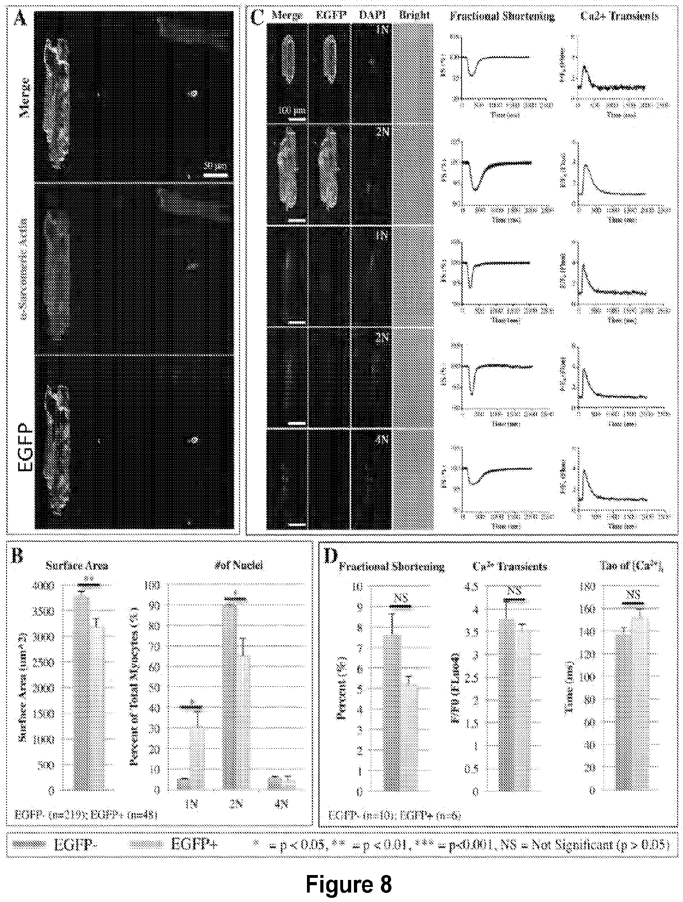

Figure 8, comprising Figure 8A through Figure 8D, depict the results of experiments where isolated myocyte size, number of nuclei, and cell physiology were analyzed 6 weeks post-MI+CBSC. Figure 8A: Myocytes were immunostained against α-sarcomeric actin, EGFP, and DAPI. Figure 8B: Average surface area of EGFP+ versus EGFP- myocytes and percent of total EGFP+ or EGFP- cells that were mono-, bi-, or tetranucleated. Figure 8C: Representative fractional shortening and Ca²⁺ transients of EGFP+ myocytes with 1 or 2 nuclei, or EGFP- myocytes with 1, 2, or 4 nuclei. Scale bars = 100 µm. Figure 8D: Mean fractional shortening, peak Ca²⁺ F/F0, and the time constant of decay (τ) of Ca²⁺ transients for all EGFP+ versus EGFP- myocytes.
Figure 9 is a set of graphs depicting the characterization of stem cells using quantitative real-time PCR (qPCR). Detected levels of c-kit and Sca-1 mRNA from cortical bone stem cells (CBSCs) or cardiac-derived stem cells (CDCs) were normalized to levels of GAPDH mRNA transcripts expressed by each cell type. Results are presented as normalized arbitrary units.
Figure 10, comprising Figure 10A and Figure 10B, is a set of images depicting the characterization of stem cells by immunostaining. CBSCs or CDCs were stained for c-kit (Figure 10A) or Sca-1 (Figure 10B) and nuclei were labeled with DAPI. Isotype controls are also shown (goat IgG for c-kit or rat IgG for Sca-1). Scale bars = 50 µm.
Figure 11 depicts the characterization of stem cells using flow cytometry. Flow cytometry against c-kit, Sca-1, CD29, CD34, CD45 and lineage markers (Lin). Results are shown along with negative isotype controls in which cells were labeled only with APC-conjugated Rat IgG2A.
Figure 12, comprising Figure 12A through Figure 12D, is a set of images demonstrating that bone-derived stem cells differentiate *in vitro.* CBSCs were co-cultured with neonatal rat ventricular myocytes for 3 days. Cells were fixed and stained for α-sarcomeric actin (Figure 12A and Figure 12B) or connexin43 (Figure 12C and Figure 12D). Nuclei are labeled with DAPI. EGFP+ CBSCs are shaded.

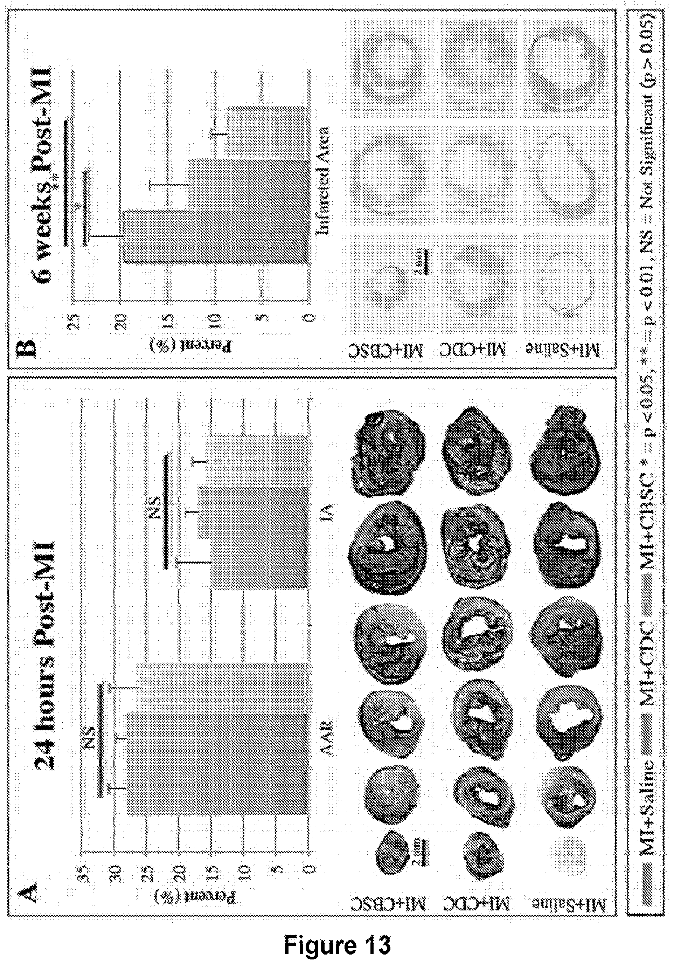

Figure 13, comprising Figure 13A and Figure 13B, depicts the results of infarct size analysis. Figure 13A: Acute infarct size analysis was performed on animals receiving MI+Saline (n=5), MI+CDC (n=5), or MI+CBSC (n=5) that were sacrificed 24 hours post-MI. Their area at risk (AAR) or infarct area (IA) was determined using Evan's Blue or triphenyltetrazolium chloride staining, respectively, and results are reported as a percentage of total ventricular area. Figure 13B: Chronic infarct size was determined by staining short-axis cross-sections from hearts fixed at 6 weeks post-MI+Saline (n=6), MI+CDC (n=6), or MI+CBSC (n=5) with hematoxylin and eosin (H&E) and measuring the infarct area as a percent of total myocardial surface area.

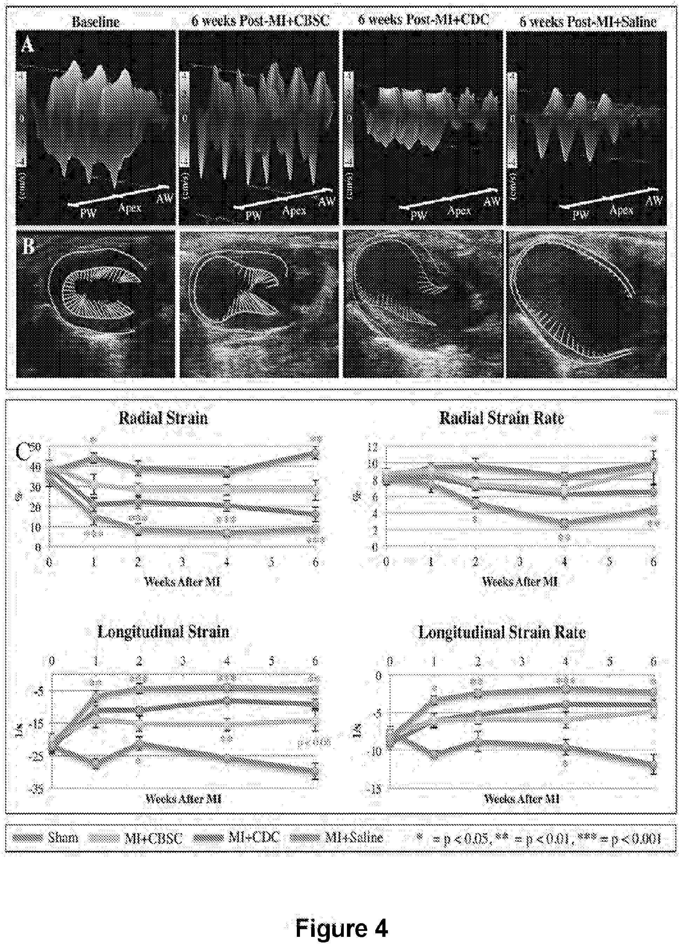

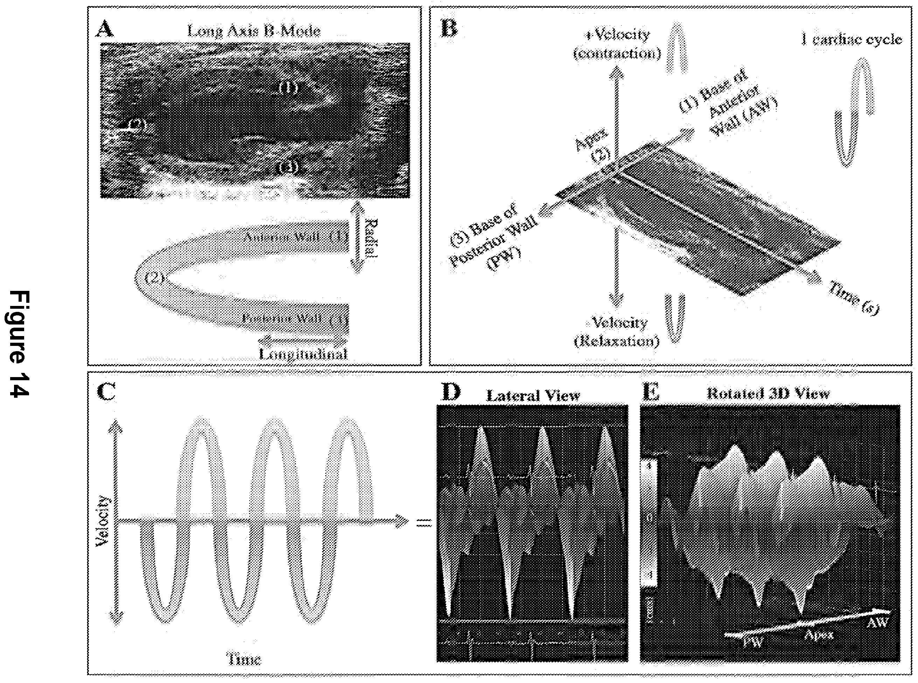

Figure 14, comprising Figure 14A through Figure 14E, depict a set of diagrams of strain analysis measurements. Figure 14A: Schematic showing the measurement parameters used for strain analysis on B-mode images taken in the parasternal long axis. A sample B-mode tracing of a mouse heart at baseline is included on top. Figure 14B: Diagram explaining the three axes used to generate the 3D wall velocity diagrams. The X-axis represents the location along the LV endocardial surface from the base of the anterior wall (1) to the apex (2) to the base of the posterior wall (3). The Z-axis represents time (in seconds), which is measured off of the electrocardiogram. The Y-axis represents contraction (positive values) or relaxation (negative values). An example of a positive wall velocity tracing and a negative wall velocity tracing are shown next to the positive and negative Y-axes. Figure 14C: Diagram demonstrating how the 3D wall velocity diagram is constructed. A single cardiac cycle is shown, and below a cartoon illustrating 3 consecutive cardiac cycles is shown. This is equivalent to what is seen in the lateral view of the wall velocity diagram (Figure 14D). Figure 14E: Shows the rotated view of the 3D wall velocity diagram from the animal at baseline that was shown in Figure 4.

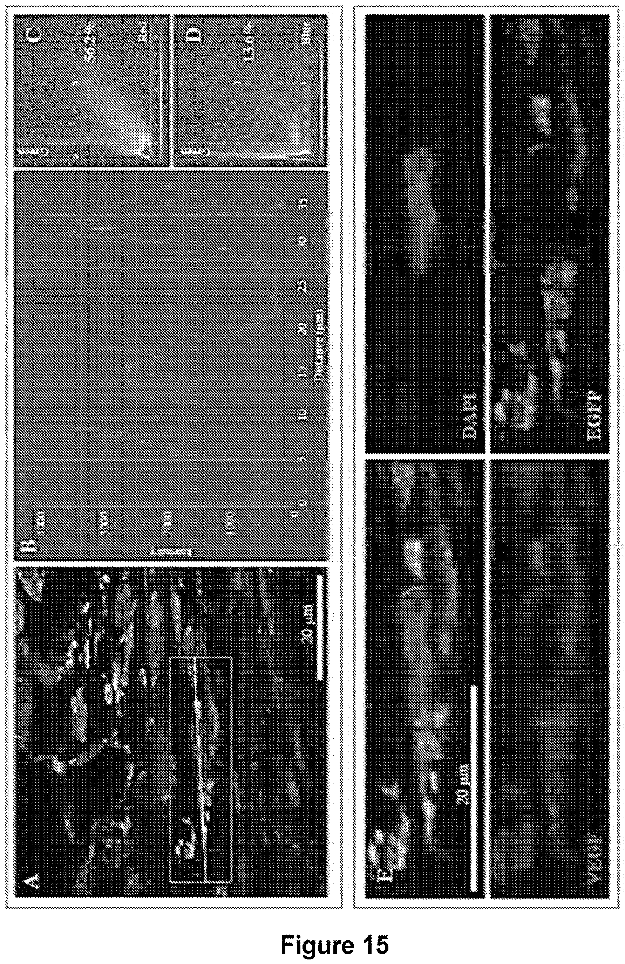

Figure 15, comprising Figure 15A through Figure 15E, depicts the characterization of paracrine factors secreted by cortical bone stem cells *in vivo 2* weeks after MI. Animals receiving MI+CBSCs were sacrificed 2 weeks post-MI and EGFP+ CBSC injection sites were identified and immunostained for VEGF. Nuclei are labeled with DAPI. Figure 15A: Immunostain showing an EGFP+/VEGF+ cell that was selected for fluorophore colocalization analysis by confocal line scan. Figure 15B: Intensity of phlorophores across the line scan of the cell selected in Figure 15A. From these data, scatterplots were constructed depicting colocalization of the different channels (Figure 15C and 15D; control). Figure 15E: Magnified image depicting the cell in Figure 15A along with single color channel images.
Figure 16 depicts the results of experiments demonstrating that CBSC-treated animals have increased von Willebrand Factor+ blood vessels near the infarct border zone by 6 weeks post-MI. Slides from animals receiving MI+Saline, MI+CDC or MI+CBSC injection were stained for von Willebrand factor (purple) and α-sarcomeric actin, and the number of von Willebrand Factor+ blood vessels observed per visual field were quantified. Nuclei are labeled with DAPI. Both a merged image and a single color image showing only the von Willebrand factor channel are shown for each group. ** = p < 0.001
Figure 17 depicts individual channel images and staining controls from Figure 7A and B. Scale bars = 50 µm.
Figure 18 depicts low magnification images showing EGFP+ and EGFP- regions of myocardium. Scale bars = 20 µm.
Figure 19 depicts low magnification images showing EGFP+ and EGFP- regions around vasculature. Scale bars = 10 µm.
Figure 20, comprising Figure 20A and Figure 20B, depicts individual color channel images and staining controls for Figure 7C. Scale bars = 20 µm.
Figure 21, comprising Figure 21A and Figure 21B, depicts individual color channel images and staining controls for Figure 7D. Scale bars = 10 µm.
Figure 22, comprising Figure 22A and Figure 22B, depict results of experiments demonstrating that cardiac stem cells grow and expand but do not adopt a myocyte or vascular phenotype within 6 weeks. Injection sites from MI animals receiving CDC treatment were sacrificed at 1, 2, or 6 weeks and were stained for α-sarcomeric actin and EGFP (Figure 22A), or connexin43, EGFP and α-sarcomeric actin (white) (Figure 22B). Nuclei are labeled with DAPI.
Figure 23 depicts the results of experiments demonstrating the proportion of CBSCs expressing α-sarcomeric actin after 6 weeks post-MI. EGFP+ injection sites were identified in 3 animals sacrificed 6 weeks after MI+CBSC injection, and these tissues were stained for α-sarcomeric actin (a-SA). Cells expressing EGFP (n=265) were analyzed at high magnification for expression of unorganized or striated α-SA, and their numbers are expressed as percentages in the pie chart on the left.
Figure 24 is a series of images summarizing the morphometric differences between CBSCs, CDCs and MSCs.
Figure 25 is a graph proliferation differences between CBSCs, CDCs and MSCs.
Figure 26 is a chart showing gene expression profiling of CBSCs, CDCs and MSCs from pigs.
Figure 27 is a chart showing the summary of gene expression profiling of CBSCs isolated from mice and pigs.
Figure 28 is an image showing induction of ischemia-reperfusion myocardial infarction (MI).
Figure 29 is an image showing baseline electromechanical assessment.
Figure 30 is an image showing CBSC injection post-MI with the NOGA Myostar injection catheter.
Figure 31 is an image showing post MI + CBSC's electromechanial assessment.
Figure 32 is an image showing one month post MI + CBSC's electromechanial assessment.

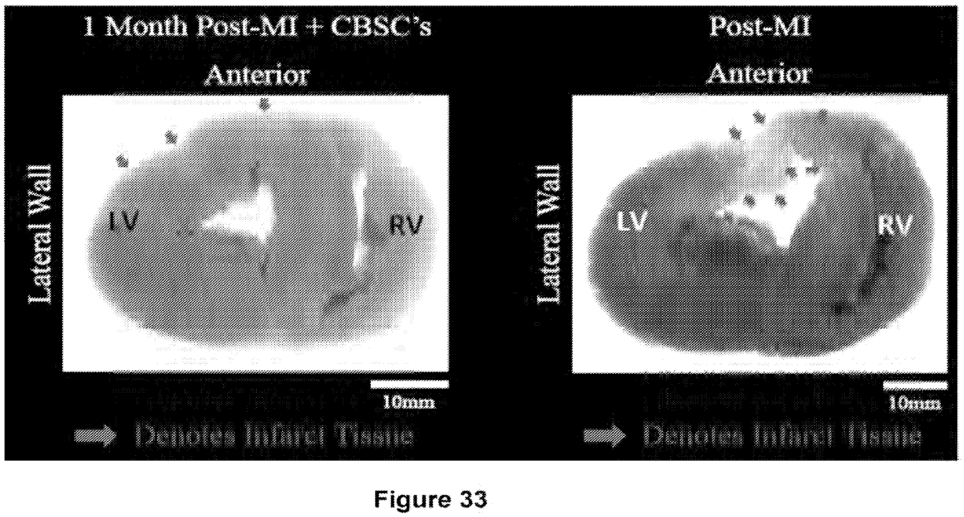

Figure 33 is an image showing gross anatomy cross-section MI + swine CBSC's versus control MI.

### DETAILED DESCRIPTION

The present invention provides isolated cortical bone-derived stem cells (CBSCs , wherein the cell expresses CD29, Sca-1, CD105, CD44, CD106, CD73, CD271, and CD90. In one embodiment, the cells of the invention are pluripotent. In another embodiment, the cells of the invention are capable of differentiating into cardiac myocytes. The present invention also includes the CBSCs of the invention for use in the treatment of heart disease.

The invention is based partly on the discovery that CBSCs injected into the border zone of an induced myocardial infarction (MI) resulted in significant improvements in cardiac structure, function and survival. CBSCs enhanced angiogenesis in the MI border zone after MI by secreting factors that trigger endogenous blood vessel formation. In addition, CBSCs injected into the MI border zone differentiated into new, functionally mature heart muscle cells that enhanced cardiac function in the regions of cell injection.

In one embodiment, the invention provides a novel population of stem cells derived from cortical bone (e.g., CBSCs) whereby the cells are c-kit+ and Sca1+ but did not express hematopoietic lineage markers. In one embodiment, expression of c-kit by CBSCs is decreased following subsequent culturing. However, CBSCs continue to express all of the other signature markers from early to late passage including CD29, Sca-1, CD105, CD106, CD73, CD44, CD271 and CD90. CBSCs remain negative for hematopoietic lineages markers including CD45, CD11b.

In another embodiment, the CBSCs of the invention can survive when injected into the ischemic heart and have the potential to differentiate into cardiac myocytes with mature function and to secrete factors that promote endogenous repair.

### Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

As used herein, each of the following terms has the meaning associated with it in this section.
The articles "a" and "an" are used herein to refer to one or to more than one (*i.e*., to at least one) of the grammatical object of the article. By way of example, "an element" means one element or more than one element.
"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.
The term "abnormal" when used in the context of organisms, tissues, cells or components thereof, refers to those organisms, tissues, cells or components thereof that differ in at least one observable or detectable characteristic (e.g., age, treatment, time of day) from those organisms, tissues, cells or components thereof that display the "normal" (expected) respective characteristic. Characteristics which are normal or expected for one cell or tissue type, might be abnormal for a different cell or tissue type.
"Cardiomyocyte" refers to cells that comprise the heart and are also known as cardiac muscle cells. A "myoblast" is a mononucleated, undifferentiated muscle precursor cell.
As used herein, the phrase "cardiovascular condition, disease or disorder" is intended to include all disorders characterized by insufficient, undesired or abnormal cardiac function, e.g., ischemic heart disease, hypertensive heart disease and pulmonary hypertensive heart disease, valvular disease, congenital heart disease and any condition which leads to congestive heart failure in a subject, particularly a human subject. Insufficient or abnormal cardiac function can be the result of disease, injury and/or aging. By way of background, a response to myocardial injury follows a well-defined path in which some cells die while others enter a state of hibernation where they are not yet dead but are dysfunctional. This is followed by infiltration of inflammatory cells, deposition of collagen as part of scarring, all of which happen in parallel with in-growth of new blood vessels and a degree of continued cell death. As used herein, the term "ischemia" refers to any localized tissue ischemia due to reduction of the inflow of blood. The term "myocardial ischemia" refers to circulatory disturbances caused by coronary atherosclerosis and/or inadequate oxygen supply to the myocardium. For example, an acute myocardial infarction represents an irreversible ischemic insult to myocardial tissue. This insult results from an occlusive (e.g., thrombotic or embolic) event in the coronary circulation and produces an environment in which the myocardial metabolic demands exceed the supply of oxygen to the myocardial tissue.
The terms "cells" and "population of cells" are used interchangeably and refer to a plurality of cells, i.e., more than one cell. The population may be a pure population comprising one cell type. Alternatively, the population may comprise more than one cell type. In the present invention, there is no limit on the number of cell types that a cell population may comprise.
As used herein, "a cell that differentiates into a mesodermal (or ectodermal or endodermal) lineage" defines a cell that becomes committed to a specific mesodermal, ectodermal or endodermal lineage, respectively. Examples of cells that differentiate into a mesodermal lineage or give rise to specific mesodermal cells include cells that are adipogenic, chondrogenic, cardiogenic, dermatogenic, hematopoetic, hemangiogenic, myogenic, nephrogenic, urogenitogenic, osteogenic, pericardiogenic, or stromal. Examples of cells that differentiate into ectodermal lineage include epidermal cells, neurogenic cells, and neurogliagenic cells. Examples of cells that differentiate into endodermal lineage include pleurigenic cells, and hepatogenic cells, cell that give rise to the lining of the intestine, and cells that give rise to pancreogenic and splanchogenic cells.
As used herein "conditioned media" defines a medium in which a specific cell or population of cells have been cultured in, and then removed. While the cells were cultured in said medium, they secrete cellular factors that include hormones, cytokines, extracellular matrix (ECM), proteins, vesicles, antibodies, and granules. The medium plus the cellular factors is the conditioned medium.
The term "dedifferentiation", as used herein, refers to the return of a cell to a less specialized state. After dedifferentiation, such a cell will have the capacity to differentiate into more or different cell types than was possible prior to re-programming. The process of reverse differentiation (i.e., de-differentiation) is likely more complicated than differentiation and requires "re-programming" the cell to become more primitive.
"Differentiated" is used herein to refer to a cell that has achieved a terminal state of maturation such that the cell has developed fully and demonstrates biological specialization and/or adaptation to a specific environment and/or function. Typically, a differentiated cell is characterized by expression of genes that encode differentiation associated proteins in that cell. When a cell is said to be "differentiating," as that term is used herein, the cell is in the process of being differentiated.
"Differentiation medium" is used herein to refer to a cell growth medium comprising an additive or a lack of an additive such that a stem cell, adipose derived adult stromal cell or other such progenitor cell, that is not fully differentiated when incubated in the medium, develops into a cell with some or all of the characteristics of a differentiated cell.
The term "derived from" is used herein to mean to originate from a specified source.
A "disease" is a state of health of an animal wherein the animal cannot maintain homeostasis, and wherein if the disease is not ameliorated then the animal's health continues to deteriorate.
In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.
A disease or disorder is "alleviated" if the severity of a symptom of the disease or disorder, the frequency with which such a symptom is experienced by a patient, or both, is reduced.
"Expandability" is used herein to refer to the capacity of a cell to proliferate, for example, to expand in number or in the case of a cell population to undergo population doublings.
An "effective amount" or "therapeutically effective amount" of a compound is that amount of compound which is sufficient to provide a beneficial effect to the subject to which the compound is administered. An "effective amount" of a delivery vehicle is that amount sufficient to effectively bind or deliver a compound.
As used herein "growth factors" is intended the following non-limiting factors including growth hormone, erythropoietin, thrombopoietin, interleukin 3, interleukin 6, interleukin 7, macrophage colony stimulating factor, c-kit ligand/stem cell factor, osteoprotegerin ligand, insulin, insulin like growth factors, epidermal growth factor (EGF), fibroblast growth factor (FGF), nerve growth factor, ciliary neurotrophic factor, platelet derived growth factor (PDGF), transforming growth factor (TGF-beta), hepatocyte growth factor (HGF), and bone morphogenetic protein at concentrations of between picogram/ml to milligram/ml levels.
As used herein, the term "growth medium" is meant to refer to a culture medium that promotes growth of cells. A growth medium will generally contain animal serum. In some instances, the growth medium may not contain animal serum.
An "isolated cell" refers to a cell which has been separated from other components and/or cells which naturally accompany the isolated cell in a tissue or mammal.
As used herein, the "lineage" of a cell defines the heredity of the cell, i.e.; which cells it came from and what cells it can give rise to. The lineage of a cell places the cell within a hereditary scheme of development and differentiation.
A "multi-lineage stem cell" or "multipotent stem cell" refers to a stem cell that reproduces itself and at least two further differentiated progeny cells from distinct developmental lineages. The lineages can be from the same germ layer (i.e. mesoderm, ectoderm or endoderm), or from different germ layers. An example of two progeny cells with distinct developmental lineages from differentiation of a multi-lineage stem cell is a myogenic cell and an adipogenic cell (both are of mesodermal origin, yet give rise to different tissues). Another example is a neurogenic cell (of ectodermal origin) and adipogenic cell (of mesodermal origin).
As used herein, the term "myocardial injury" or "injury to myocardium" refers to any structural or functional disorder, disease, or condition that affects the heart and/or blood vessels. Examples of myocardial injury can include arterial disease, atheroma, atherosclerosis, arteriosclerosis, coronary artery disease, arrhythmia, angina pectoris, congestive heart disease, ischemic cardiomyopathy, myocardial infarction, stroke, transient ischemic attack, aortic aneurysm, cardiopericarditis, infection, inflammation, valvular insufficiency, vascular clotting defects, and combinations thereof.
As used herein, a "passage" refers to a round of subculturing. Thus, when cells are subcultured, they are referred to as having been passaged. A specific population of cells, or a cell line, is sometimes referred to or characterized by the number of times it has been passaged. For example, a cultured cell population that has been passaged ten times may be referred to as a P10 culture. The primary culture, i.e., the first culture following the isolation of cells from tissue, is designated P0. Following the first subculture, the cells are described as a secondary culture (PI or passage 1). After the second subculture, the cells become a tertiary culture (P2 or passage 2), and so on. It will be understood by those of skill in the art that there may be many population doublings during the period of passaging; therefore the number of population doublings of a culture is greater than the passage number. The expansion of cells (i.e., the number of population doublings) during the period between passaging depends on many factors, including the seeding density, substrate, medium, and time between passaging.
As used herein, a "pluripotent cell" defines a less differentiated cell that can give rise to at least two distinct (genotypically and/or phenotypically) further differentiated progeny cells.
The terms "precursor cell," "progenitor cell," and "stem cell" are used interchangeably in the art and herein and refer either to a pluripotent, or lineage-uncommitted, progenitor cell, which is potentially capable of an unlimited number of mitotic divisions to either renew itself or to produce progeny cells which will differentiate into the desired cell type. Unlike pluripotent stem cells, lineage-committed progenitor cells are generally considered to be incapable of giving rise to numerous cell types that phenotypically differ from each other. Instead, progenitor cells give rise to one or possibly two lineage-committed cell types.
"Proliferation" is used herein to refer to the reproduction or multiplication of similar forms, especially of cells. That is, proliferation encompasses production of a greater number of cells, and can be measured by, among other things, simply counting the numbers of cells, measuring incorporation of ³H-thymidine into the cell.
"Progression of or through the cell cycle" is used herein to refer to the process by which a cell prepares for and/or enters mitosis and/or meiosis. Progression through the cell cycle includes progression through the G1 phase, the S phase, the G2 phase, and the M-phase.
A cell of the present invention may be characterized as "positive" for a particular biomarker. A cell positive for a biomarker is one wherein a cell of the invention expresses a specific biomarker protein, or a nucleic acid encoding said protein.
A cell of the present invention may be characterized as "negative" for a particular biomarker. A cell negative for a biomarker is one wherein a cell of the invention does not express a detectable specific biomarker protein, or a nucleic acid encoding said protein.
The terms "patient," "subject," "individual," and the like are used interchangeably herein, and refer to any animal, or cells thereof whether *in vitro* or *in situ,* amenable to the methods described herein. In certain non-limiting embodiments, the patient, subject or individual is a human.
As used herein, a cell exists in a "purified form" when it has been isolated away from all other cells that exist in its native environment, but also when the proportion of that cell in a mixture of cells is greater than would be found in its native environment. Stated another way, a cell is considered to be in "purified form" when the population of cells in question represents an enriched population of the cell of interest, even if other cells and cell types are also present in the enriched population. A cell can be considered in purified form when it comprises in some embodiments at least about 10% of a mixed population of cells, in some embodiments at least about 20% of a mixed population of cells, in some embodiments at least about 25% of a mixed population of cells, in some embodiments at least about 30% of a mixed population of cells, in some embodiments at least about 40% of a mixed population of cells, in some embodiments at least about 50% of a mixed population of cells, in some embodiments at least about 60% of a mixed population of cells, in some embodiments at least about 70% of a mixed population of cells, in some embodiments at least about 75% of a mixed population of cells, in some embodiments at least about 80% of a mixed population of cells, in some embodiments at least about 90% of a mixed population of cells, in some embodiments at least about 95% of a mixed population of cells, and in some embodiments about 100% of a mixed population of cells, with the proviso that the cell comprises a greater percentage of the total cell population in the "purified" population that it did in the population prior to the purification. In this respect, the terms "purified" and "enriched" can be considered synonymous.
"Self-renewal" refers to the ability to produce replicate daughter stem cells having differentiation potential that is identical to those from which they arose. A similar term used in this context is "proliferation."
As used herein, "stem cell" defines an undifferentiated cell that can produce itself and a further differentiated progeny cell.
As used herein, "tissue engineering" refers to the process of generating tissues ex vivo for use in tissue replacement or reconstruction. Tissue engineering is an example of "regenerative medicine," which encompasses approaches to the repair or replacement of tissues and organs by incorporation of cells, gene or other biological building blocks, along with bioengineered materials and technologies.

### Description

The present invention relates to isolated cortical bone-derived stem cells (CBSCs), wherein the cell expresses CD29, Sca-1, CD105, CD44, CD106, CD73, CD271, and CD90, and to the CBSCs of the invention for use in methods of treatment of a cardiovascular disease in a subject. In one embodiment, the cells of the invention reside in cortical bone tissue and can be isolated therefrom. The invention further provides *in vitro* methods of obtaining a population of CBSCs.

The inventive CBSCs are useful for tissue engineering, wound repair, in vivo and ex vivo tissue regeneration, tissue transplantation, and other methods that require cells that can differentiate into a variety of phenotypes and genotypes, or can support other cell types in vivo or in vitro.

In one embodiment, the invention provides a novel population of stem cells derived from cortical bone whereby the cells are c-kit+ and Scal+ but did not express hematopoietic lineage markers upon isolation from cortical bone. In one embodiment, expression of c-kit by CBSCs is decreased following subsequent culturing. However, CBSCs continue to express all of the other signature markers from early to late passage including CD29, Sca-1, CD105, CD106, CD73, CD44, CD271 and CD90. CBSCs remain negative for hematopoietic lineages markers including CD45, CD11b.

In one embodiment, the cells of the invention differentiate into adult cardiomyocytes and vascular cells as well as secrete pro-angiogenic paracrine factors when administered to the injured heart.

The cells described herein are capable of cardiac repair. In one embodiment, the cells of the invention are capable of myogenesis. In another embodiment, the cells of the invention are capable of angiogenesis. Accordingly, the cells of the present invention can be used to treat cardiac tissue damaged due to injury or disease. It is understood by those of skill in the art that the term treating, as used herein, includes repairing, replacing, augmenting, improving, rescuing, repopulating, or regenerating.

### Compositions

The present invention provides an isolated population of cortical bone-derived stem cells (CBSCs), wherein the cell expresses CD29, Sca-1, CD105, CD44, CD106, CD73, CD271, and CD90. Cells derived from the CBSCs include cells that differentiate from, dedifferentiate from, propagate from, and are downstream progeny of the CBSCs.

In one embodiment, the CBSCs can differentiate into two or more distinct lineages from different germ layers (such as endodermal and mesodermal), for example myocytes and adipocytes.

In one embodiment, the CBSCs of the invention can differentiate into cells of two or more lineages, for example adipogenic, chondrogenic, cardiogenic, dermatogenic, hematopoetic, hemangiogenic, myogenic, nephrogenic, neurogenic, neuralgiagenic, urogenitogenic, osteogenic, pericardiogenic, peritoneogenic, pleurogenic, splanchogenic, and stromal developmental phenotypes. While such cells can retain two or more of these different lineages (or developmental phenotypes), preferably, such CBSCs can differentiate into three or more different lineages. The most preferred CBSCs can differentiate into four or more lineages.

In one embodiment, the CBSCs of the invention may differentiate into mesodermal tissues, such as mature adipose tissue, bone, various tissues of the heart (e.g., pericardium, epicardium, epimyocardium, myocardium, pericardium, valve tissue.), dermal connective tissue, hemangial tissues (e.g., corpuscles, endocardium, vascular epithelium.), hematopoetic tissue, muscle tissues (including skeletal muscles, cardiac muscles, smooth muscles.), urogenital tissues (e.g., kidney, pronephros, meta- and meso-nephric ducts, metanephric diverticulum, ureters, renal pelvis, collecting tubules, epithelium of the female reproductive structures (particularly the oviducts, uterus, and vagina), mesodermal glandular tissues (e.g., adrenal cortex tissues), and stromal tissues (e.g., bone marrow). Of course, inasmuch as the CBSC can retain potential to develop into a mature cell, it also can realize its developmental phenotypic potential by differentiating into an appropriate precursor cell (e.g., a preadipocyte, a premyocyte, a preosteocyte).

In another embodiment, the CBSCs may differentiate into ectodermal tissues, such as neurogenic tissue, and neurogliagenic tissue.

In another embodiment, the CBSCs may differentiate into endodermal tissues, such as pleurogenic tissue, and splanchnogenic tissue, and hepatogenic tissue, and pancreogenic tissue.

In another embodiment, the inventive CBSCs can give rise to one or more cell lineages from one or more germ layers such as neurogenic cells (of ectodermal origin) and myogenic cells (of mesodermal origin).

In yet another embodiment, CBSCs may dedifferentiate into developmentally immature cell types, include dedifferentiating into an immature cell type, include embryonic cells and fetal cells or embryonic-like and fetal-like cells.

In one embodiment, the CBSCs have the ability to form new cardiac tissue, blood vessels and cardiac myocytes with mature functional characteristics.

### Methods of obtaining and culturing cells of the invention

Cortical bone tissue can be used as a source of the CBSCs of the invention. The cells isolated from cortical bone tissue can be introduced into a subject for tissue regeneration, wound repair or other applications requiring a source of stem cells. In addition, cortical bone tissue can be treated to cause the CBSCs therein to differentiate into a desired cell type for introduction into a subject. The CBSCs can also be cultured in vitro to maintain a source of CBSCs, or can be induced to produce further differentiated CBSCs that can develop into a desired tissue.

The CBSCs of the invention can be obtained by mechanically and enzymatically dissociating cells from cortical bone tissue. Mechanical dissociation can be brought about using methods that include, without limitation, chopping and/or mincing the tissue, and/or centrifugation. Enzymatic dissociation of connective tissue and from cell-to-cell associations can be brought about by enzymes including Blendzyme, DNAse I, collegenase and trypsin, or a cocktail of enzymes found to be effective in liberating cells from the cortical bone sample. The procedure for mechanically and enzymatically isolating a cell of the present invention should not be construed to be limited to the materials and techniques presented herein, but rather it will be recognized that these techniques are well-established and fall well within the scope of experimental optimization performed routinely in the art.

The CBSCs of the invention are isolated from cortical tissue of the bone. In the isolation of the cells of the invention, cortical bone tissue can be obtained from any animal by any suitable method. A first step in any such method requires the isolation of cortical bone tissue from the source animal. The animal can be alive or dead, so long as cells within cortical bone tissue are viable. Typically, human cortical bone tissue is obtained from a living donor, using well-recognized surgical protocols. The CBSCs of the invention are present in the initially excised or extracted cortical bone tissue, regardless of the method by which cortical bone tissue is obtained. In another embodiment, cortical bone tissue may be obtained from non-human animals.

In one aspect of the disclosure, a cortical bone tissue or section of cortical bone tissue is removed from the animal. In one embodiment, cortical bone tissue is washed with a physiologically-compatible solution, such as phosphate buffer saline (PBS). The washing step consists of rinsing cortical bone tissue with PBS, agitating the tissue, and allowing the tissue to settle. In one embodiment, cortical bone tissue is dissociated. The dissociation can occur by enzyme degradation and neutralization. Alternatively, or in conjunction with such enzymatic treatment, other dissociation methods can be used such as mechanical agitation, sonic energy, or thermal energy.

In some instances, it may be desirable to further process the dissociated tissue. For example, the dissociated cortical bone tissue can be filtered to isolate cells from other connective tissue. The extracted cells can be concentrated into a pellet. One method to concentrate the cells includes centrifugation, wherein the sample is centrifuged and the pellet retained. The pellet includes the cortical bone derived stem cells of the invention.

In one embodiment, the cells are resuspended and can be washed (e.g. in PBS). Cells can be centrifuged and resuspended successive times to achieve a greater purity. In one embodiment, the cells extracted from cortical bone tissue may be a heterogeneous population of cell which includes the cortical bone derived stem cells of the invention. Cortical bone derived adult stem cells may be separated from other cells by methods that include cell sorting, size fractionation, granularity, density, molecularity, morphologically, and immunohistologically. In one embodiment, cortical bone derived stem cells of the invention are separated from other cells by assaying the length of the telomere, as stem cells tend to have longer telomeres compared to differentiated cells. In another embodiment, cortical bone derived stem cells of the invention are separated from other cells by assaying telomeric activity, as telomeric activity can serve as a stem-cell specific marker. In another embodiment, cortical bone derived adult stem cells of the invention are separated from other cells immunohistochemically, for example, by panning, using magnetic beads, or affinity chromatography. For example, in one embodiment, cortical bone derived stem cells can be separated through positive selection of one or more of expressed c-kit (CD117), Sca-1, and β₁-Integrin (CD29) located on the surface of the CBSCs. In one embodiment, the CBSCs of the invention lack the expression of the hematopoietic stem cell marker CD34, the common leukocyte antigen CD45, and other common markers of the hematopoietic lineage that can be detected by a cocktail of antibodies (Lin) against CD5, CD11b, CD45R, antigen 7-4, Gr-1, Ly6G/C, and Terr-119. Accordingly, separation of CBSCs may be carried out through positive selection, negative selection, or depletion. Such methods are well known in the art.

The CBSCs can be cultured and, if desired, assayed for number and viability, to assess the yield. In one embodiment, the stem cells are cultured without differentiation using standard cell culture media (e.g., DMEM, typically supplemented with 5-15% (e.g., 10%) serum (e.g., fetal bovine serum, horse serum). In one embodiment, the stem cells are passaged at least five times in such medium without differentiating, while still retaining their developmental phenotype. In one embodiment, the stem cells are passaged at least 10 times (e.g., at least 15 times or even at least 20 times) while retaining potency.

The CBSCs can be separated by phenotypic identification, to identify those cells that have two or more of the aforementioned developmental lineages. In one embodiment, all cells extracted from cortical bone tissue are cultured. To phenotypically separate the CBSCs from the other cells of cortical bone tissue, the cells are plated at a desired density, such as between about 100 cells/cm² to about 100,000 cells/cm² (such as about 500 cells/cm² to about 50,000 cells/cm², or, more particularly, between about 1,000 cells/cm² to about 20,000 cells/cm²).

In one embodiment the extracted cells of cortical bone tissue is plated at a lower density (e.g., about 300 cells/cm²) to facilitate the clonal isolation of the CBSCs. For example, after a few days, CBSCs plated at such densities will proliferate (expand) into a clonal population of CBSCs.

Such CBSCs can be used to clone and expand a CBSC clonal populations, using a suitable method for cloning cell populations. The cloning and expanding methods include cultures of cells, or small aggregates of cells, physically picking and seeding into a separate plate (such as the well of a multi-well plate). Alternatively, the stem cells can be subcloned onto a multi-well plate at a statistical ratio for facilitating placing a single cell into each well (e.g., from about 0.1 to about 1 cell/well or even about 0.25 to about 0.5 cells/well, such as 0.5 cells/well). The CBSCs can be cloned by plating them at low density (e.g., in a petri-dish or other suitable substrate) and isolating them from other cells using devices such as a cloning rings. Alternatively, where an irradiation source is available, clones can be obtained by permitting the cells to grow into a monolayer and then shielding one and irradiating the rest of cells within the monolayer. The surviving cell then will grow into a clonal population. Production of a clonal population can be expanded in any suitable culture medium, for example, an exemplary culture condition for cloning stem cells (such as the inventive stem cells or other stem cells) is about 2/3 F12 medium+20% serum (e.g. fetal bovine serum) and about 1/3 standard medium that has been conditioned with stromal cells, the relative proportions being determined volumetrically).

In any event, whether clonal or not, the isolated CBSCs can be cultured in a specific inducing medium to induce the CBSCs to differentiate and express its potency. The CBSCs give rise to cells of mesodermal, ectodermal and endodermal lineage, and combinations thereof. Thus, CBSCs can be treated to differentiate into a variety of cell types.

The CBSCs also can be induced to dedifferentiate into a developmentally more immature phenotype (e.g., a fetal or embryonic phenotype). Such an induction is achieved upon exposure of the CBSCs to conditions that mimic those within fetuses and embryos. For example, the inventive CBSCs, can be co-cultured with cells isolated from fetuses or embryos, or in the presence of fetal serum.

The CBSCs of the invention can be induced to differentiate into a mesodermal, ectodermal, or an endodermal lineage by co-culturing the cells of the invention with mature cells from the respective germ layer, or precursors thereof.

In an embodiment, induction of the CBSCs into specific cell types by co-culturing with differentiated mature cells includes myogenic differentiation induced by co-culturing the CBSCs with myocytes or myocyte precursors. Induction of the CBSCs into a neural lineage by co-culturing with neurons or neuronal precursors, and induction of the CBSCs into an endodermal lineage, may occur by co-culturing with mature or precursor pancreatic cells or mature hepatocytes or their respective precursors.

Alternatively, the CBSCs are cultured in a conditioned medium and induced to differentiate into a specific phenotype. Conditioned medium is medium which was cultured with a mature cell that provides cellular factors to the medium such as cytokines, growth factors, hormones, and extracellular matrix. For example, a medium that has been exposed to mature myoctytes is used to culture and induce CBSCs to differentiate into a myogenic lineage. Other examples of conditioned media inducing specific differentiation include culturing in a medium conditioned by exposure to heart valve cells to induce differentiation into heart valve tissue. In addition, CBSCs are cultured in a medium conditioned by neurons to induce a neuronal lineage, or conditioned by hepatocytes to induce an endodermal lineage.

For co-culture, it may be desirable for the CBSCs and the desired other cells to be co-cultured under conditions in which the two cell types are in contact. This can be achieved, for example, by seeding the cells as a heterogeneous population of cells onto a suitable culture substrate. Alternatively, the CBSCs can first be grown to confluence, which will serve as a substrate for the second desired cells to be cultured within the conditioned medium.

Other methods of inducing differentiation are known in the art and can be employed to induce the CBSCs to give rise to cells having a mesodermal, ectodermal or endodermal lineage.

After culturing the stem cells of the invention in the differentiating-inducing medium for a suitable time (e.g., several days to a week or more), the CBSCs can be assayed to determine whether, in fact, they have acquired the desired lineage.

Methods to characterize differentiated cells that develop from the CBSCs of the invention, include histological, morphological, biochemical and immunohistochemical methods, or using cell surface markers, or genetically or molecularly, or by identifying factors secreted by the differentiated cell, and by the inductive qualities of the differentiated CBSCs.

In another embodiment, a population of CBSCs can support cells for culturing other cells. For example, cells that can be supported by CBSCs populations include other types of stem cells, such as neural stem cells (NSC), hematopoetic stem cells (HPC, particularly CD34+ stem cells), embryonic stem cells (ESC) and mixtures thereof), osteoblasts, neurons, chondrocytes, myocytes, and precursors thereof. In other embodiments, the population is substantially homogeneous, consisting essentially of the inventive CBSCs.

In one embodiment, the CBSCs of the invention are capable of cardiac repair. In one embodiment, the CBSCs of the invention are capable of myogenesis. In another embodiment, the CBSCs of the invention are capable of angiogenesis. Accordingly, the CBSCs of the present invention can be used to treat cardiac tissue damaged due to injury or disease. It is understood by those of skill in the art that the term treating, as used herein, includes repairing, replacing, augmenting, improving, rescuing, repopulating, or regenerating.

It may be desirable to induce differentiation of the CBSCs of the invention in a controlled manner and/or by employing factors which are not easily or desirably introduced into the damaged cardiac tissue. Therefore, in one embodiment of the invention, the CBSCs of the invention can be induced to differentiate prior to being introduced into the recipient by, for example, in vitro exposure to extracellular and/or intracellular factors such as trophic factors, cytokines, mitogens, hormones, cognate receptors for the foregoing. In another embodiment, the CBSCs of the invention are not induced to differentiate, but are introduced into the recipient as a substantially pure population of cells that may differentiate following introduction into the recipient.

In one embodiment of the present invention, the CBSCs of the present invention is autologous. That is, a cell of the invention is procured from a donor and returned to the same individual after selection and expansion of said cell; i.e. donor and recipient are the same individual. In another embodiment of the present invention, the CBSCs of the present invention are allogenic. That is, the CBSCs of the invention are procured from a donor but administered to a different individual after selection and expansion of said cell; i.e. the donor and recipient are genetically different individuals.

### Genetic modification

In another embodiment, the CBSCs can be genetically modified, e.g., to express exogenous (e.g., introduced) genes ("transgenes") or to repress the expression of endogenous genes, and the disclosure provides a method of genetically modifying such cells and populations. In accordance with this method, the CBSCs is exposed to a gene transfer vector comprising a nucleic acid including a transgene, such that the nucleic acid is introduced into the cell under conditions appropriate for the transgene to be expressed within the cell. The transgene generally is an expression cassette, including a polynucleotide operably linked to a suitable promoter. The polynucleotide can encode a protein, or it can encode biologically active RNA (e.g., antisense RNA or a ribozyme). Of course, where it is desired to employ gene transfer technology to deliver a given transgene, its sequence will be known.

In the context of gene therapy, the cells of the invention can be treated with a gene of interest prior to delivery of the cells into the recipient. In some cases, such cell-based gene delivery can present significant advantages of other means of gene delivery, such as direct injection of an adenoviral gene delivery vector. Delivery of a therapeutic gene that has been pre-inserted into cells avoids the problems associated with penetration of gene therapy vectors into desired cells in the recipient.

Accordingly, the disclosure provides the use of genetically modified cells that have been cultured according to the methods of the invention. Genetic modification may, for instance, result in the expression of exogenous genes ("transgenes") or in a change of expression of an endogenous gene. Such genetic modification may have therapeutic benefit. Alternatively, the genetic modification may provide a means to track or identify the cells so-modified, for instance, after implantation of a composition of the invention into an individual. Tracking a cell may include tracking migration, assimilation and survival of a transplanted genetically-modified cell. Genetic modification may also include at least a second gene. A second gene may encode, for instance, a selectable antibiotic-resistance gene or another selectable marker.

The cells of the invention may be genetically modified using any method known to the skilled artisan. See, for instance, Sambrook et al. (2001, Molecular Cloning: A Laboratory Manual, Cold Spring Harbor Laboratory Press, Cold Spring Harbor, New York). For example, a cell may be exposed to an expression vector comprising a nucleic acid including a transgene, such that the nucleic acid is introduced into the cell under conditions appropriate for the transgene to be expressed within the cell. The transgene generally is an expression cassette, including a polynucleotide operably linked to a suitable promoter. The polynucleotide can encode a protein, or it can encode biologically active RNA (e.g., antisense RNA or a ribozyme). Thus, for example, the polynucleotide can encode a gene conferring resistance to a toxin, a hormone (such as peptide growth hormones, hormone releasing factors, sex hormones, adrenocorticotrophic hormones, cytokines (e.g., interferins, interleukins, lymphokines)), a cell-surface-bound intracellular signaling moiety (e.g., cell adhesion molecules, hormone receptors), a factor promoting a given lineage of differentiation (e.g., bone morphogenic protein (BMP)), and insulin.

Nucleic acids can be of various lengths. Nucleic acid lengths typically range from about 20 nucleotides to 20 Kb, or any numerical value or range within or encompassing such lengths, 10 nucleotides to 10 Kb, 1 to 5 Kb or less, 1000 to about 500 nucleotides or less in length. Nucleic acids can also be shorter, for example, 100 to about 500 nucleotides, or from about 12 to 25, 25 to 50, 50 to 100, 100 to 250, or about 250 to 500 nucleotides in length, or any numerical value or range or value within or encompassing such lengths. Shorter polynucleotides are commonly referred to as "oligonucleotides" or "probes" of single- or double-stranded DNA.

Nucleic acids can be produced using various standard cloning and chemical synthesis techniques. Techniques include nucleic acid amplification, e.g., polymerase chain reaction (PCR), with genomic DNA or cDNA targets using primers (e.g., a degenerate primer mixture) capable of annealing to antibody encoding sequence. Nucleic acids can also be produced by chemical synthesis (e.g., solid phase phosphoramidite synthesis) or transcription from a gene. The sequences produced can then be translated in vitro, or cloned into a plasmid and propagated and then expressed in a cell (e.g., a host cell such as yeast or bacteria, a eukaryote such as an animal or mammalian cell or in a plant).

Nucleic acids can be included within vectors as cell transfection typically employs a vector. The term "vector," refers to, e.g., a plasmid, virus, such as a viral vector, or other vehicle known in the art that can be manipulated by insertion or incorporation of a polynucleotide, for genetic manipulation (i.e., "cloning vectors"), or can be used to transcribe or translate the inserted polynucleotide (i.e., "expression vectors"). Such vectors are useful for introducing polynucleotides in operable linkage with a nucleic acid, and expressing the transcribed encoded protein in cells in vitro, ex vivo or in vivo.

A vector generally contains at least an origin of replication for propagation in a cell. Control elements, including expression control elements, present within a vector, are included to facilitate transcription and translation. The term "control element" is intended to include, at a minimum, one or more components whose presence can influence expression, and can include components other than or in addition to promoters or enhancers, for example, leader sequences and fusion partner sequences, internal ribosome binding sites (IRES) elements for the creation of multigene, or polycistronic, messages, splicing signal for introns, maintenance of the correct reading frame of the gene to permit in-frame translation of mRNA, polyadenylation signal to provide proper polyadenylation of the transcript of a gene of interest, stop codons, among others.

Vectors included are those based on viral vectors, such as retroviral (lentivirus for infecting dividing as well as non-dividing cells), foamy viruses (U.S. Pat. Nos. 5,624,820, 5,693,508, 5,665,577, 6,013,516 and 5,674,703; WO92/05266 and WO92/14829), adenovirus (U.S. Pat. Nos. 5,700,470, 5,731,172 and 5,928,944), adeno-associated virus (AAV) (U.S. Pat. No. 5,604,090), herpes simplex virus vectors (U.S. Pat. No. 5,501,979), cytomegalovirus (CMV) based vectors (U.S. Pat. No. 5,561,063), reovirus, rotavirus genomes, simian virus 40 (SV40) or papilloma virus (Cone et al., Proc. Natl. Acad. Sci. USA 81:6349 (1984); Eukaryotic Viral Vectors, Cold Spring Harbor Laboratory, Gluzman ed., 1982; Sarver et al., Mol. Cell. Biol. 1:486 (1981); U.S. Pat. No. 5,719,054). Adenovirus efficiently infects slowly replicating and/or terminally differentiated cells and can be used to target slowly replicating and/or terminally differentiated cells. Simian virus 40 (SV40) and bovine papilloma virus (BPV) have the ability to replicate as extra-chromosomal elements (Eukaryotic Viral Vectors, Cold Spring Harbor Laboratory, Gluzman ed., 1982; Sarver et al., Mol. Cell. Biol. 1:486 (1981)). Additional viral vectors useful for expression include reovirus, parvovirus, Norwalk virus, coronaviruses, paramyxo- and rhabdoviruses, togavirus (e.g., sindbis virus and semliki forest virus) and vesicular stomatitis virus (VSV) for introducing and directing expression of a polynucleotide or transgene in pluripotent stem cells or progeny thereof (e.g., differentiated cells).

Vectors including a nucleic acid can be expressed when the nucleic acid is operably linked to an expression control element. As used herein, the term "operably linked" refers to a physical or a functional relationship between the elements referred to that permit them to operate in their intended fashion. Thus, an expression control element "operably linked" to a nucleic acid means that the control element modulates nucleic acid transcription and as appropriate, translation of the transcript.

The term "expression control element" refers to nucleic acid that influences expression of an operably linked nucleic acid. Promoters and enhancers are particular non-limiting examples of expression control elements. A "promoter sequence" is a DNA regulatory region capable of initiating transcription of a downstream (3' direction) sequence. The promoter sequence includes nucleotides that facilitate transcription initiation. Enhancers also regulate gene expression, but can function at a distance from the transcription start site of the gene to which it is operably linked. Enhancers function at either 5' or 3' ends of the gene, as well as within the gene (e.g., in introns or coding sequences). Additional expression control elements include leader sequences and fusion partner sequences, internal ribosome binding sites (IRES) elements for the creation of multigene, or polycistronic, messages, splicing signal for introns, maintenance of the correct reading frame of the gene to permit in-frame translation of mRNA, polyadenylation signal to provide proper polyadenylation of the transcript of interest, and stop codons.

Expression control elements include "constitutive" elements in which transcription of an operably linked nucleic acid occurs without the presence of a signal or stimuli. For expression in mammalian cells, constitutive promoters of viral or other origins may be used. For example, SV40, or viral long terminal repeats (LTRs), or inducible promoters derived from the genome of mammalian cells (e.g., metallothionein IIA promoter; heat shock promoter, steroid/thyroid hormone/retinoic acid response elements) or from mammalian viruses (e.g., the adenovirus late promoter; mouse mammary tumor virus LTR) are used.

Expression control elements that confer expression in response to a signal or stimuli, which either increase or decrease expression of operably linked nucleic acid, are "regulatable." A regulatable element that increases expression of operably linked nucleic acid in response to a signal or stimuli is referred to as an "inducible element." A regulatable element that decreases expression of the operably linked nucleic acid in response to a signal or stimuli is referred to as a "repressible element" (i.e., the signal decreases expression; when the signal is removed or absent, expression is increased).

Expression control elements include elements active in a particular tissue or cell type, referred to as "tissue-specific expression control elements." Tissue-specific expression control elements are typically more active in specific cell or tissue types because they are recognized by transcriptional activator proteins, or other transcription regulators active in the specific cell or tissue type, as compared to other cell or tissue types.

In accordance with the invention, there are provided CBSCs and their progeny transfected with a nucleic acid or vector. Such transfected cells include to a primary cell isolate, populations or pluralities of pluripotent stem cells, cell cultures (e.g., passaged, established or immortalized cell line), as well as progeny cells thereof (e.g., a progeny of a transfected cell that is clonal with respect to the parent cell, or has acquired a marker or other characteristic of differentiation).

The nucleic acid or protein can be stably or transiently transfected (expressed) in the cell and progeny thereof. The cell(s) can be propagated and the introduced nucleic acid transcribed and protein expressed. A progeny of a transfected cell may not be identical to the parent cell, since there may be mutations that occur during replication.

Viral and non-viral vector means of delivery into CBSCs, in vitro, in vivo and ex vivo are included. Introduction of compositions (e.g., nucleic acid and protein) into the cells can be carried out by methods known in the art, such as osmotic shock (e.g., calcium phosphate), electroporation, microinjection, cell fusion. Introduction of nucleic acid and polypeptide in vitro, ex vivo and in vivo can also be accomplished using other techniques. For example, a polymeric substance, such as polyesters, polyamine acids, hydrogel, polyvinyl pyrrolidone, ethylene-vinylacetate, methylcellulose, carboxymethylcellulose, protamine sulfate, or lactide/glycolide copolymers, polylactide/glycolide copolymers, or ethylenevinylacetate copolymers. A nucleic acid can be entrapped in microcapsules prepared by coacervation techniques or by interfacial polymerization, for example, by the use of hydroxymethylcellulose or gelatin-microcapsules, or poly (methylmethacrolate) microcapsules, respectively, or in a colloid system. Colloidal dispersion systems include macromolecule complexes, nano-capsules, microspheres, beads, and lipid-based systems, including oil-in-water emulsions, micelles, mixed micelles, and liposomes.

Liposomes for introducing various compositions into cells are known in the art and include, for example, phosphatidylcholine, phosphatidylserine, lipofectin and DOTAP (e.g., U.S. Pat. Nos. 4,844,904, 5,000,959, 4,863,740, and 4,975,282; and GIBCO-BRL, Gaithersburg, Md.). Piperazine based amphilic cationic lipids useful for gene therapy also are known (see, e.g., U.S. Pat. No. 5,861,397). Cationic lipid systems also are known (see, e.g., U.S. Pat. No. 5,459,127). Polymeric substances, microcapsules and colloidal dispersion systems such as liposomes are collectively referred to herein as "vesicles."

### Therapy

The invention is based on the discovery that CBSCs of the invention can survive when injected into the ischemic heart and have the potential to differentiate into cardiac myocytes with mature function and to secrete factors that promote endogenous repair.

In one embodiment, the CBSCs of the invention are capable of cardiac repair. In one embodiment, the cells of the invention are capable of myogenesis. In another embodiment, the cells of the invention are capable of angiogenesis. Accordingly, the cells of the present invention can be used to treat cardiac tissue damaged due to injury or disease. It is understood by those of skill in the art that the term treating, as used herein, includes repairing, replacing, augmenting, improving, rescuing, repopulating, or regenerating.

Cardiovascular diseases and/or disorders include diseases and/or disorders of the pericardium (i.e., pericardium), heart valves (i.e., incompetent valves, stenosed valves, rheumatic heart disease, mitral valve prolapse, aortic regurgitation), myocardium (coronary artery disease, myocardial infarction, heart failure, ischemic heart disease, angina) blood vessels (i.e., arteriosclerosis, aneurysm) or veins (i.e., varicose veins, hemorrhoids). In specific embodiments, the cardiovascular disease includes coronary artery diseases (i.e., arteriosclerosis, atherosclerosis, and other diseases of the arteries, arterioles and capillaries or related complaint), acute myocardial infarct, organizing myocardial infarct, ischemic heart disease, arrhythmia, left ventricular dilatation, emboli, heart failure, congestive heart failure, subendocardial fibrosis, left or right ventricular hypertrophy, and myocarditis. Yet further, one skilled in the art recognizes that cardiovascular diseases and/or disorders can result from congenital defects, genetic defects, environmental influences (i.e., dietary influences, lifestyle, stress), and other defects or influences.

In one embodiment of the present invention, the CBSCs provides a source of cardiomyocytes and/or endothelial repair progenitors as a cellular agent for cardiovascular therapy. As a source of cardiomyocyte and/or endothelial repair progenitors and based on in vitro, ex vivo and in vivo studies, the present invention utilizes autologous or allogeneic ex vivo-expanded CBSCs.

In one example, the intracoronary injection (implant via catheter or direct injection) of a mixture of autologous or allogeneic CBSCs represents an effective and enduring myocardial and/or cardiovascular replacement therapy. In one embodiment, CBSCs are not immunologically rejective, and, thus, do not need to come from the patient's bone tissue, but can instead come from a suitable human donor.

In one embodiment, the CBSCs of the invention are for use in a method of treating cardiovascular diseases and disorders. CBSCs of the invention have several properties that can contribute to reducing and/or minimizing damage and promoting myocardial or cardiovascular repair and regeneration following damage. These include, among other things, the ability to synthesize and secrete growth factors stimulating new blood vessel formation, the ability to synthesize and secrete growth factors stimulating cell survival and proliferation, the ability to proliferate and differentiate into cells directly participating in new blood vessel formation, the ability to engraft damaged myocardium and inhibit scar formation (collagen deposition and cross-linking), the ability to proliferate and differentiate into muscle cells capable of contributing to myocardial contractility, and the ability to proliferate and differentiate into myocardial cells.

The foregoing means for reducing and/or minimizing damage and promoting myocardial or cardiovascular repair and regeneration following damage using the CBSCs of the invention are described in detail elsewhere herein. Specifically, the present invention demonstrates, for the first time, that the CBSCs of the invention express numerous angiogenic growth factors, including Placenta Growth Factor (PIGF) and Vascular Endothelial Growth Factor (VEGF), contain endothelial progenitor cells (EPC) which have a well-established function in blood vessel formation, develop into blood vessels in vitro, support ischemic tissue survival in vivo, induce reperfusion following occlusion/reperfusion injury of the hind limb, home to the heart when injected into animals after heart injury, and differentiate into cells expressing markers consistent with their differentiation into cardiac myocytes when injected into an animals after heart injury.

Accordingly, in one aspect of the present disclosure, CBSCs are extracted from a donor's cortical bone tissue and are used to elicit a therapeutic benefit to damaged or degenerated myocardium or other cardiovascular tissue. In a preferred embodiment the cells were obtained from cortical tissue of the person into whom they are to be implanted, thereby reducing potential complications associated with antigenic and/or immunogenic responses to the transplant. Patients are typically evaluated to assess myocardial damage or disease by one or more of the following procedures performed by a physician or other clinical provider: patient's health history, physical examination, and objective data including EKG, serum cardiac enzyme profile, and echocardiography.

Cells may be administered to a patient in any setting in which myocardial function is compromised. Examples of such settings include acute myocardial infarction (heart attack), congestive heart failure (either as therapy or as a bridge to transplant), and supplementation of coronary artery bypass graft surgery, among other things. The cells may be extracted in advance and stored in a cryopreserved fashion or they may be extracted at or around the time of defined need. As disclosed herein, the cells may be administered to the patient, or applied directly to the damaged tissue, or in proximity of the damaged tissue, without further processing or following additional procedures to further purify, modify, stimulate, or otherwise change the cells. For example, the cells obtained from a patient may be administered to a patient in need thereof without culturing the cells before administering them to the patient.

The cells may also be applied with additives to enhance, control, or otherwise direct the intended therapeutic effect. For example, in one embodiment, and as described herein, the cells may be further purified by use of antibody-mediated positive and/or negative cell selection to enrich the cell population to increase efficacy, reduce morbidity, or to facilitate ease of the procedure. Similarly, cells may be applied with a biocompatible matrix which facilitates in vivo tissue engineering by supporting and/or directing the fate of the implanted cells. In the same way, cells may be administered following genetic manipulation such that they express gene products that are believed to or are intended to promote the therapeutic response(s) provided by the cells. Examples of manipulations include manipulations to control (increase or decrease) expression of factors promoting angiogenesis or vasculogenesis (for example VEGF), expression of developmental genes promoting differentiation into specific cell lineages (for example MyoD) or that stimulate cell growth and proliferation (for example bFGF-1).

In one embodiment, the CBSCs for use in the method of the invention involves intramyocardial transplantation of CBSCs of the invention. Such therapeutic methods can repair and regenerate damaged myocardium and restore cardiac function after, for example, acute myocardial infarction and/or other ischemic or reperfusion related injuries. Methods generally include contacting a composition containing CBSCs of the invention with cardiac tissue or cells.

In accordance with one method, a composition containing CBSCs of the invention is introduced into the cardiac tissue or a desired site in the subject. In brief, this method can be performed as follows. CBSCs of the invention were obtained from cortical bone tissue. Once obtained, the CBSCs of the invention can be purified and/or expanded. The isolated CBSCs of the invention can then be formulated as a composition comprising the CBSCs of the invention along with, for example, a pharmaceutically acceptable carrier or adjuvant. The composition so formed can then be introduced into the heart tissue of a subject. The subject will usually have been diagnosed as having, or being at risk for, a heart condition, disease, or disorder. Introduction of the composition can be according to methods generally known to the art. For example, the CBSC composition can be administered to a subject's heart by way of direct injection delivery or catheter delivery. Introduction of CBSCs can be a single occurrence or can occur sequentially over a period of time selected by the attending physician. The time course and number of occurrences of CBSC implantation into a subject's heart can be dictated by monitoring generation and/or regeneration of cardiac tissue, where such methods of assessment of treatment course is within the skill of the art of an attending physician.

Cardiac tissue into which CBSCs of the invention can be introduced includes the myocardium of the heart (including cardiac muscle fibers, connective tissue (endomysium), nerve fibers, capillaries, and lymphatics); the endocardium of the heart (including endothelium, connective tissue, and fat cells); the epicardium of the heart (including fibroelastic connective tissue, blood vessels, lymphatics, nerve fibers, fat tissue, and a mesothelial membrane consisting of squamous epithelial cells); and any additional connective tissue (including the pericardium), blood vessels, lymphatics, fat cells, progenitor cells (e.g., side-population progenitor cells), and nervous tissue found in the heart. Cardiac muscle fibers are composed of chains of contiguous heart-muscle cells, or "cardiomyocytes", joined end to end at intercalated disks. These disks possess two kinds of cell junctions: expanded desmosomes extending along their transverse portions, and gap junctions, the largest of which lie along their longitudinal portions. Each of the above tissues can be selected as a target site for introduction of CBSCs, either individually or in combination with other tissues.

A determination of the need for treatment will typically be assessed by a history and physical exam consistent with the myocardial defect, disorder, or injury at issue. Subjects with an identified need of therapy include those with diagnosed damaged or degenerated heart tissue (i.e., heart tissue which exhibits a pathological condition). Causes of heart tissue damage and/or degeneration include chronic heart damage, chronic heart failure, damage resulting from injury or trauma, damage resulting from a cardiotoxin, damage from radiation or oxidative free radicals, damage resulting from decreased blood flow, and myocardial infarction (such as a heart attack). Preferably, a subject in need of treatment according to the methods described herein will be diagnosed with degenerated heart tissue resulting from a myocardial infarction or heart failure. The subject is preferably an animal, including mammals, reptiles, and avians, more preferably horses, cows, dogs, cats, sheep, pigs, and chickens, and most preferably human.

It should be recognized that methods of this invention can easily be practiced in conjunction with existing myocardial therapies to effectively treat or prevent disease. The methods, compositions, and devices of the invention can include concurrent or sequential treatment with non-biologic and/or biologic drugs.

The subject receiving cardiac implantation of CBSCs according to the CBSCs for use in the methods described herein will usually have been diagnosed as having, or being at risk for, a heart condition, disease, or disorder. The CBSCs for use in the methods of the invention can be useful to alleviate the symptoms of a variety of disorders, such as disorders associated with aberrant cell/tissue damage, ischemic disorders, and reperfusion related disorders. For example, the methods are useful in alleviating a symptom of myocardial infarction, chronic coronary ischemia, arteriosclerosis, congestive heart failure, dilated cardiomyopathy, restenosis, coronary artery disease, heart failure, arrhythmia, angina, atherosclerosis, hypertension, or myocardial hypertrophy. The condition, disease, or disorder can be diagnosed and/or monitored, typically by a physician using standard methodologies. Alleviation of one or more symptoms of the condition, disease, or disorder indicates that the composition confers a clinical benefit, such as a reduction in one or more of the following symptoms: shortness of breath, fluid retention, headaches, dizzy spells, chest pain, left shoulder or arm pain, and ventricular dysfunction.

Cardiac cell/tissue damage is characterized by a loss of one or more cellular functions characteristic of the cardiac cell type which can lead to eventual cell death. For example, cell damage to a cardiomyocyte results in the loss of contractile function of the cell resulting in a loss of ventricular function of the heart tissue. An ischemic or reperfusion related injury results in tissue necrosis and scar formation. Injured myocardial tissue is defined for example by necrosis, scarring, or yellow softening of the myocardial tissue. Injured myocardial tissue leads to one or more of several mechanical complications of the heart, such as ventricular dysfunction, decreased forward cardiac output, as well as inflammation of the lining around the heart (i.e., pericarditis). Accordingly, regenerating injured myocardial tissue according to the methods described herein can result in histological and functional restoration of the tissue.

The CBSCs for use in the methods of the invention can promote generation and/or regeneration of heart tissue, and/or promote endogenous myocardial regeneration of heart tissue in a subject. Promoting generation of heart tissue generally includes activating, enhancing, facilitating, increasing, inducing, initiating, or stimulating the growth and/or proliferation of heart tissue, as well as activating, enhancing, facilitating, increasing, inducing, initiating, or stimulating the differentiation, growth, and/or proliferation of heart tissue cells. Thus, the term includes initiation of heart tissue generation, as well as facilitation or enhancement of heart tissue generation already in progress. Differentiation is generally understood as the cellular process by which cells become structurally and functionally specialized during development. Proliferation and growth, as used herein, generally refer to an increase in mass, volume, and/or thickness of heart tissue, as well as an increase in diameter, mass, or number of heart tissue cells. The term generation is understood to include the generation of new heart tissue and the regeneration of heart tissue where heart tissue previously existed.

Generation of new heart tissue and regeneration of heart tissue, resultant from the therapeutic methods described herein, can be measured or detected by procedures known to the art. Such procedures include Western blotting for heart-specific proteins, electron microscopy in conjunction with morphometry, simple assays to measure rate of cell proliferation (including trypan blue staining, the CellTiter-Blue cell viability assay from Promega (Madison, Wis.), the MTT cell proliferation assay from ATCC, differential staining with fluorescein diacetate and ethidium bromide/propidium iodide, estimation of ATP levels, flow-cytometry assays), and any of the methods, molecular procedures, and assays disclosed herein.

In one embodiment of the CBSCs for use in the method of the invention, direct administration of cells to the site of intended benefit is preferred. This may be achieved by direct injection into the external surface of the heart (epicardial), direct injection into the myocardium through the internal surface (endocardial) through insertion of a suitable cannula, by arterial or venous infusion (including retrograde flow mechanisms) or by other means disclosed herein or known in the art. Routes of administration known to one of ordinary skill in the art intravenous, intracoronary, endomyocardial, epimyocardial, intraventicular, retrosinus or intravenous.

As disclosed elsewhere herein, CBSCs may be applied by several routes including systemic administration by venous or arterial infusion (including retrograde flow infusion) or by direct injection into the heart. Systemic administration, particularly by peripheral venous access, has the advantage of being minimally invasive relying on the natural perfusion of the heart and the ability of the CBSCs to target the site of damage. Cells may be injected in a single bolus, through a slow infusion, or through a staggered series of applications separated by several hours or, provided cells are appropriately stored, several days or weeks. Cells may also be applied by use of catheterization such that the first pass of cells through the heart is enhanced by using balloons to manage myocardial blood flow. As with peripheral venous access, cells may be injected through the catheters in a single bolus or in multiple smaller aliquots. Cells may also be applied directly to the myocardium by epicardial injection. This could be employed under direct visualization in the context of an open heart procedure (such as a Coronary Artery Bypass Graft Surgery) or placement of a ventricular assist device. Catheters equipped with needles may be employed to deliver cells directly into the myocardium in an endocardial fashion which would allow a less invasive means of direct application.

In one embodiment, the route of delivery includes intravenous delivery through a standard peripheral intravenous catheter, a central venous catheter, or a pulmonary artery catheter. In other embodiments, the cells may be delivered through an intracoronary route to be accessed via currently accepted methods. The flow of cells may be controlled by serial inflation/deflation of distal and proximal balloons located within the patient's vasculature, thereby creating temporary no-flow zones which promote cellular engraftment or cellular therapeutic action. In another embodiment, cells may be delivered through an endocardial (inner surface of heart chamber) method which may require the use of a compatible catheter as well as the ability to image or detect the intended target tissue. Alternatively, cells may be delivered through an epicardial (outer surface of the heart) method. This delivery may be achieved through direct visualization at the time of an open heart procedure or through a thoracoscopic approach requiring specialized cell delivery instruments. Furthermore, cells could be delivered through the following routes, alone, or in combination with one or more of the approaches identified above: subcutaneous, intramuscular, sublingual, retrograde coronary perfusion, coronary bypass machinery, extracorporeal membrane oxygenation (ECMO) equipment and via a pericardial window.

In one embodiment, cells are administered to the patient as an intra-vessel bolus or timed infusion. In another embodiment, cells may be resuspended in an artificial or natural medium or tissue scaffold prior to be administered to the patient.

In one embodiment, the effects of cell delivery therapy would be demonstrated by one of the following clinical measures: increased heart ejection fraction, decreased rate of heart failure, decreased infarct size, decreased associated morbidity (pulmonary edema, renal failure, arrhythmias) improved exercise tolerance or other quality of life measures, and decreased mortality. The effects of cellular therapy can be evident over the course of days to weeks after the procedure. However, beneficial effects may be observed as early as several hours after the procedure, and may persist for several years.

### EXPERIMENTAL EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the compounds of the present invention and practice the claimed methods. The following working examples therefore, specifically point out the preferred embodiments of the present invention, and are not to be construed as limiting in any way the remainder of the disclosure.

### Example 1: Bone-derived stem cells repair the heart after myocardial infarction through transdifferentiation and paracrine signaling mechanisms

Autologous bone marrow- or cardiac-derived stem cell therapy for heart disease has demonstrated safety and efficacy in clinical trials but functional improvements have been limited. Finding the optimal stem cell type best suited for cardiac regeneration is key toward improving clinical outcomes.

In the experiments presented herein, the cells of the invention were evaluated for the secretion of eight specific paracrine factors: angiopoietin-1 (Ang-1), basic fibroblast growth factor (bFGF), hepatocyte growth factor (HGF), insulin-like growth factor-1 (IGF-1), platelet-derived growth factor (PDGF), stem cell factor (SCF), stromal-derived factor-1 (SDF-1), and vascular-endothelial growth factor (VEGF). HGF and IGF-1 are thought to be cardioprotective: HGF has cytoprotective, anti-apoptotic and pro-angiogenic effects (Gnecchi et al., 2006, FASEB J 20:661-669; Gnecchi et al., 2008, Circ Res 103:1204-1219), while IGF-1 can inhibit apoptosis and may stimulate growth and proliferation of stem cells (Rota et al., 2008, Circ Res 103:107-116; Gnecchi et al., 2006, FASEB J 20:661-669; Gnecchi et al., 2008, Circ Res 103:1204-1219; Williams et al., 2011, Circ Res 109:923-940; Urbanek et al., 2005, Circ Res 97:663-673). SCF, and SDF-1 are thought to stimulate stem cell function: SCF, the ligand for the c-kit receptor (Zsebo et al., 1990, Cell 63:213-224), may stimulate stem cell homing (Kinnaird et al., 2004, Circ Res 94:678-685), while SDF-1 is a chemotactic ligand that induces stem cell proliferation and homing to the site of injury (Gnecchi et al., 2008, Circ Res 103:1204-1219; Williams et al., 2011, Circ Res 109:923-940; Caplan and Dennis, 2006, J Cell Biochem 98:1076-1084). Ang-1, bFGF, PDGF and VEGF all promote angiogenesis: Ang-1 induces vascular cell migration and enhances stability of newly-formed vasculature (Gnecchi et al., 2008, Circ Res 103:1204-1219; Kinnaird et al., 2004, Circ Res 94:678-685), bFGF induces proliferation of endothelial and smooth muscle cells (Gnecchi et al., 2006, FASEB J 20:661-669; Gnecchi et al., 2008, Circ Res 103:1204-1219; Kinnaird et al., 2004, Circulation 109:1543-1549; Kinnaird et al., 2004, Circ Res 94:678-685; Williams et al., 2011, Circ Res 109:923-940), PDGF stimulates smooth muscle cell proliferation (Gnecchi et al., 2008, Circ Res 103:1204-1219; Williams et al., 2011, Circ Res 109:923-940; Kinnaird et al., 2004, Circ Res 94:678-685), and VEGF induces endothelial cell proliferation and tube formation (Gnecchi et al., 2006, FASEB J 20:661-669; Gnecchi et al., 2008, Circ Res 103:1204-1219; Williams et al., 2011, Circ Res 109:923-940; Kinnaird et al., 2004, Circ Res 94:678-685; Kinnaird et al., 2004, Circulation 109:1543-1549; Caplan and Dennis, 2006, J Cell Biochem 98:1076-1084).

It has long been hypothesized that both the heart and bone marrow contain stem cell niches where cells with the capability to differentiate down the cardiac or mesenchymal lineage exist in a dedifferentiated state (Orlic et al., 2001, Nature 410:701-705; Anversa et al., 2006, Circulation 113:1451-1463; Urbanek et al., 2006, Proc Natl Acad Sci USA 103:9226-9231; Kucia et al., 2004, Circ Res 95:1191-1199). In these environments, the stem cell's pluripotent state is thought to be supported by complex signaling interactions with surrounding cells, such as those found in the stromal lining of the bone marrow cavity. Investigators looking for a source of multipotent stem cells developed a new isolation technique using cortical bone tissue, rather than the bone marrow, to isolate cells that might be in a more primitive state (Zhu et al., 2010, Nat Protoc 5:550-560). These cells, which can be easily obtained through routine bone biopsy procedures, were negative for most markers of the hematopoietic lineage and expressed the pluripotency marker Sca-1. Under specific culture conditions these cells differentiate *in vitro* into osteoblasts, chondrocytes, and adipocytes (Zhu et al., 2010, Nat Protoc 5:550-560) but no one has yet tested their cardiogenic potential in the injured heart. In the present study it is demonstrated that injection of cortical bone-derived stem cells (CBSCs) into the heart after MI improved survival and cardiac function, and CBSCs demonstrated greater improvements across all parameters compared to the more widely studied CDCs. CBSCs differentiated into mature cardiac tissue, while CDCs did not, and CBSCs demonstrated a greater capacity for paracrine-mediated endogenous repair to produce these effects. The present study demonstrates that CBSCs are a source of stem cells that are more abundant and more easily isolated than CDCs, and that CBSCs have a greater capacity to repair hearts damaged by ischemic injury.

The materials and methods used in this experimental example are now described.

### Methods and Materials

### Animals and Anesthesia

For all procedures (bone isolation, myocardial infarction, echocardiography and cardiectomy) anesthesia was induced using 3% isoflurane and maintained using 1% isoflurane (Butler Shein Animal Health; Dublin, Ohio). Adequate induction of anesthesia was confirmed prior to any intervention by observation of a negative paw- or tail-pinch reflex. For procedures involving a thoracotomy (i.e. myocardial infarction surgeries) animals were intubated after induction of anesthesia and ventilated at a rate of 180-190 breaths per minute and a tidal volume of 250-300 µL (ventilation parameters were adjusted accordingly depending on the size of each animal). For all other procedures, maintenance anesthesia was delivered via nose cone. All animals used for in vivo studies were 12-week-old male C57BL/6 mice (The Jackson Laboratory; Bar Harbor, ME).

### Isolation and culture of cortical bone-derived stem cells

Cortical bone stem cells (CBSCs) were isolated using previously published techniques (Zhu et al., 2010, Nat Protoc. 5:550-560). Femurs and tibias were isolated from transgenic 12-week-old male C57BL/6-Tg(CAG-EGFP)1Osb/J mice (The Jackson Laboratory; Bar Harbor, ME), which constitutively express enhanced green fluorescent protein (EGFP) off of the β-actin promoter in most cells of the body. The epiphyses of the bones were removed, and the marrow cavity was flushed three times with phosphate-buffered saline (PBS) and the marrow was discarded. The remaining cortical bone was crushed using a sterilized mortar and pestle, and bone fragments were further digested using collagenase II. Bone chunks were then plated in CBSC culture media: DMEM/F12 Media (Lonza/Biowhittaker; Basel, Switzerland) + 10% fetal bovine serum (Gibco Life Technologies; Grand Island, NY), 1% Penicillin/Streptomycin/L-glutamine (Gibco Life Technologies; Grand Island, NY), 0.2% insulin-transferrin-selenium (Lonza; Basel, Switzerland), 0.02% basic-fibroblast growth factor (Peprotech; Rock Hill, NJ), 0.02% epidermal growth factor (Sigma; St. Louis, MO), and 0.01% leukemia inhibitory factor (Millipore; Billerica, MA). Over the first week in culture, fibroblast-like stem cells began to grow out from the bone chunks, and after 1 week in culture, the remaining chunks of bone were washed away and the adherent population of CBSCs could be passaged for expansion. Expanded cells could be resuspended in CBSC culture media + 10% DMSO to be frozen and stored long-term in liquid nitrogen. CBSCs were derived from bone that was devoid of bone marrow cells, and only contained cells that were within the stroma, requiring culture of bone chips. The culture conditions were optimized to allow CBSCs to migrate from the bone chips to the culture dish, where they were then allowed to expand.

### Isolation of cardiac-derived stem cells

Cardiectomy was performed on transgenic 12 week-old male C57BL/6-Tg(CAG-EGFP)1Osb/J mice (The Jackson Laboratory; Bar Harbor, ME) under general anesthesia. Hearts were cannulated and perfusion digested to dissociate stem cells from the left ventricle, and cardiac-derived stem cells (CDCs) underwent sorting for c-kit using magnetic beads (Miltenyi Biotec; Cologne, Germany) following a previously described protocol (Beltrami et al., 2003, Cell. 114:763-776; Urbanek Ket al., 2005, Circ Res. 97:663-673). Cells were cultured and stored long term under identical conditions to CBSCs as previously described.

### Isolation and Culture of Mouse Left Ventricular Myocytes

Left ventricular myocytes were isolated from mice receiving MI and CBSC therapy 6 weeks after injection for myocyte staining and cell physiology experiments. Cardiectomy was performed under general anesthesia then hearts were cannulated and perfusion digested with collagenase-containing Tyrodes solution on a constant-flow Langendorff apparatus, and left ventricular myocytes were isolated and cultured as previously described (Zhang et al., 2012, Circ Res.110:831-840; Makarewich et al., 2012, Circ Res. 110:669-674). All myocytes isolated from the left ventricle of each MI+CBSC mouse were plated on laminin-coated 18mm round glass coverslips. Some coverslips were used to measure fractional shortening and calcium transients, others were fixed and immunostained for cell counts, surface area analysis, and nuclei counts.

To estimate the percentage of all LV myocytes that were EGFP+ (and thus derived from injected CBSCs), an average number of 15.67±3.67 EGFP+ myocytes were counted on each coverslip. The total number of myocytes on each coverslip was estimated by counting the myocytes in ten random 10X visual fields, and an average of 12.4±1.51 total myocytes/field were counted. A 10X visual field has a surface area of 1.302 mm2, and an 18mm coverslip has a surface area of 81πmm², so there are 81π/1.30² = 150.18 10X visual fields/coverslip. Thus there were an average of 12.4 * 150.18 = 1862.26 total myocytes/coverslip. So by 6 weeks post-MI, an estimated 15.67/1862.26 = 0.84% of myocytes isolated from CBSC-injected animals were EGFP+.

### Fractional Shortening and Calcium Transients

Mouse left ventricular myocytes isolated from CBSC-injected animals 6 weeks post-MI were simultaneously measured for fractional shortening and calcium transients as has been previously described (Makarewich et al., 2012, Circ Res. 110:669-674; Zhang et al., 2012, Circ Res.110:831-840).

### Flow Cytometry

For flow cytometry, 5x10⁶ CBSCs or CDCs were incubated for 15 minutes at 4°C under gentle agitation in the appropriate antibody diluted 1:11 in wash buffer (PBS+ 0.5% bovine serum albumin + 2 mM EDTA, pH = 7.3). After incubation, cells were washed with 2 mL wash buffer, centrifuged at 300 Xg for 5 min, and supernatants were aspirated and discarded. Stained cells were resuspended in PBS for flow cytometry. A second sample of CBSCs or CDCs was stained in each condition with APCconjugated Rat IgG2b, which was used as a negative isotype control. The following conjugated antibodies or pairs of primary and secondary antibodies along were their corresponding nonspecific negative isotype controls were used:

**Table 1: Antibodies used for flow cytometry**

| **Marker** | **Primary Antibody** | **Negative Isotype Control** |
|---|---|---|
| Sca-1 | Rat IgG2a anti-Sca-1-APC(Miltenyi Biotec; Cologne, Germany) | Rat IgG2a-APCInvitrogen; Cambridge, MA |
| CD29 | Rat IgG anti-CD29-APC(eBiosciences; San Diego, CA) | Rat IgG-APC(eBiosciences; San Diego, CA) |
| CD34 | Rat IgG2a anti-CD34-Alexa Fluor 647(AbD Serotec; Kidlington, UK) | Rat IgG2a-APC(AbD Serotec; Kidlington, UK) |
| CD45 | Rat IgG2b anti-CD45-Alexa Fluor 647(AbD Serotec; Kidlington, UK) | Rat IgG2b-APC(AbD Serotec; Kidlington, UK) |
| C-kit | 1° goat IgG anti-SCF-R(R&D Systems; Minneapolis, MN)2° anti-Goat IgG Rhodamine Red-X(Jackson ImmunoResearch Labs; WestGrove, PA) | 1° goat IgG(R&D Systems; Minneapolis, MN)2° anti-Goat IgG Rhodamine Red-X(Jackson ImmunoResearch Labs; WestGrove, PA) |
| Lineage Cocktail | 1° Biotin-Conjugated Lineage Cocktail(Miltenyi Biotec; Cologne, Germany)2° anti-Biotin-APC (Miltenyi Biotec; Cologne, Germany) | 2° anti-Biotin-APC only(Miltenyi Biotec; Cologne, Germany) |

### RNA Isolation and PCR Analysis

CDCs or CBSCs were resuspended in QIAzol Lysis Reagent, and mRNA was isolated using an RNeasy Mini Kit. DNA was eliminated from the samples using RNase-free DNase I, and then cDNA was generated using RT2 First Strand Kit. RT² qPCR Primer Assays for mouse Kit (c-kit), mouse Ly-6A (Sca-1), and mouse glyceraldehyde 3-phosphate dehydrogenase (GAPDH) were used with RT² SYBR Green qPCR mastermix to detect c-kit, Sca-1, or GAPDH mRNA expression, respectively. The amount of GAPDH mRNA in each cell type was determined using a 6-point standard curve with a 1:10 serial dilution of each transcript run on each primer set. The amount of transcript detected for c-kit or Sca-1 was normalized to detected levels of GAPDH and these data are presented as normalized arbitrary units. All qPCR reagents were purchased from Qiagen (Valencia, CA).

### Protein Isolation and Western Analysis

CBSC or CDC lysates were prepared and analyzed using Western analysis as previously described (Makarewich et al., 2012, Circ Res. 110:669-674; Kubo et al., 2001, Circulation. 104:1012-1018). The following primary antibodies purchased from Abcam (Cambridge, MA), Cell Signaling (Danvers, MA) or AbD Serotec (Kidlington, UK) were used to detect target antigens: insulin-like growth factor-1 (Abcam ab106836), angiopoietin-1 (Abcam ab95230), basic-fibroblast growth factor (Abcam, ab8880), hepatocyte growth factor (Abcam ab83760), platelet-derived growth factor (Abcam ab125268), stem cell factor (Abcam ab9753), stromal-derived factor-1 (Cell Signaling #3740S), vascular endothelial growth factor (Abcam ab46154) and Glyceraldehyde 3-phosphate dehydrogenase (AbD Serotec) . The following secondary antibodies were used: rabbit-HRP (GE#NA934V) and mouse-HRP (GE#NA931V) purchased from GE Healthcare (Little Chalfont, UK) and goat-HRP (sc-2020) purchased from Santa Cruz Biotechnology (Dallas, TX).

### Enzyme-Linked Immunosorbent Assays

CBSCs or CDCs were plated at a low density of 25,000 cells/well in complete CBSC media or serum free media in a 6 well plate and allowed to proliferate to 90% confluency over 72 hours. Serum samples were collected every 24 hours and frozen at -20°C. Samples were analyzed using mouse DuoSet ELISA Kits for hepatocyte growth factor, insulin-like growth factor, stem cell factor, stromal-derived factor-1 and vascular endothelial growth factor. The presence of serum in the cell cultures did not affect cytokine production. All ELISA kits were purchased from R&D Systems (Minneapolis, MN). The data in each case is presented as a mean of 3 samples, and for each ELISA, background signal was subtracted using the mean of 3 samples containing unconditioned media only. A student's T test was used to detect any significant difference in production of each paracrine factor between CBSCs and CDCs.

### In vitro Differentiation Co-cultures

Neonatal rat ventricular myocytes were isolated following the Simpson and Savion protocol (Simpson et al., 1982, Circ Res. 50:101-116) with minor modifications that we have previously described (Kubo et al., 2009, Clin Transl Sci. 2:26-32; Gaughan et al., 1998, Am J Physiol. 275:H577-590). Cells were plated overnight on gelatin-coated 18mm glass coverslips in a 12-well dish. Stem cells were added the following day at low densities (1000-5000 cells/well) on top of the neonatal rat myocytes. Cells were allowed to differentiate in co-culture for 72 hours. During this time some EGFP+ cells were observed to beat. After 72 hours, coverslips were fixed and stained for α-sarcomeric actin or connexin43, and the percentage of cells expressing either marker was determined after counting cells in 10 visual fields on 5 individual coverslips.

### Mouse Myocardial Infarction and Intramyocardial Stem Cell Transplantation

Permanent occlusion myocardial infarction (MI) surgery was performed by ligating the left anterior descending coronary artery following a widely cited protocol (Tarnavski et al., 2004, Physiol Genomics. 16:349-360). Immediately after MI, 40,000 CBSCs (n=67) or CDCs (n=36) suspended in normal saline were injected intramyocardially into the infarct border zone in four x 5 µL injections. MI control animals (n=60) received saline injection only, and sham control animals (n=21) received all surgical procedures except for ligation of the coronary artery and intramyocardial injection.

Each group of animals that underwent surgery was assigned a sacrifice date prior to surgery. This prevented selection bias at the time of sacrifice, so our Kaplan-Meier analysis was designed to measure 6-week survival from the time of myocardial infarction surgery. The Kaplan-Meier analysis was designed to detect "death" as event occurrence (if the animal died prior to its assigned sacrifice date). Animals that survived until their assigned sacrifice date (either at 1, 2, or 6 weeks post-MI, depending on the group) were censored in the Kaplan-Meier analysis.

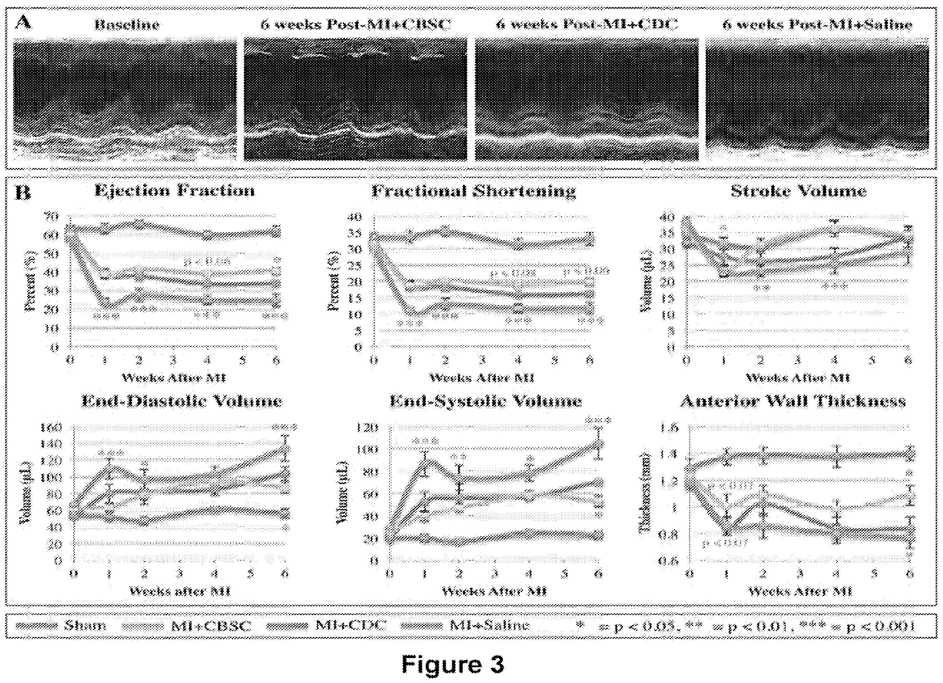

Animals selected for 24-hour sacrifice were not included in Kaplan-Meier analysis. For the MI+Saline group, 5 were sacrificed at 24 hours post-MI for infarct size analysis. For the MI+CDC or MI+CBSC groups, a total of 10 animals were sacrificed at 24 hours post-MI (5 animals for infarct size analysis and 5 animals for histology). Animals assigned to 6-week sacrifice groups underwent serial echocardiography and strain analysis using the Vevo2100 ultrasound system and these data are presented in Figures 3 and 4.

Sham (n=21): 5 animals were sham operated for 1 week sacrifice, 5 animals were operated for 2 week sacrifice, and 11 animals were operated for 6 week sacrifice for a total of 21 animals. No animals in the sham-operated group died prior to their assigned sacrifice date (100% 6 week survival). All hearts were fixed for histology at each time point.

MI+Saline (n=60): 5 animals were sacrificed 24 hours post-MI for infarct size analysis and were not included in the Kaplan-Meier analysis. The remaining 55 animals were assigned to sacrifice groups at 1, 2, or 6 weeks post-MI and were included in Kaplan-Meier analysis. Of these 55 animals, 8 animals were operated for 1-week sacrifice and 2 died prior to sacrifice (the remaining 6 were fixed for histology). 23 animals were operated for 2-week sacrifice and 10 died prior to sacrifice (the remaining 13 hearts were fixed for histology). 24 animals were operated for 6-week sacrifice and 11 died prior to sacrifice (the remaining 13 hearts were fixed for histology). Thus, for Kaplan-Meier analysis, 55 animals were included and a total of 23 died prior to sacrifice and were counted as deaths.

MI+CBSC (n=67): 10 animals were sacrificed 24 hours post-MI for infarct size analysis (n=5) or histology (n=5) and were not included in the Kaplan-Meier analysis. The remaining 57 animals were assigned to sacrifice groups at 1, 2, or 6 weeks post-MI and were included in Kaplan-Meier analysis. Of these animals, 20 were operated for 1-week sacrifice and 6 died prior to sacrifice (the remaining 14 hearts were fixed for histology). 21 animals were operated for 2-week sacrifice and 3 died prior to sacrifice (the remaining 18 hearts were fixed for histology). 16 animals were operated for 6-week sacrifice and 3 died prior to sacrifice. Of the 13 animals sacrificed at 6 weeks post-MI, 5 were digested for myocyte isolation and 8 were perfusion fixed for histology. Thus, for Kaplan-Meier analysis, 57 animals were included and a total of 12 died prior to sacrifice and were counted as deaths. For histological analysis, 14 MI+CBSC hearts were fixed at 1-week post-MI, 18 were fixed at 2 weeks post-MI, and 8 were fixed at 6 weeks post-MI. When stem cell-injected hearts were analyzed, each heart was sectioned completely into 5 um-thick sections and every single slide was stained. Because such comprehensive histological analysis is necessary, 6 hearts fixed at 1 week post-MI and 6 hearts fixed at 2 weeks post-MI were randomly selected for analysis. All hearts fixed at 6 weeks post-MI were completely sectioned, stained, and analyzed.

MI+CDC (n=36): 10 animals were sacrificed 24 hours post-MI for infarct size analysis (n=5) or histology (n=5) and were not included in the Kaplan-Meier analysis. The remaining 26 animals were assigned to sacrifice groups at 1, 2, or 6 weeks post-MI and were included in Kaplan-Meier analysis. Of these animals, 8 were operated for 1-week sacrifice and all animals survived (all 8 hearts were fixed for histology). 8 animals were operated for 2-week sacrifice and 4 died prior to sacrifice (the remaining 4 hearts were fixed for histology). 10 animals were operated for 6-week sacrifice and 4 died prior to sacrifice (the remaining 6 hearts were fixed for histology). Thus, for Kaplan-Meier analysis, 26 animals were included and a total of 8 died prior to sacrifice and were counted as deaths.

For histological analysis, all hearts were completely sectioned and analyzed on histology (8 at 1 week post-MI, 4 at 2 weeks post-MI and 6 at 6 weeks post-MI).

### Infarct Size Analysis

After 24 hours post-MI, five animals from each study group (MI+CBSC, MI+CDC, or MI+Saline) were randomly selected to undergo acute infarct size analysis. Cardiectomy was performed under general anesthesia, and the hearts were perfused with 2% Evan's Blue dye in PBS to stain the area at risk (AAR), which accounts for all areas of the myocardium except for those perfused by the ligated coronary artery. The heart was then flash-frozen in liquid nitrogen so it could be cut into 6-8 short-axis cross sections. The sections were washed in PBS to remove excess Evan's Blue dye then incubated in 2% triphenyltetrazolium chloride in PBS for 10 minutes at 37°C to stain ischemic tissues white (ischemic area, IA) and viable tissues shaded. Samples were washed again in PBS then photographed under a top-lit dissecting scope. AAR and IA were measured on each photograph using NIH image J software, and a mean AAR and IA for each heart was calculated as a percentage of total ventricular area.

For chronic infarct size analysis, paraffin-embedded short-axis heart sections from MI+CBSC, MI+CDC, or MI+Saline sacrificed 6 weeks post-MI were stained with hematoxylin and eosin (H&E). Brightfield photographs were acquired on a dissecting microscope using a DS-Fi1 color camera and NIS Elements software (all from Nikon Inc.; Melville, NY). Pathologically infarcted regions of the myocardium were identified and their surface area was quantified using NIH Image J software. Infarct area was calculated as a percentage of total ventricular surface area for 3-4 cross-sections

### Two-Dimensional Echocardiography and Strain Analysis

Anesthetized mice underwent transthoracic echocardiography using a Vevo2100 ultrasound system (VisualSonics; Toronto, Canada). Repeated measurements were performed as previously described (Zhang et al., 2012, Circ Res.110:831-840; Duran et al., 2012, Clin Transl Sci. 5:416-421; Taghavi et al., 2012, Am J Transl Res. 4:240-246) at baseline and at 1, 2, 4, and 6 weeks post-MI. Images were acquired by JMD in the short-axis B-mode and M-mode for analysis of cardiac function and dimensions. Long-axis B-mode images were recorded for longitudinal and radial strain analysis using the VevoStrain software following a recently published protocol (Bauer et al., 2011, Circ Res. 108:908-916). After echocardiograms were recorded, image series were randomly ordered and renumbered by CAM. All images were analyzed under their coded numbers in a blinded fashion by JMD, then the code was broken by CAM and animal data was sorted by treatment group then analyzed.

### Perfusion Fixation

Cardiectomy was performed under general anesthesia and the heart was rinsed and weighed. The aorta was then cannulated and the coronary arteries were cleared by perfusion with 1 mL cold Krebs- Henseleit Buffer. The heart was then arrested in diastole by perfusion with 1 mL of 100 mM cadmium chloride/1 M potassium chloride solution. The hearts were then gravity perfused with 30 mL 10% formalin at mean arterial pressure (100 mmHg). Fixed hearts were immersed overnight in 10% formalin and then stored in 70% ethanol for up to 1 week before being processed and embedded in paraffin wax blocks.

### Immunohistochemistry

For *in vitro* staining, cells were plated on gelatin-coated coverslips overnight and then fixed with 4% paraformaldehyde. Cells were permeablized with 0.1% Triton X-100 in PBS (Fluka/Sigma-Aldrich; St. Louis, MO) and stained for the following proteins: c-kit and goat IgG isotype control for c-kit (AF1356 and AB-108-C, R&D Systems; Minneapolis, MN), Rat anti-Sca-1-biotin (130-093-421, Miltenyi Biotec; Auburn, CA) and rat IgG2a-biotin isotype control for Sca-1 (IC006B, R&D Systems; Minneapolis, MN), insulin-like growth factor-1 (SC-9013, Santa Cruz Biotechnology; Dallas, TX), angiopoietin-1 (ab95230), basic-fibroblast growth factor (ab8880), hepatocyte growth factor (ab83760), platelet-derived growth factor (ab61219), stem cell factor (ab64677), stromal-derived factor-1 (ab64677), and vascular endothelial growth factor (ab46154) all purchased from Abcam (Cambridge, MA). For neonatal rat ventricular myocyte co-cultures, cells were stained for α-sarcomeric actin (A2172, Sigma; St. Louis, MO) or connexin43 (AB1728, Millipore; Billerica, MA). The following secondary antibodies were purchased from Jackson Immunoresearch Laboratories (West Grove, PA) and used for detection of primary antibodies as follows: Rhodamine Red-X donkey anti-goat IgG (705-295-147) was used to detect c-kit; Rhodamine Red-X donkey anti-biotin (200-292-211) was used to detect Sca-1; rhodamine red-X donkey anti-rabbit IgG (711-295-152) was used to detect Ang-1, bFGF, HGF, PDGF, SCF, SDF-1, VEGF, and connexin43; and rhodamine red-X donkey anti-Mouse IgM (715-295-020) was used to detect α-sarcomeric actin.

For fixed tissues, wax blocks were cut into 5µm thick sections that were mounted on glass slides for staining. Slides were deparaffinized and underwent antigen retrieval in hot citric acid buffer. Stains were conducted against the following proteins: α-sarcomeric actin and α-smooth muscle actin (A2172 and A2547, Sigma; St. Louis, MO), EGFP and von Willebrand factor (ab111258 and ab6994, Abcam; Cambridge, MA), and connexin43 (AB1728 Millipore; Billerica, MA). The following secondary antibodies were purchased from Jackson Immunoresearch Laboratories (West Grove, PA) and used for detection of primary antibodies as follows: rhodamine red-X donkey anti-Mouse IgM (715-295-020) or Cy5 donkey anti-Mouse IgM (715-175-140) were used to detect α-sarcomeric actin; rhodamine red-X donkey anti-Mouse IgG (715-295-151) or Cy5 donkey anti-Mouse IgG (715-175-151) were used to detect α-smooth muscle actin; FITC donkey anti-goat IgG (705-095-147) was used to detect EGFP, and rhodamine red-X donkey anti-rabbit IgG (711-295-152) or Cy5 donkey anti-rabbit IgG (711-175-152) was used to detect von Willebrand factor and connexin43. Nuclei in both cells and embedded tissues were stained with 4',6-diamidino-2-phenylindole (DAPI, Millipore; Billerica, MA).

Confocal micrographs of all immunostains were acquired using a Nikon Eclipse T1 confocal microscope (Nikon Inc.; Mellvile, NY).

### Statistical Analysis

Survival analysis is presented using a Kaplan-Meier regression and statistical significance was determined using the log-rank test. For ELISA, infarct size analysis, blood vessel counts and isolated myocyte measurements (where discrete measurements were compared at a single time point), a two-way T test was used. For follow-up parameters with repeated measures (echocardiography and strain analysis), growth curve models with cubic splines were used. All growth curve coefficients were fitted as random effects to allow deviation of individual growth from the mean of the treatment group. Interaction term with the treatment was also included to compare the mean growth rates by treatment. For all statistical tests, a p-value < 0.05 was considered statistically significant.

The results of the experiments in this experimental example are now described.

### Cortical Bone Stem Cell Characterization

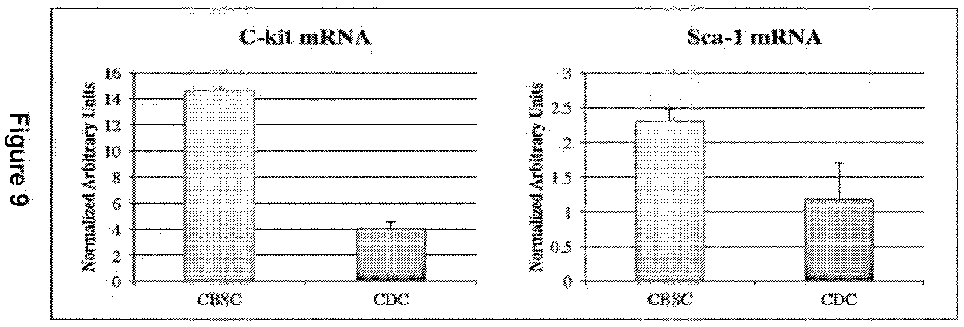

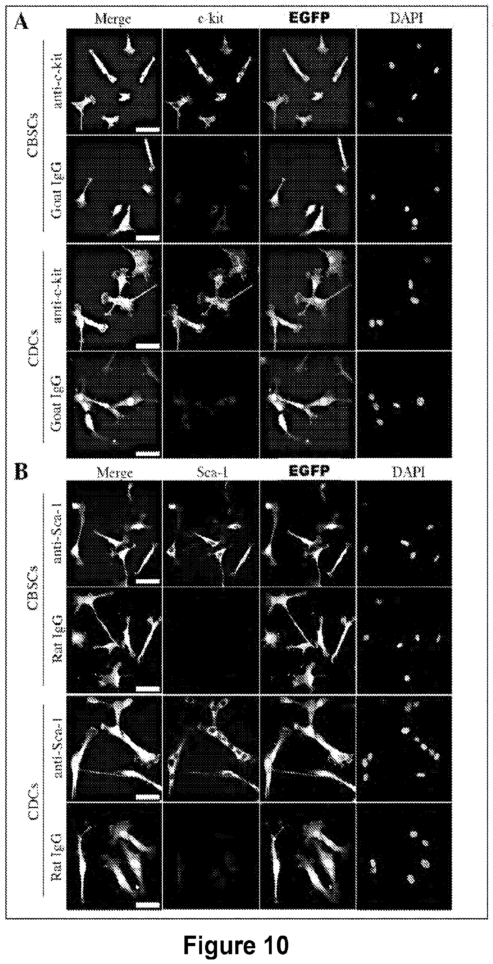

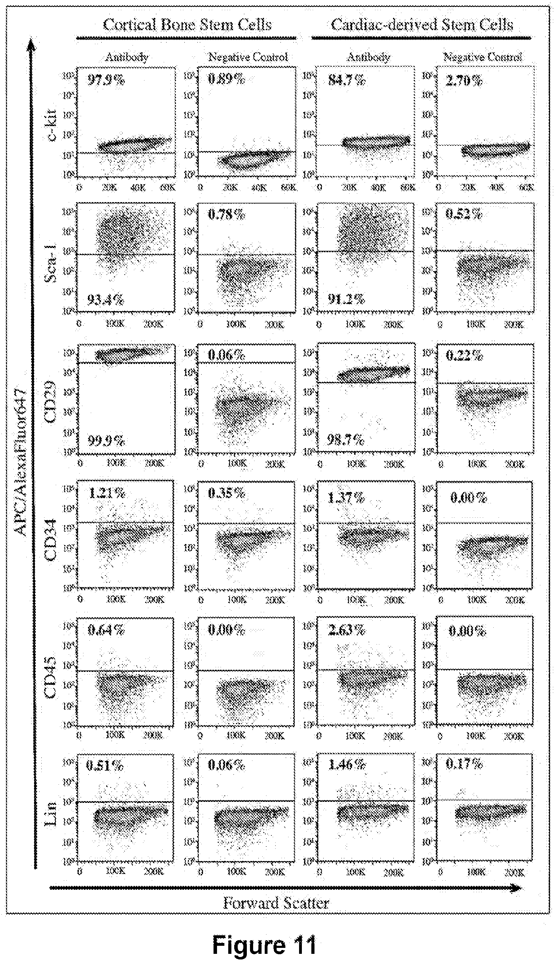

Cortical bone stem cells (CBSCs) or cardiac-derived stem cells (CDCs) were analyzed for expression of c-kit and Sca-1 mRNA abundance using quantitative real-time PCR (qPCR) (Figure 9). C-kit and Sca-1 protein expression was detected using both immunostaining (Figure 10) and flow cytometry (Figure 11). CBSCs expressed greater levels of both transcripts than did CDCs: over 3-fold higher levels of c-kit and 2-fold higher levels of Sca-1 (Figure 9). Both stem cells types demonstrated positive membrane immunostaining for c-kit and Sca-1 (Figure 10). Flow cytometry analysis demonstrated that the majority of CBSCs and CDCs expressed c-kit (CD117), Sca-1, and β₁-Integrin (CD29). Additionally both cell types lacked expression of the hematopoietic stem cell marker CD34, the common leukocyte antigen CD45, and other common markers of the hematopoietic lineage that can be detected by a cocktail of antibodies (Lin) against CD5, CD11b, CD45R, antigen 7-4, Gr-1, Ly6G/C, and Terr-119 (Figure 11).

Next, it was examined if the stem cells, cultured *in vitro,* expressed or secreted paracrine factors that are thought to be involved in cardioprotection, neovascularization, or recruitment of endogenous cardiac stem cells (Gnecchi et al., 2008, Circ Res 103:1204-1219; Williams et al., 2011, Circ Res 109:923-940; Caplan and Dennis, 2006, J Cell Biochem 98:1076-1084). Eight specific factors produced by CBSCs and CDCs were analyzed: Ang-1, bFGF, HGF, IGF-1, PDGF, SCF, SDF-1, and VEGF. Protein expression of all eight factors was detected by Western analysis performed on CBSC or CDC lysates collected *in vitro* (Figure 1A). Positive expression of these factors *in vitro* was confirmed by immunostaining (Figure 1B). Enzyme-linked immunosorbent assays of stem cell-conditioned media demonstrated that IGF-1, VEGF, and SDF-1 were all secreted by proliferating CBSC and CDCs in culture (Figure 1C), and no significant difference between the amount of paracrine factors secreted by each cell type could be detected. Neither HGF nor SCF were secreted in detectable amounts by either stem cell type, even though both factors were seen at the protein level by both Western analysis and immunostaining. These results show that both CBSCs and CDCs produce factors known to be associated with beneficial cardiac remodeling after MI (Gnecchi et al., 2005, Nat Med 11:367-368; Gnecchi et al., 2006, FASEB J 20:661-669; Williams et al., 2011, Circ Res 109:923-940).

### CBSCs differentiate in vitro in co-culture with neonatal rat ventricular myocytes

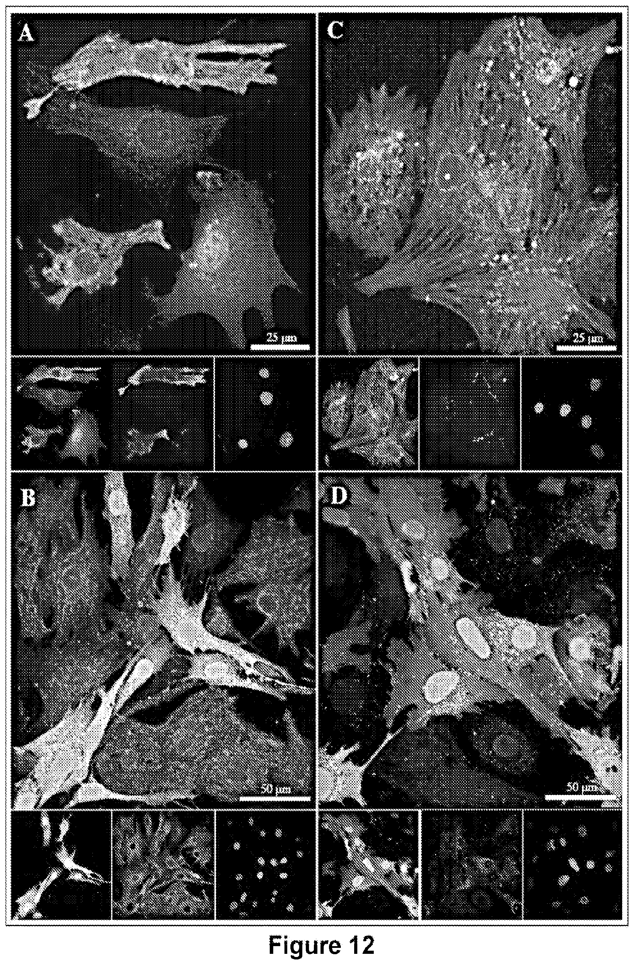

Both bone marrow-derived (Kubo et al., 2009, Clin Transl Sci. 2:26-32; Hatzistergos et al., 2010, Circ Res 107:913-922) and CDCs (Messina et al., 2004, Circ Res 95:911-921) have previously been shown to differentiate *in vitro* in co-culture with neonatal rat ventricular myocytes. To quantify the rate of *in vitro* differentiation into cells expressing cardiac myocyte proteins, CBSCs cocultured with neonatal rat ventricular myocytes were fixed and stained for α-sarcomeric actin (Figure 12A) or connexin43 (Figure 12B). Staining revealed that 11.5±0.9% (n=5) of EGFP+ cells expressed α-sarcomeric actin and 7.5±1.7% (n=5) appeared coupled via connexin43 gap junctions to their neighboring cells. A very small fraction of EGFP+ cells (<1%) were observed to contract spontaneously after 72 hours in co-culture. Thus, like bone marrow- or CDCs, CBSCs, *in-vitro,* demonstrate some capacity to differentiate into cells expressing cardiac specific proteins *in vitro.*

### CBSC transplants improved survival and cardiac function, attenuated adverse left ventricular remodeling and reduced infarct size

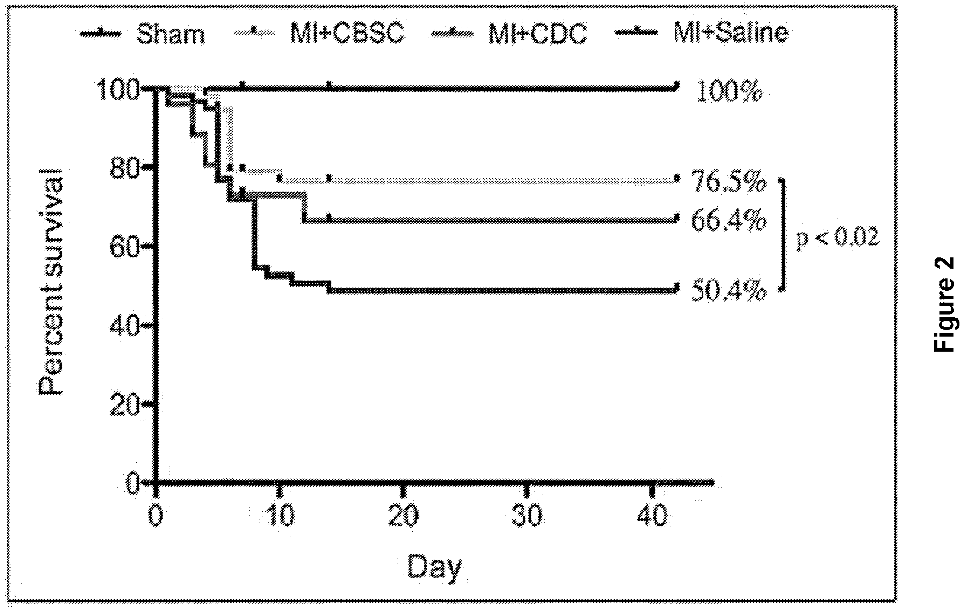

The effects of CBSCs versus CDCs on post-MI structural and functional remodeling were studied. The MI procedure reduced 6-week survival to 50.4% in animals receiving sham saline injections. Animals receiving MI and CBSC therapy demonstrated a 76.5% 6-week survival, which was greater than CDC-treated animals and significantly greater than the saline-treated controls (Figure 2). Animals receiving CDC therapy did demonstrate an improvement in survival relative to saline-treated MI controls (66.4%), but this improvement was not statistically significant. Both stem cell therapies improved cardiac function and attenuated adverse cardiac remodeling characteristic of MIs in this model system (Figure 3), and these changes were most pronounced in the MI+CBSC group. Animals receiving MI+CBSC had significantly improved ejection fraction and fractional shortening compared to the MI+Saline control animals as early as 1 week post-MI, and these changes were sustained through 6 weeks post-MI. Improvements in ejection fraction and fractional shortening were greater in CBSC treated animals versus CDC treated animals. Both stem cell treatment groups had an initial decline in stroke volume (SV), but SV returned to normal by 2 weeks post-MI and remained significantly improved relative to MI+Saline control animals. Animals in the MI+Saline group demonstrated significant increases in end-diastolic and systolic volumes with thinning of the infarct-affected anterior wall (Figure 3). By contrast, MI animals treated with CBSCs or CDCs had significantly smaller post-MI diastolic and systolic volumes at all time points studied, and they demonstrated attenuated thinning of the anterior wall relative to saline-injected controls, with CBSC treated animals demonstrating the most profound attenuation across all parameters (Figure 3).

Five mice were randomly selected from each group (MI+Saline, MI+CDC, or MI+CBSC) to undergo acute infarct size analysis at 24 hours post-MI, and no significant difference between the area at risk (AAR) or ischemic area (IA) was detected between animals in any group (Figure 13). These results demonstrate that acute infarct size was similar in all study animals. Therefore, the structural and functional improvements in the MI+CBSC and MI+CDC groups can be attributed to the effects of cell transplantation alone and are not the result of a stem cell mediated reduction in initial infarct size. After 6 weeks post-MI, chronic infarct size was analyzed by measuring the area of infarcted tissues relative to total myocardial area on hematoxylin and eosin (H&E)-stained cross sections from saline-, CDC-, or CBSC-injected hearts. Animals receiving either CDC or CBSC therapy had significantly smaller infarcted areas relative to saline-treated controls, with CBSC-treated animals demonstrating the most significantly reduced infarct sizes, suggesting that infarct size or expansion were reduced 6 weeks after CBSC therapy.

### Contractile myocardium was detected at CBSC injection sites

Strain analysis was performed on long-axis B-mode images to determine if regions injected with CBSCs developed contractile activity. Figure 4A shows representative three-dimensional wall velocity diagrams for 3 consecutive cardiac cycles taken from animals at baseline, 6 weeks post-MI+Saline, and 6 weeks post-MI+CBSC. Figure 14 outlines how 3D wall velocity diagrams are constructed relative to the acquired parasternal long-axis B-mode echocardiogram of the left ventricle. Points along the left ventricular (LV) endocardial surface are plotted along the x-axis, from the base of the posterior wall to the apex to the base of the anterior wall. At baseline, all hearts demonstrated uniform and synchronous contraction and relaxation across the LV endocardium (Figure 4A). In the MI+Saline control group after 6 weeks, there was a dramatic reduction in wall velocity across the endocardium of the infarct-related anterior wall (Figure 4A). CDC-treated animals demonstrated some improvements in wall velocity at the infarct border zone, while CBSC-treated MI animals demonstrated even greater improvements in wall velocity at the infarct border zone segments where CBSCs were injected (Figure 4A).

Figure 4B shows vector diagrams taken from long-axis B-mode echocardiograms. It was also observed that normal hearts exhibited uniform, synchronous contractions and relaxations throughout the cardiac cycle. Animals with MI injury showed pronounced LV chamber dilation, wall thinning, and hypokinesis of the infarcted wall, as evidenced by the lack of vector activity in this region. In animals receiving stem cell therapy, the LV chamber was less dilated, with attenuated wall thinning and increased contractile activity in the border zone segments that received cell therapy. While CDC-treated animals did show some improvements in wall velocity and strain at the border zone segments, CBSC-treated animals had much greater wall velocities and contraction vectors nearly equal in magnitude to baseline control hearts at the infarct border zone segments where CBSCs were injected.

Global averages of LV endocardial strain and strain rate are plotted in Figure 4C. Strain, which measures change in length relative to the initial length (Strain = Final Length [L]/Initial Length [L₀]) was measured either in the radial (from the center of the ventricle cavity outward) or longitudinal axis (from the apex to the base). The rate of change in strain (Strain Rate = Strain/Time) was also calculated. In both axes, strain and strain rate were significantly reduced following MI. Significant improvements in both strain and strain rate in both the radial and longitudinal axes were observed in MI animals receiving CBSC or CDC therapy relative to MI+Saline controls, and CBSC-treated animals showed improved strain relative to CDC animals across all parameters at all time points. These data demonstrate that CBSC-treated hearts had significantly improved global and regional contractility by 6 weeks post-MI.

### Expression of pro-angiogenic paracrine factors in vivo by stem cells induces new blood vessel formation

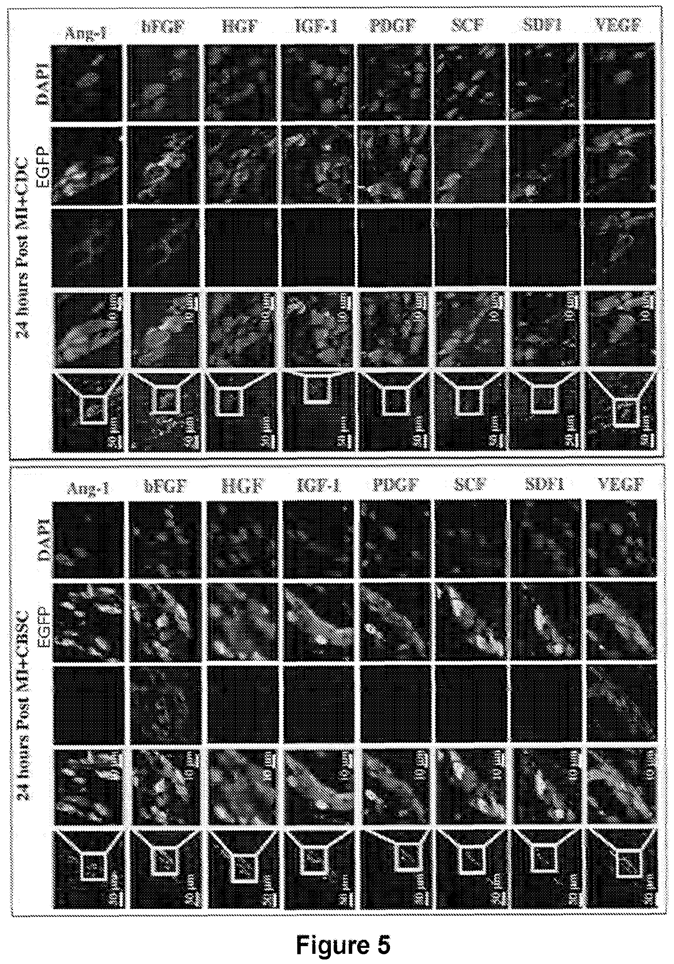

The environment in the infarct border zone is dramatically different from that in the tissue culture dish. Therefore, paracrine factor expression and production are likely to change after transplantation of CBSCs into the infarcted heart. At 24 hours post-MI, hearts receiving CBSC transplants were immunostained for the 8 paracrine factors studied *in vitro.* Injection sites containing EGFP+ CBSCs were identified and found to express bFGF and VEGF, but Ang-1, IGF-1, HGF, or PDGF were not detected at any injection sites examined (Figure 5). Injection sites containing EGFP+ CDCs were found to express Ang-1 in addition to bFGF and VEGF, but the 5 other factors were not detected. These studies suggest that, when injected into the MI border zone, CBSCs express only bFGF and VEGF (and CDCs express only Ang-1, bFGF, and VEGF). Despite expressing an additional paracrine factor after 24 hours, expression of all paracrine factors by CDCs was not sustained (Table 3). No amounts of Ang-1, bFGF, or VEGF could be detected in any CDC injection sites after 24 hours post-MI (data not shown).

VEGF expression was detected in CBSC injection sites as late as 2 weeks post-MI (Figure 15). The fluorescence intensity tracings of each color channel (green = EGFP, red = VEGF, blue = DAPI) across a confocal line scan of the cell selected in Figure 15A demonstrates the colocalization of the VEGF and EGFP signals (Figure 15B). A scatterplot of pixel intensities (Figure 15C) in the red channel versus the green channel was generated, and the diagonal deflection documents colocalized red/green pixels (56.2% of green and red pixels in this line scan were colocalized). A control scatterplot of the blue (DAPI) versus green channel (EGFP) shows only 13.6% of blue/green pixels were colocalized (Figure 15D). Paracrine factors involved in cardioprotection or homing and recruitment of endogenous stem cells (HGF, IGF-1, SCF and SDF-1) were not detected at any time point *in vivo.* An overview of all *in vitro* and *in vivo* paracrine factor analyses for CBSCs can be found in Table 2, while a summary of CDC paracrine factor analyses is shown in Table 3.

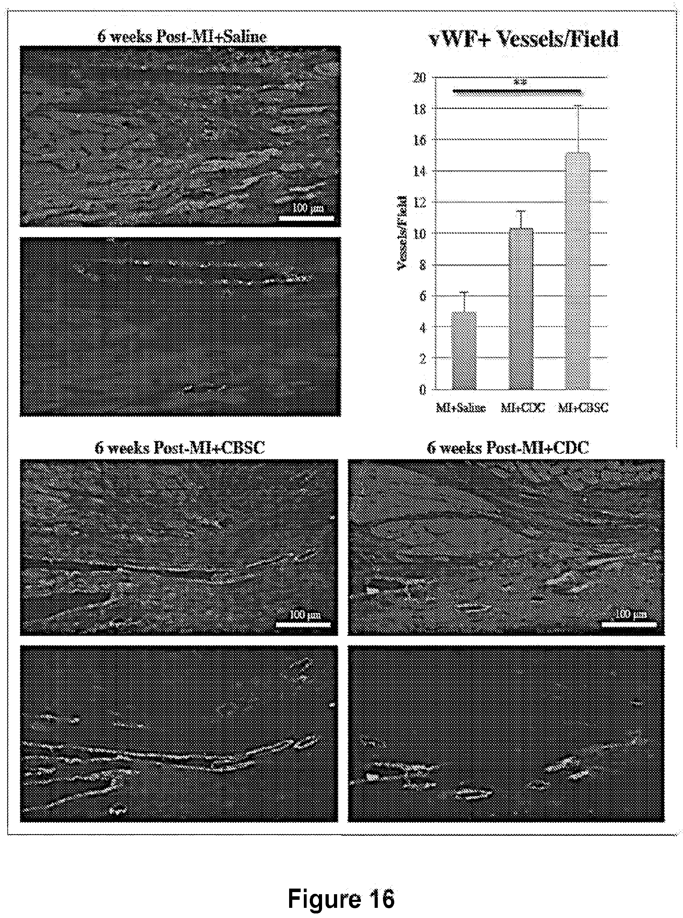

To determine whether the *in vivo* expression of the CBSC-secreted pro-angiogenic factors bFGF and VEGF (and Ang-1 secreted by CDCs) resulted in increased neovascularization, blood vessel density was measured in the infarct zones from MI animals treated with saline, CDCs or CBSCs (Figure 16). MI animals receiving CBSC treatment had 15.13±3.04 von Willebrand factor (vWF)-positive blood vessels (purple) per 20X field of view and MI animals receiving CDC therapy had 10.13±1.13 vWF+ vessels per field, while saline-treated controls had only 4.95±1.30 vWF+ vessels per field. These data support the idea that secretion of pro-angiogenic paracrine factors *in* vivo by both stem cell types, and especially by CBSCs, induced increased neovascularization. Increased blood supply to the injured heart could have contributed to the functional benefits we observed following CBSC transplant. The majority of these blood vessels did not contain EGFP+ cells, suggesting that they were derived via endogenous repair rather than from the injected cells.

**Table 2: Summary of paracrine factors produced by cortical bone stem cells as measured by FACS, Western, ELISA, and immunostaining in vitro and in vivo**

| | Western | ELISA | Staining: | | | |
|---|---|---|---|---|---|---|
| | | | *In vitro* | 24 hr | 1 week | 2 week |
| AP-1 | + | N/A | + | - | - | - |
| bFGF | + | N/A | + | + | - | - |
| HGF | + | - | + | - | - | - |
| IGF-1 | + | + | + | - | - | - |
| PDGF | + | N/A | + | - | - | - |
| SCF | + | - | + | - | - | - |
| SDF-1 | + | +++ | + | - | - | - |
| VEGF | + | ++ | + | + | + | + |

**Table 3: Summary of paracrine factors produced by cardiac-derived stem cells as measured by FACS, Western, ELISA, and immunostaining in vitro and in vivo**

| | Western | ELISA | Staining: | | | |
|---|---|---|---|---|---|---|
| | | | *In vitro* | 24 hr | 1 week | 2 week |
| AP-1 | + | N/A | + | + | - | - |
| bFGF | + | N/A | + | + | - | - |
| HGF | + | - | + | - | - | - |
| IGF-1 | + | + | + | - | - | - |
| PDGF | + | N/A | + | - | - | - |
| SCF | + | - | + | - | - | - |
| SDF-1 | + | +++ | + | - | - | - |
| VEGF | + | ++ | + | + | - | - |

### EGFP+ CBSCs expand over time as cells proliferate and CBSCs transdifferentiate into five distinct phenotypes

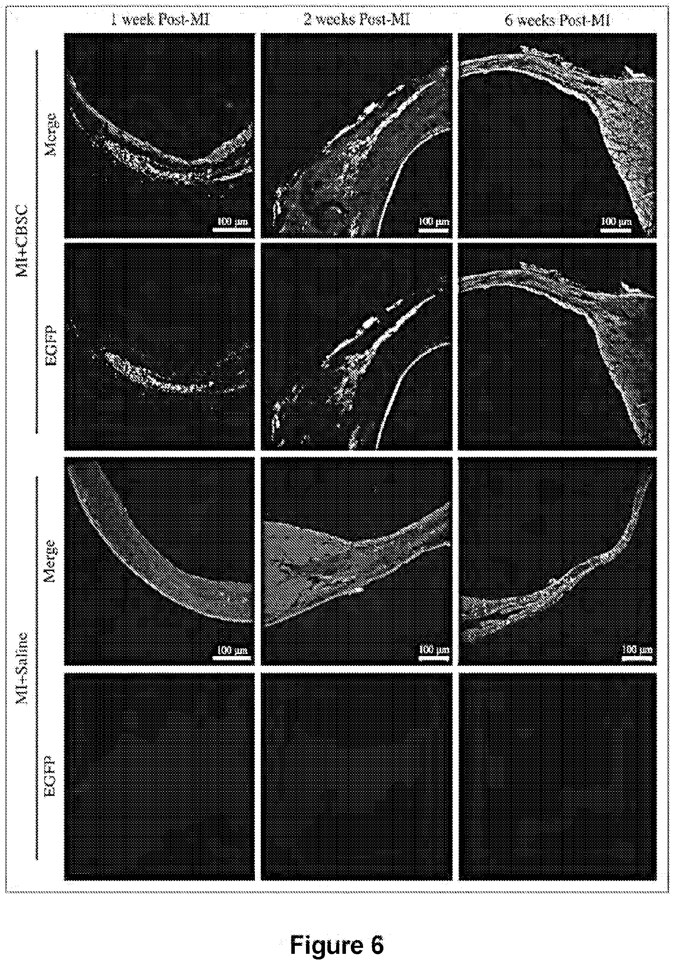

CBSC injection sites were examined 1, 2 and 6 weeks after MI (Figure 6). Injection sites were identified after immunostaining against α-sarcomeric actin and EGFP. Histological samples from CBSC- and saline-injected hearts were stained simultaneously, and the absence of EGFP signal in the saline-injected controls was confirmed, documenting a very low level of autofluorescence under the conditions employed. All tissue sections were first examined under low magnification to identify injection sites and then high magnification confocal images were analyzed. At 1-week post-MI, the majority of EGFP+ cells were found in discrete groups of small, round, poorly differentiated cells. By 2 weeks post-MI, the area of the heart with EGFP+ cells had expanded and the majority of these EGFP+ cells were enlarged and had an elongated appearance. By 6 weeks Post-MI, injected stem cells had spread throughout the infarct zone and into the infarct border zone and discrete injection sites were no longer visible. Six weeks after injection, the EGFP+ stem cells were spread throughout the border zone and infarct zone.

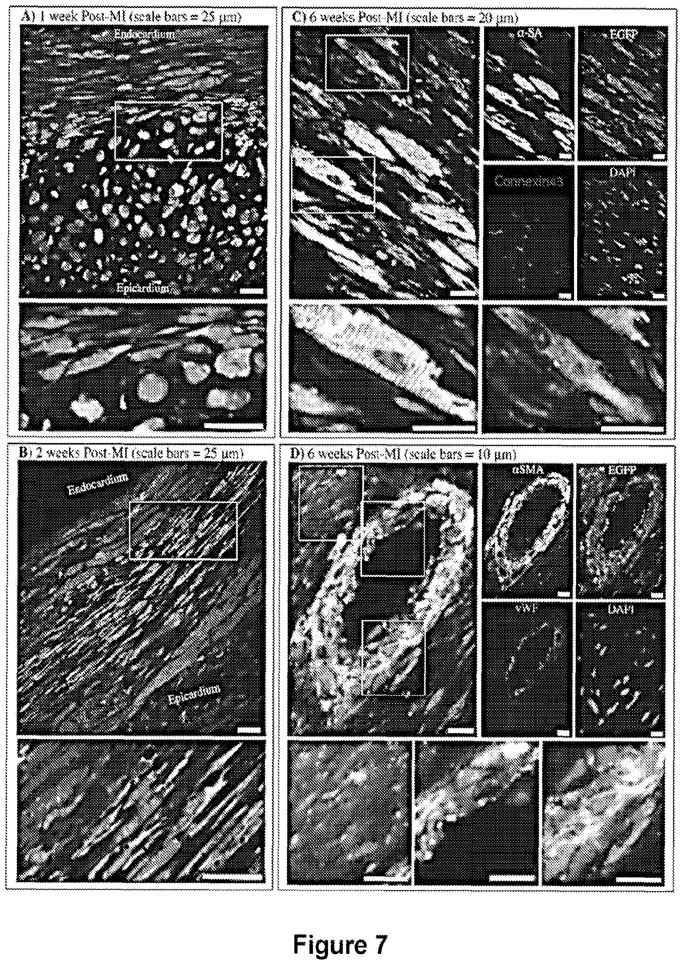

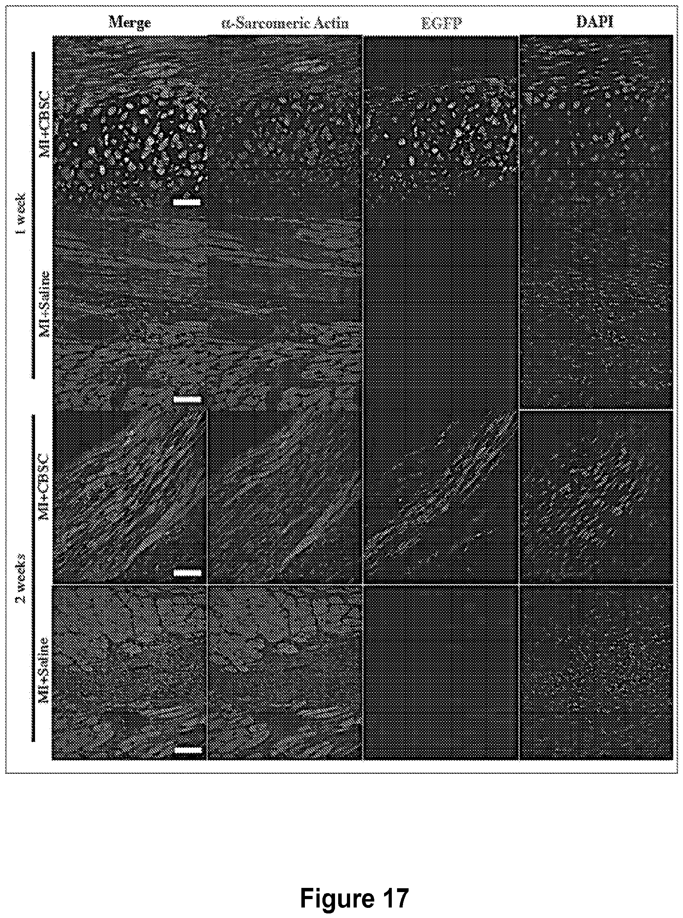

Time-related changes in CBSC phenotype were studied. After 1 week, most EGFP+ cells were still small (10-20 µm) and round in shape (similar to the morphology of the injected cells), but the majority had begun to express unorganized cytosolic α-sarcomeric actin (Figure 7A). A small number of cells that were in close contact with viable endogenous myocytes began to elongate and align in the axis of contraction, as defined by the sarcomeric organization of surviving myocytes in the region. By 2 weeks post-MI, as the injection sites had begun to expand, the majority of cells were elongated, aligned along the axis of contraction, and were expressing additional cytosolic α-sarcomeric actin (Figure 7B). As at 1 week, EGFP+ cells that had engrafted closest to viable endogenous myocytes appeared the largest and most mature, with even greater amounts of α-sarcomeric actin visible in their cytoplasm. Although actin expression appeared to be increased by 2 weeks post-MI, it was still relatively unorganized, without the characteristic sarcomeric striations of adult myocytes. Individual color channel images for Figure 7A and B and staining controls for 1 and 2 week post-MI+Saline are shown in Figure 17.

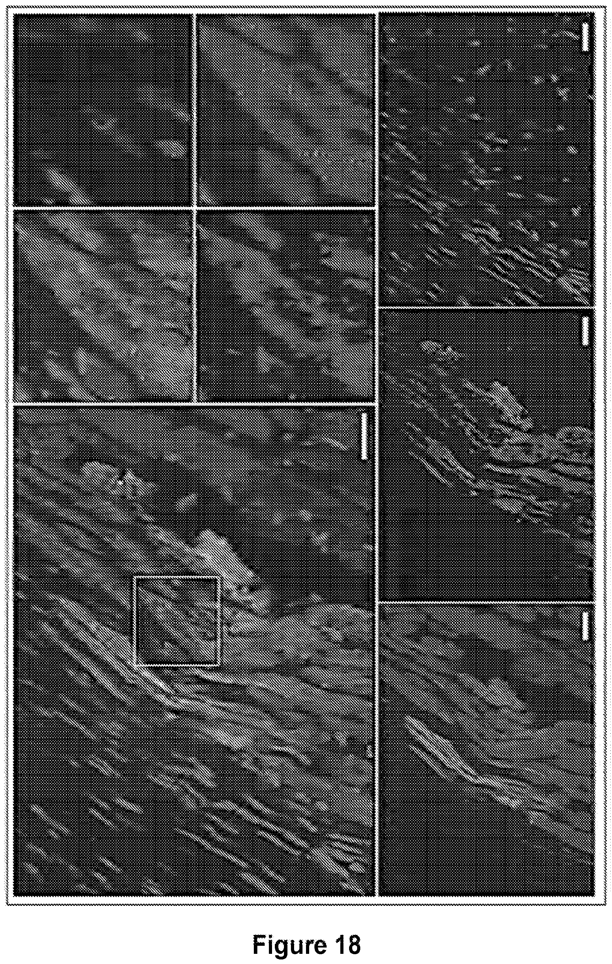

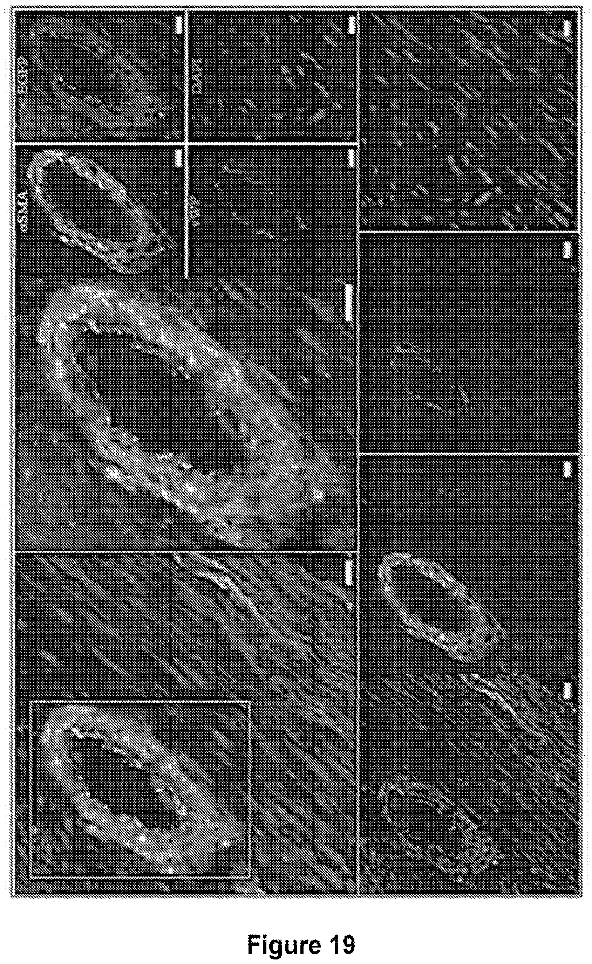

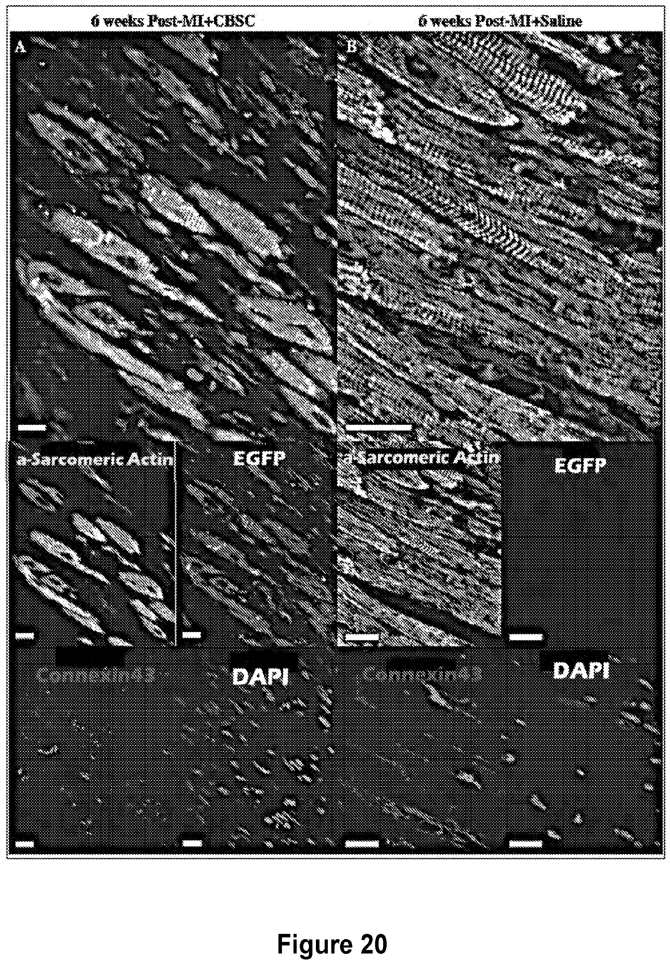

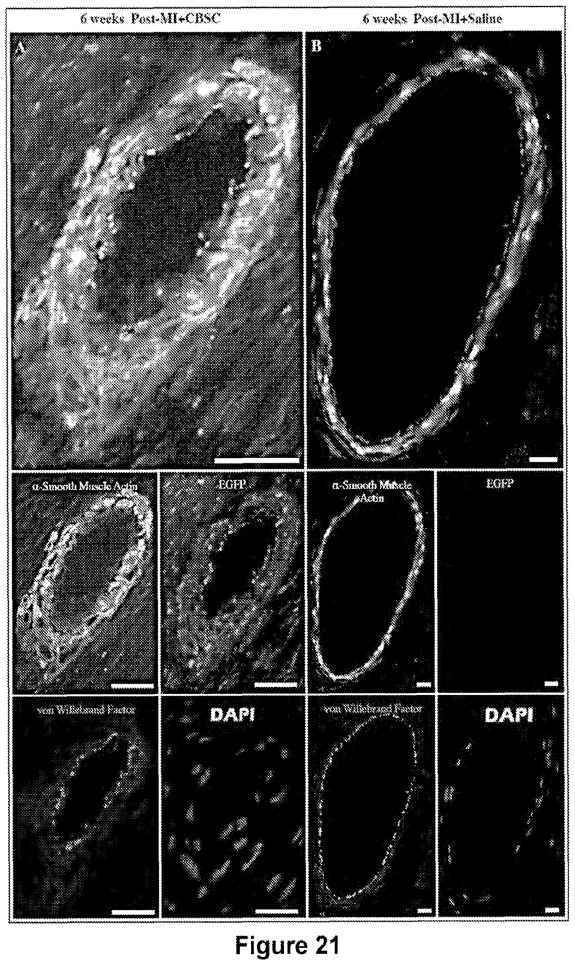

By 6 weeks Post-MI, newly formed EGFP+ myocardium was visible at the border zone and five distinct EGFP+ stem cell phenotypes were identified: 1) cardiac myocytes with α-sarcomeric actin in organized sarcomeres were found to be coupled to neighboring cells via connexin43+ gap-junctions (Figure 7C), 2) cells that express unorganized α-sarcomeric actin (Figure 7C), 3) vascular smooth muscle cells that stain positive for α-smooth muscle actin (Figure 7D), 4) vascular endothelial cells that stain positive for von Willebrand factor (Figure 7D), and 5) EGFP+ cells that are smaller (<20 µm) and lack any expression of adult cardiomyocyte or vascular cell proteins (Figure 7D). Figure 18 shows an additional low-magnification image of the CBSC-treated border zone at 6 weeks post-MI in which a newly formed region of EGFP+ myocardium can be seen adjacent to a region EGFP- endogenous myocardium. Figure 19 shows a low magnification view of the blood vessel shown in Figure 7D, in which the EGFP+ vessel is clearly surrounded by regions of EGFP-myocardium. Individual confocal color channel images and staining controls for Figure 7C are shown in Figure 20. Individual color channel images and staining controls for Figure 7D are shown in Figure 21. These results support the idea that CBSCs can differentiate into the major cell types of the adult heart.

### Cardiac-derived stem cells expand and proliferate over time but adopt a less mature adult myocyte phenotype

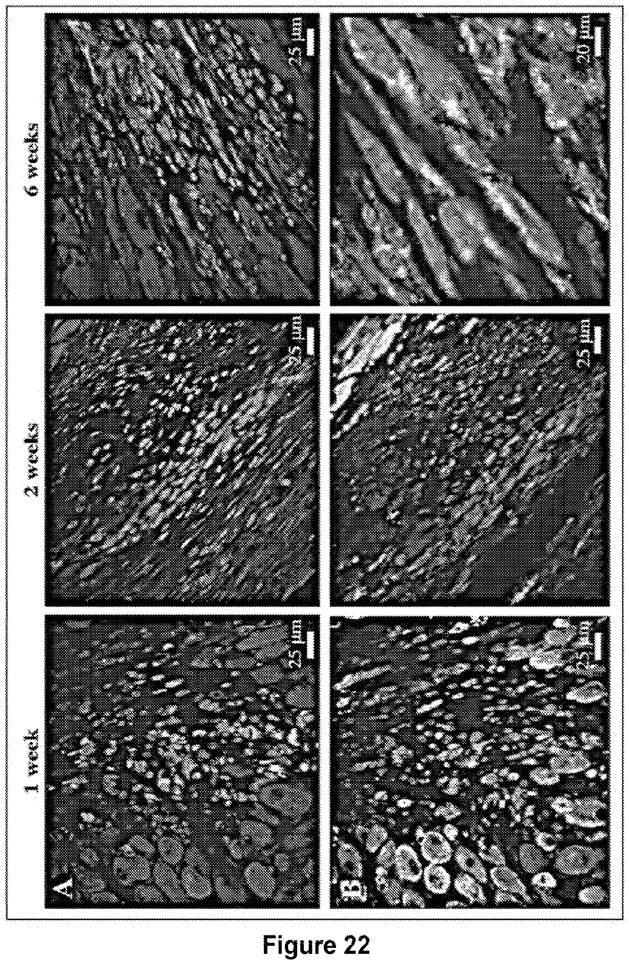

Figure 22 shows images from the hearts of CDC-treated MI animals studied at 1, 2, and 6 weeks post-MI. EGFP+ CDC injection sites were identified and stained for α-sarcomeric actin in Figure 22A, or for α-sarcomeric actin (white) and connexin43 in Figure 22B. Similar to what was observed with CBSCs, by 1 week post-MI CDCs initially appear small (10-20 µm) and round and they had begun to express unorganized α-sarcomeric actin. By 2 weeks, the cells have begun to align and elongate like CBSCs and they continue to express unorganized α-sarcomeric actin. By 6 weeks post-MI, however, no EGFP+ cells with organized sarcomeres were found. Cells continue to grow larger and are still aligned, and some had even begun to express connexin43 gap junctions (Figure 22B, right panel), but cytosolic α-sarcomeric actin was not yet striated and mature gap junctional plaques were not identified. These data suggest that under these conditions, CDCs adopted a less mature cardiomyocyte phenotype over the 6-week course of this study.

### CBSC-derived EGFP+ myocytes isolated 6 weeks after injection demonstrated mature contractile properties

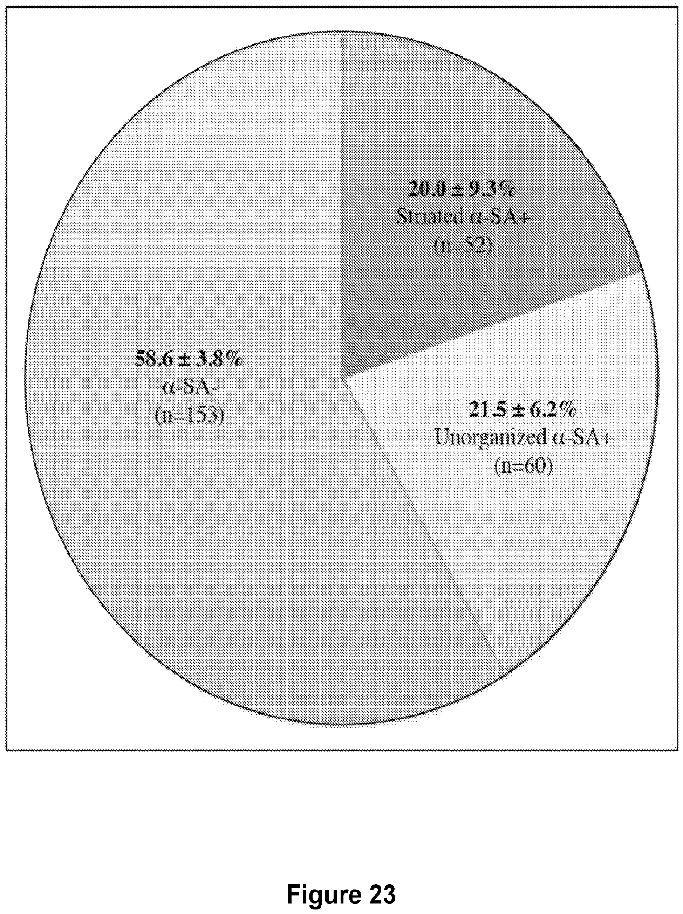

Some animals that received MI and CBSC therapy were sacrificed 6 weeks after injury and myocytes from their left ventricles were isolated and studied *in vitro.* Isolated myocytes were also immunostained for α-sarcomeric actin, EGFP and nuclei were labeled with DAPI in order analyze myocyte size and the number of nuclei per cell (Figure 8). Of all the myocytes in the left ventricle, an estimated 0.84% expressed EGFP, suggesting that these cells had been derived from transplanted CBSCs. In order to estimate how many new myocytes were formed from EGFP+ CBSCs (assuming that 0.84% of isolated myocytes were EGFP+), the total number and volume of myocytes in the heart was approximated. The total number of ventricular myocytes in the feline heart has been previously estimated (Chen et al., 2007, Circ Res 100:536-544) by measuring the average volume of the heart and the average volume of a myocyte. The mean weights of the mouse ventricles 6 weeks post-MI+CBSC = 205.3±10.8 mg and the average volume of a mouse myocyte can be approximated using average myocyte dimensions (100 x 20 x 10 µm = 20,000 µm³ = 2x10⁻⁸ mL). If the density of myocardial tissues = 1.06 mg/mm³ (Anversa et al., 1985, Am J Physiol 248:H876-882), then the average volume of hearts at 6 weeks post-MI+CBSC = 205.3/1.06 = 193.7 mm³ = 0.1937 mL. Myocytes are known to make up about 80% of the heart by volume (Legato, 1979, Circ Res 44:263-279), so the volume of the heart constituting myocytes = 0.8 x 0.1937 = 0.1550 mL. Thus, the total number of new EGFP+ myocytes in the average mouse heart at 6 weeks post-MI+CBSC = 0.1550 mL/2xl⁻⁸ mL = 7.75x10⁶ total myocytes/heart. If 0.84% of myocytes analyzed were positive for EGFP, then 0.0084 x 7.75x10⁶ = 65,100 new EGFP+ myocytes were formed per heart. At the time of surgery, 40,000 cells were initially injected. To form this number of new myocytes the injected CBSCs must have proliferated and committed to the cardiac lineage. As shown in Figure 7, only a fraction of the EGFP+/a sarcomeric actin+ cells were striated. The relative numbers of striated (muture) and nonstriated (less mature) EGFP+/a-sarcomeric actin+ cells were measured in 6 week post MI CBSC hearts (Figure 23). This histological analysis showed that there were approximately equal numbers of striated and nonstriated EGFP+/α-sarcomeric actin+ new myocytes. Collectively these analyses suggest that the number of CBSC-derived new myocytes (about 130,000) is about 3X greater than the number of cells originally injected (40,000).

EGFP+ isolated myocytes were identified, and the size and number of nuclei per cell were analyzed and compared to EGFP- myocytes isolated from the same hearts. Figure 8A shows a representative EGFP+ myocyte with two nuclei. When compared to EGFP- controls, EGFP+ myocytes had smaller average cross-sectional surface area (Figure 8B). Over 90% of EGFP- myocytes were binucleated, while only 5.4% of EGFP- cells were mononucleated and 4.1 % were tetranucleated. In contrast, there were significantly more mononucleated and significantly fewer binucleated EGFP+ myocytes, with 1/3 EGFP+ myocytes having only one nucleus (Figure 8B). There was no significant difference in tetranucleation between EGFP+ and EGFP- myocytes.

Isolated EGFP+ and EGFP- myocyte fractional shortening (FS) and Ca²⁺ transients were measured. Figure 8C shows representative EGFP+ and EGFP-myocytes under fluorescence or bright field histology with their corresponding FS or Ca²⁺ transients. Mean FS, peak Ca²⁺ (F/F0) and time constant of decay of the Ca²⁺ transients were measured (Figure 8D). EGFP+ myocytes isolated 6 weeks after MI had contractions and Ca²⁺ transients that were indistinguishable from EGFP- controls, documenting that they had assumed a mature adult phenotype.

### Compact bone stem cells

The present study demonstrates the beneficial effects of transplanting a novel population of c-kit+/Sca-1+ cells from the stem cell niches within the bone, rather than from bone marrow, into the border zone of a myocardial infarction, and the effects of this novel cell type were compared to a widely studied population of c-kit+/Sca-1+ cardiac-derived stem cells. The present results show that CBSCs have beneficial effects on the structure and function of the heart after MI. Animals with MI that received a CBSC transplant had improved 6-week survival, cardiac function, and attenuation of adverse left ventricular remodeling compared with both saline-injected MI controls and CDC-treated animals. Strain analysis demonstrated that CBSC-treated MI animals not only had improved global function, but their hearts also had greater contractile function at the MI border zone relative to saline- and CDC-injected hearts. Strain analysis allows for precise imaging at the infarct border zone, where stem cells were specifically administered to achieve optimal effect (Duran et al., 2012, Clin Transl Sci 5:416-421). At border zone segments where new myocardium was detected using histological techniques, strain analysis of CBSC-treated mice showed greater contraction (Figure 4A-B), suggesting that the stem cells enhanced contractile function in the region where they were injected. Histological analysis showed that, while CDCs adopted less mature adult phenotypes, EGFP+ stem cell-derived adult cardiac myocytes with normally striated α-sarcomeric actin networks and connexin43+ gap junctions were present throughout the border zone in CBSC treated mice (Figure 7C). CBSC-derived new cardiac myocytes that were isolated from hearts 6 weeks after MI had normal contractions and Ca²⁺ transients. These findings support the idea that newly formed cardiac muscle was derived from injected CBSCs and these cells make a contribution to the improved contractile function of the injured heart.

### CBSCs secrete pro-angiogenic factors and promote neovascularization

The experiments described herein explored the paracrine effects of injected CBSCs. Many studies have looked at stem cell paracrine factors *in vitro* and drawn conclusions without studying the changes in expression of these factors after injection into the heart (Li et al., 2012, J Am Coll Cardiol 59:942-953). Other groups have looked at the changing paracrine factor expression by stem cells after injection, but they have either studied total myocardial expression of these factors (Kamihata et al., 2001, Circulation 104:1046-1052) (rather than expression specifically by stem cells) or they have only examined specific stem cell expression of a single factor (Kinnaird et al., 2004, Circ Res 94:678-685). The present study establishes *in vitro* expression of multiple paracrine factors and examines the temporal changes in expression of all of these factors specifically by the stem cells after transplantation (Li et al., 2012, J Am Coll Cardiol 59:942-953; Gnecchi et al., 2005, Nat Med 11:367-368; Gnecchi et al., 2006, FASEB J 20:661-669; Gnecchi et al., 2008, Circ Res 103:1204-1219; Zsebo et al., 1990, Cell 63:213-224; Kinnaird et al., 2004, Circ Res 94:678-685; Kinnaird et al., 2004, Circulation 109:1543-1549; Mirotsou et al., 2007, Proc Natl Acad Sci USA 104:1643-1648). Neovascularization and the presence of paracrine factors in regions with injected CBSCs was specifically explored. The present studies showed that CBSCs produced the pro-angiogenic factors bFGF and VEGF (both *in vitro* and *in vivo*). These factors are known to be involved in vascular cell proliferation and induction of angiogenesis (Gnecchi et al., 2005, Nat Med 11:367-368; Gnecchi et al., 2006, FASEB J 20:661-669; Gnecchi et al., 2008, Circ Res 103:1204-1219; Williams et al., 2011, Circ Res 109:923-940; Kinnaird et al., 2004, Circ Res 94:678-685; Kinnaird et al., 2004, Circulation 109:1543-1549; Caplan and Dennis, 2006, J Cell Biochem 98:1076-1084), and there was evidence of increased neovascularization in the infarct zone of hearts treated with stem cells in this study (Figure 12). Although CDCs initially produced Ang-1 in addition to bFGF and VEGF, these factors were not observed past 24 hours post-MI and the neovascularization of the border zone in CDC treated animals was less robust than CBSC-treated animals by 6 weeks post-MI. Additionally, the present studies showed that, while CDCs did not transdifferentiate into mature cardiac myocytes or blood vessels by 6 weeks post-MI, CBSCs transdifferentiated into adult vascular cells, including smooth muscle and endothelial cells, although this was observed relatively infrequently. While there was strong evidence of myocyte transdifferentiation (EGFP+ cardiac myocytes were identified in 5/8 hearts analyzed 6 weeks post-MI), and many cells from hearts at earlier time points could be identified in intermediate stages of myocyte differentiation (in 6/6 hearts analyzed after 1 week post-MI+CBSC and in 6/6 analyzed fixed at 2 weeks post-MI+CBSC), the same could not be said for cells of the vascular lineage (EGFP+ vascular cells were only identified in 2/8 hearts analyzed at 6 weeks post-MI and no intermediate EGFP+ lumen or tube like structures were found at 1 or 2 weeks post-MI). These findings demonstrate that paracrine-induced new blood vessel formation via endogenous repair appears to be the primary mechanism for the enhanced angiogenesis observed in CBSC-injected hearts. The *in vivo* paracrine factor analysis showed those factors involved in cardioprotection (HGF, IGF) or stimulation of endogenous stem cells (SCF, SDF-1) that were expressed in CBSCs *in vitro* were not found in CBSCs after they were injected into the MI border zone. These results suggest that part of the beneficial effect of CBSCs on MI hearts is paracrine-mediated enhancement of angiogenesis.

### CBSCs differentiate into new cardiac myocytes

In the present study CBSCs and CDCs from an EGFP+ mouse were used so that the fate of the injected cells could easily be traced. Using time course *in vivo* histological analysis with this stably expressed fluorophore, it was able to be demonstrated that CBSCs differentiated over time down the myocyte lineage by 6 weeks post-MI while CDCs did not adopt a fully mature cardiac myocyte phenotype. As discussed elsewhere herein, evidence was found for enhanced revascularization of the MI border and infarct zone in CBSC injected hearts. However, the new blood vessels were rarely comprised of EGFP+ cells, suggesting that CBSCs enhanced endogenous repair. The present experiments demonstrate that EGFP+ CBSCs express cardiac proteins soon after injection into the MI heart, and by 6 weeks post-MI these cells have organized sarcomeres, are connected to their neighbors via gap junctions, and contract with Ca²⁺ transients that are similar to those of EGFP- myocytes isolated from the same hearts.

The studies described herein demonstrate that the transition from an injected CBSC to a fully functional cardiac myocyte takes between 2-6 weeks under the present conditions. After one week, most the EGFP+ cells had immature characteristics and expressed unorganized cardiac contractile proteins. Two weeks after MI, areas with EGFP+ cells were larger, suggesting cell proliferation, and the cells had begun to elongate along the axis of EGFP- myocytes that had survived the infarct. More EGFP+ cells expressed cardiac contractile proteins but no organized sarcomeres were found. By 6 weeks post-MI there were many regions containing EGFP+ myocytes with organized sarcomeres and these cells were well integrated in the myocardium via gap junctions. The contribution of these new myocytes to the improved cardiac contractile performance of the post-MI heart can be further understood with additional undue experimentation. However, regional strain measurements from sites of CBSC injection documented increased contractile performance in the regions where EGFP+ myocytes were isolated with robust contractile activity. These findings demonstrate that CBSC-mediated new myocyte formation is at least partially responsible for improvements in cardiac structure and function in MI hearts with CBSC treatments.

The major evidence for CBSC-derived new myocyte formation is that EGFP+ cardiac myocytes were found in the MI border zone of hearts 6 weeks after CBSC injections. There is always concern that injected cells might have fused with existing myocytes (Nygren et al., 2004, Nat Med 10:494-501), but little or no evidence for fusion in these types of experiments has been observed (Bearzi et al., 2007, Proc Natl Acad Sci USA 104:14068-14073; Beltrami et al., 2003, Cell 114:763-776), and complex studies using multiple stem cell labeling strategies have largely contradicted the fusion hypothesis (Rota et al., 2007, Proc Natl Acad Sci USA 104:17783-17788). The present study also demonstrates that the EGFP+ myocytes that were found were newly formed from smaller CBSC progeny. These experiments showed that EGFP+ isolated myocytes were smaller and a higher percentage of these cells were mononucleated, consistent with a maturing adult cardiac myocyte. The number of new, CBSC-derived EGFP+ myocytes was estimated by isolating myocytes from hearts six weeks after CBSC injection into the MI border zone. It was estimated that the number of new myocytes significantly exceeds the number of injected CBSCs, consistent with proliferation of either CBSCs or newly formed myocyte progeny. Importantly, while not bound to any particular theory, this approach may have significantly underestimated the number of new myocytes because it is more difficult to isolate viable myocytes from infarct zones with fibrotic regions than from undamaged regions of the same heart. The present data are also consistent with previously published reports which demonstrate that newly formed myocytes were detected in the growing heart during adolescence (Chen et al., 2007, Circ Res 100:536-544). These results are also similar to observations made in several studies by the Anversa laboratory, in which newly formed isolated myocytes were observed to be smaller than spared endogenous myocytes that lacked the stem cell marker (Beltrami et al., 2003, Cell 114:763-776; Rota et al., 2008, Circ Res 103:107-116; Rota et al., 2007, Proc Natl Acad Sci USA 104:17783-17788). Collectively the results presented herein demonstrate that CBSCs survive in the MI border zone and within weeks they begin to form new cardiac tissue. The survival and differentiation of CBSCs was associated with improved cardiac structure and function and enhanced survival.

### Other CBSC cardioprotective effects

The experiments described herein demonstrate improved cardiac structure and function in the first two weeks after MI, before injected cells had enhanced vascularity and local contractility. The mechanisms of these early beneficial effects are not clear. While not wishing to be bound by any particular theory, one possibility is that injected cells thicken and stabilize the infarcted wall, thereby reducing wall stress and slowing dilation. These results are consistent with early stage clinical trials with wall stabilizing agents (Landa et al., 2008, Circulation 117:1388-1396; Segers and Lee, 2011, Circ Res 109:910-922; Kleinman et al., 2005, Semin Cancer Biol 15:378-386; Kofidis et al., 2005, Circulation 112(9 Suppl):I173-177). The cardiac functional data of MI animals treated with CDCs and CBSCs also provide some insight into the wall stabilizing effect of cell therapy. By 1 week post-MI, both the MI+CDC and MI+CBSC groups had similar cardiac functional parameters (ejection fraction and fractional shortening). However, with time the functional improvements in the MI+CBSC group were sustained while the function in MI+CDC animals continued to decline. While not wishing to be bound by any particular theory, the initial functional benefit may be attributed to a wall stabilizing effect that would likely be the same between both cell types, while the sustained functional improvement seen only in the MI+CBSC group could be the result of enhanced paracrine mediated revascularization and maturation of CBSCs into new cardiac tissue.

### CBSCs versus CDCs and other stem cell types

Overall, the present data demonstrates that a bone derived c-kit+/Sca-1+ stem cell population can support the injured heart through direct transdifferentiation into adult cardiomyocytes and vascular cells as well as through secretion of pro-angiogenic paracrine factors. CBSCs proved somewhat more effective at repairing the injured heart than CDCs, in part because CBSCs showed the capacity to transdifferentiate and form cells with a mature adult phenotypes in this animal model. Cortical bone provides an easy-to-access, more abundant source of stem cells that are potentially more pluripotent than has previously been isolated from the bone marrow. Additionally, these stem cells can be isolated from cortical bone in high numbers without the need for arduous sorting processes such as FACS or magnetic bead sorting that are required to isolate the relatively rare populations of c-kit+ cells from the bone marrow or myocardium.

Previously, there has been much skepticism that cells from outside the heart are capable of cardiomyogenesis (Murry et al., 2004, Nature 428:664-668; Laflamme and Murry, 2005, Nat Biotechnol 23:845-856), and many researchers have argued that new myocytes in stem cell treated hearts are derived from paracrine stimulation of endogenous stem cells (Williams et al., 2011, Circ Res 109:923-940; Hatzistergos et al., 2010, Circ Res 107:913-922). The present study demonstrates that bone-derived stem cells can directly form new myocytes, independently of endogenous CDCs, and these new myocytes can be isolated and their contractile properties and Ca²⁺ transients are indistinguishable from endogenous myocytes. Transplanted CDCs in the model failed to produce myocytes with a mature adult phenotypes. Additionally, the described stem cells have shown a wide potential to support the injured heart through several mechanisms without the need of modifications that have been used in other studies (Gnecchi et al., 2005, Nat Med 11:367-368; Gnecchi et al., 2006, FASEB J 20:661-669; Mohsin et al., 2012, J Am Coll Cardiol 60:1278-1287; Quijada et al., 2012, Circ Res 111:77-86).

In summary, the experiments presented herein show that CBSCs can survive the hostile environment of the post-MI heart without modification, secrete factors that enhance endogenous angiogenesis-mediated repair, and differentiate into new cardiac tissue. These beneficial effects culminate in a heart with less structural remodeling and improved cardiac pump function.

### Example 2: Differences between CBSCs, CDCs and MSCs

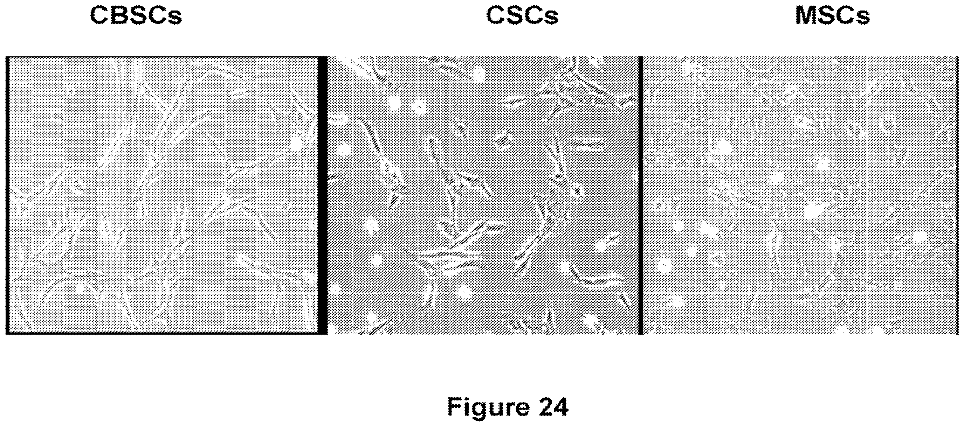

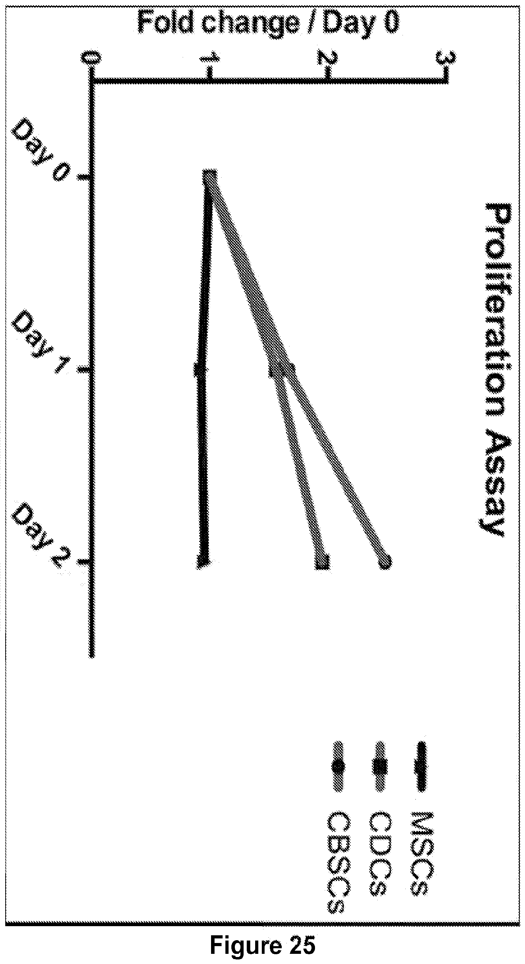

The following experiments were performed to assess the differences between different cell types. Figure 24 shows distinct differences in morphology between three stem cell types (CBSCs, CDCs and MSCs) using bright field imaging. In addition to morphological differences, the different stem cell types exhibited different growth kinetics. For example, enhanced proliferation capacity was observed in CBSCs as compared to CDCs and MSCs as measured by CyQuant assay (Figure 25).

Gene expression profiling was conducted on the three cell types using RT-PCR. It was observed that CBSCs express CD105, CD106, CD73, CD271, CD90, CD133, CD29, CD14, CD44. CD117 was expressed on CBSCs earlier and was lost in culture after few passages. Loss of c-kit expression in culture is also reported by other groups working with stem cells for example Zafir et al. (2012, Stem Cells 31(4): 765-775)) reports loss of c-kit in the classical cKit+ cardiac stem cells. For example, Zafir et al. reported that after isolation and over progressive passages (<10), flow cytometric analyses revealed that the CSCs expressed the stem cell antigen 1 (Sca-1), averaging 79.54 ± 4.5%, but were negative for blood lineage markers, CD31, CD45, were CD341ow and did not retain expression of c-kit after passage. The results presented herein demonstrate that c-kit expression in CBSC's decreased in culture with increasing passage number and is very low and difficult to detect around passage 10. CBSCs were cultured in an expansion medium during this time including Dulbecco's modified Eagle's medium and Ham's F-12 medium [ratio, 1:1], insulin-transferrin-selenium, leukemia inhibitory factor [10 ng/ml], basic fibroblast growth factor [10 ng/ml], epidermal growth factor [20 ng/ml], 10% embryonic stem cells FBS, penicillin-streptomycin-glutamine.

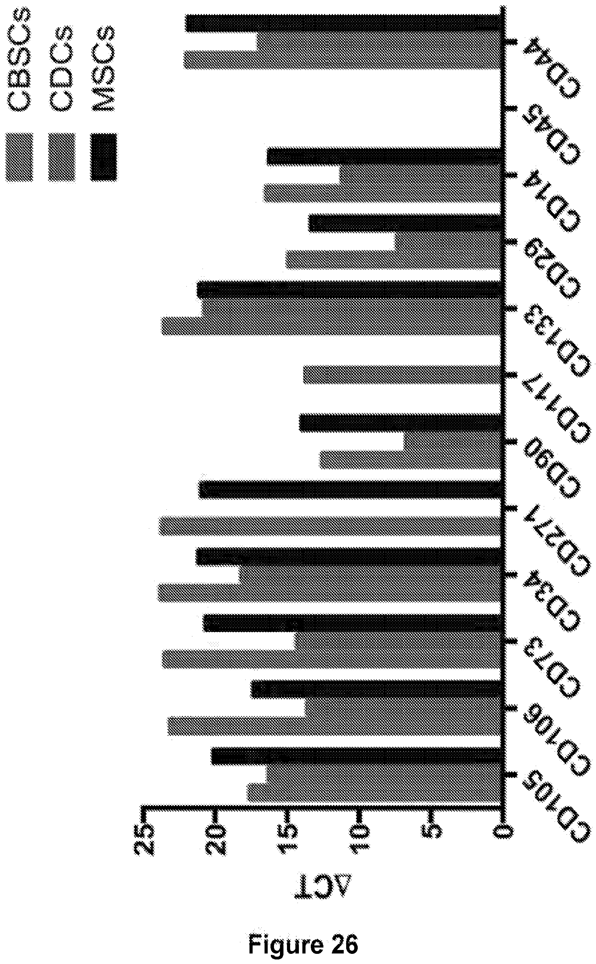

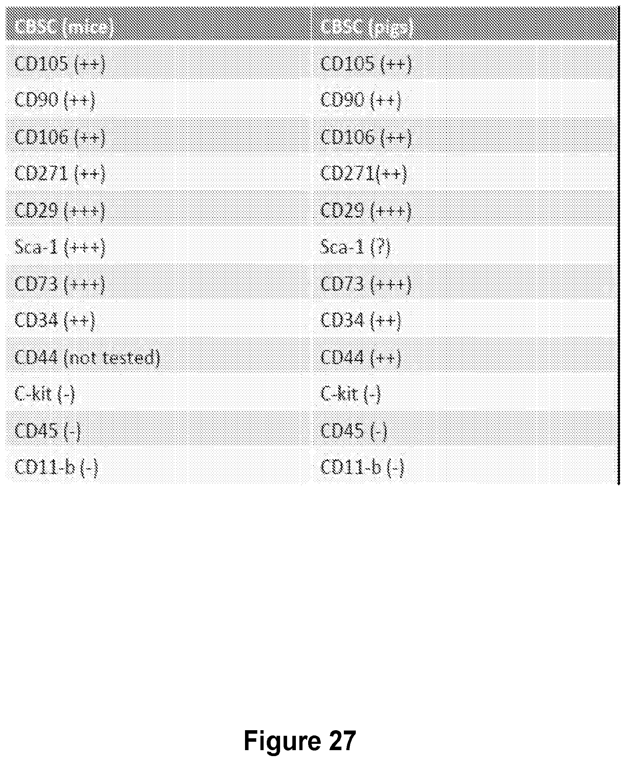

Figure 26 shows that the cell types exhibited different gene expression profiles. Figure 27 summarizes the comparison of gene expression of cell surface markers on CBSCs isolated from different species (mice and pigs) using RT-PCR analysis.

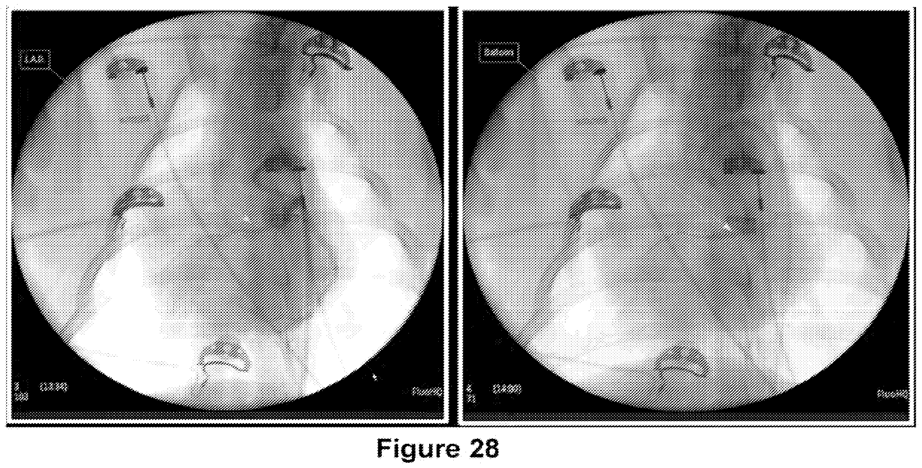

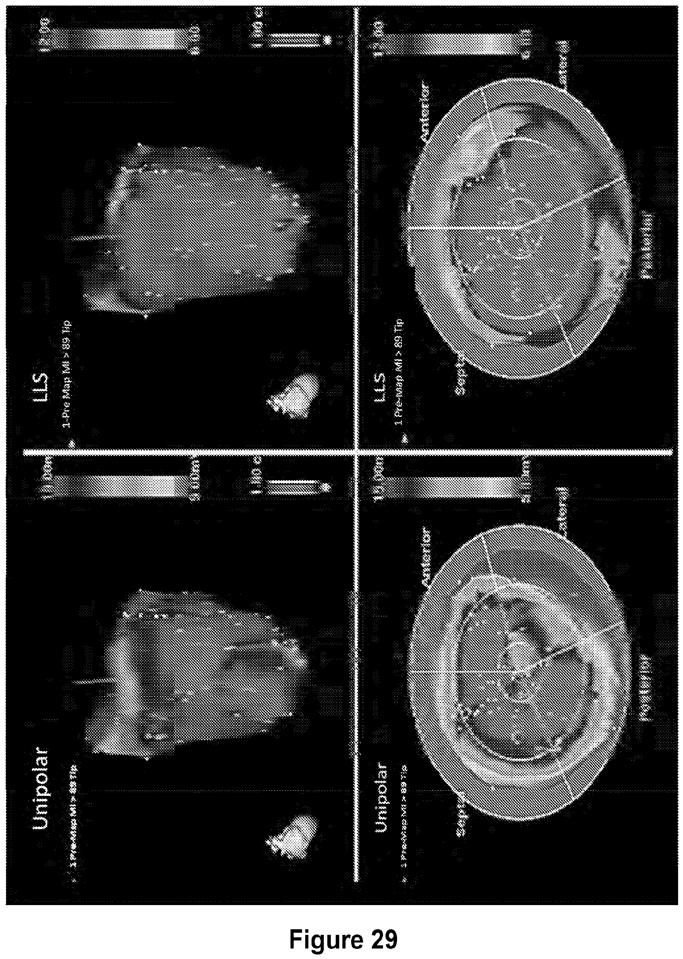

The next set of experiments was conducted to determine the therapeutic properties of CBSCs. Induction of ischemia-reperfusion myocardial infarction was used as a model. Fluoroscopic images of the coronary vasculature and balloon angioplasty placement within the left anterior descending coronary artery is shown in Figure 28. Baseline electromechanical assessment was performed on the ischemia-reperfusion myocardial infarction model. Figure 29 shows a Unipolar Map (left side panels) assessment of the electrical conduction across the catheter tip when contacted with the endocardial surface. Roughly, 80-100 discrete points are acquired to render a three dimensional left ventricular map which allows for visualization of the electrical conductance of the LV cardiac tissue. Tissue under 5 mV is considered to be none viable. LLS (Local Linear Shortening) Map (right side panels) takes the same 80-100 discrete points and uses a mathematical algorithm to compute the change in distance between multiple points within the same region of the LV. A colormetric scale is assigned to the tissue which is moving well, and tissue which has less movement through contraction and relaxation. Here, the baseline electromechanical assessment of the LV is visualized using both maps in a three dimensional view (above) and regional view (below).

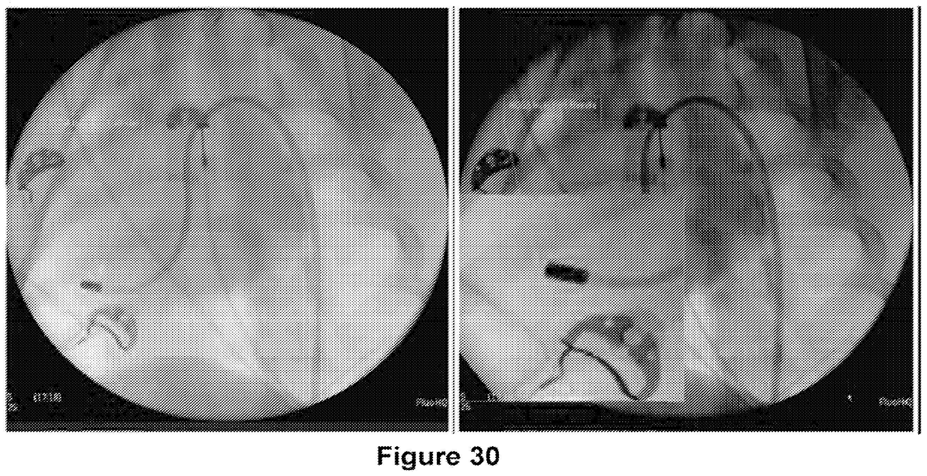

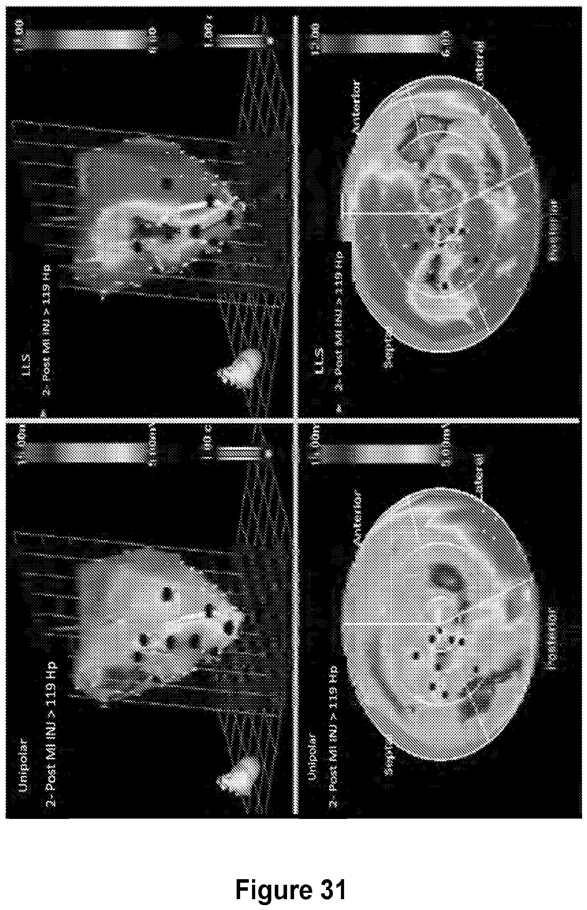

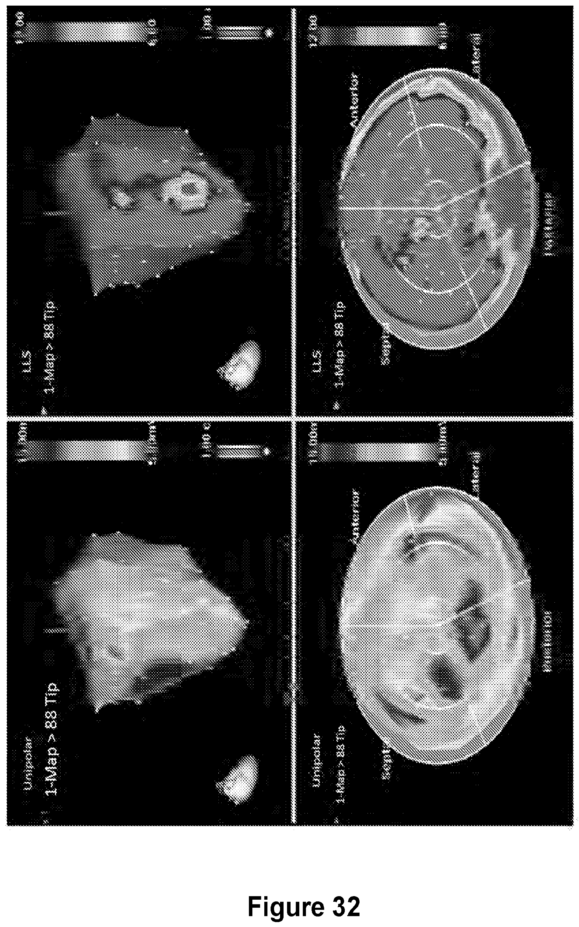

CBSCs were injected post-MI with the NOGA Myostar Injection Catheter. Fluoroscopic images of the NOGA Myostar injection catheter and magnification of the injection needle within the left ventricular myocardium during an injection of CBSC's is shown in Figure 30. Changes in both the Unipolar and LLS Maps were observed after 90 minutes of ischemia and (at least) 30 minutes of reperfusion. The black dotes denotes the areas where injection of swine CBSC's occurred. Injection of CBSC's was done using the NOGA Myostar Injection catheter, which allows for the ability to visualize the exact location of the injections. These are intramyocardial injection, of roughly two million cells per injection. Regions of the LV myocardium where poor conductance but sufficient wall motion to be the border zone region of the LV is outlined (Figure 31). An electromechanical assessment for one month post MI +CBSC injection was assessed. Preservation/restoration of both conductance (unipolar) and wall motion (LLS) was observed 1 month after injection with CBSCs (Figure 32).

Figure 10 demonstrates a cross sectional-view of the gross mid-myocardium from an animal who has received CBSC therapy versus control MI. The left image shows very small infarcts on the epicardial surface of the heart one month after MI + CBSC injections. The right image illustrates the scar formation and cardiac remodeling which occurs when no cell therapy is given post MI. The scar becomes transmural across the epi- to endocardial surface. These data correlate with functional changes. The results presented herein demonstrate the therapeutic potential of CBSC's

## Claims

1. An isolated cortical bone-derived stem cell (CBSC) capable of differentiating into a cardiomyocyte, wherein the cell expresses CD29, Sca-1, CD105, CD44, CD106, CD73, CD271, and CD90.

2. The cell of claim 1, where the cell does not express a hematopoietic biomarker protein or a nucleic acid encoding the biomarker protein.

3. The cell of claim 2, wherein the hematopoietic biomarker is selected from the group consisting of CD34, CD45, CD5, CD11b, CD45R, antigen 7-4, Gr-1, Ly6G/C, Terr-119, and any combination thereof.

4. The cell of claim 1, wherein the cell is pluripotent.

5. The cell of claim 4, wherein the cell retains the ability to differentiate into a germ layer selected from the group consisting of mesoderm, ectoderm, endoderm, and any combination thereof.

6. The cell of claim 1, wherein the cell differentiates into cardiomyocytes and secretes a pro-angiogenic paracrine factor when the cells are administered to the heart.

7. A cortical bone derived stem cell (CBSC) for use in the treatment of a cardiovascular disease in a subject, the use comprising administering to a subject in need thereof an effective amount of CBSCs, wherein the cell expresses CD29, Sca-1, CD105, CD44, CD106, CD73, CD271, and CD90.

8. The CBSC for use in the treatment of a cardiovascular disease in a subject according to claim 7, wherein the cardiovascular disease is myocardial injury, wherein the myocardial injury preferably comprises at least one of arterial disease, atheroma, atherosclerosis, arteriosclerosis, coronary artery disease, arrhythmia, angina pectoris, congestive heart disease, ischemic cardiomyopathy, myocardial infarction, stroke, transient ischemic attack, aortic aneurysm, cardiopericarditis, infection, inflammation, valvular insufficiency, vascular clotting defects, and combinations thereof.

9. The CBSC for use in the treatment of a cardiovascular disease in a subject according to claim 7, wherein the CBSCs are treated prior to administration to the subject, or wherein the CBSCs are treated during or after administration to the subject.

10. The CBSC for use in the treatment of a cardiovascular disease in a subject according to claim 7, wherein the CBSCs are administered to the subject by at least one of direct injection, venous infusion, and arterial infusion, or wherein the CBSCs differentiate into cardiac myocytes in the subject.

11. The CBSC for use in the treatment of a cardiovascular disease in a subject according to claim 7, wherein the CBSCs secrete a pro-angiogenic paracrine factor in the subject, wherein the pro-angiogenic paracrine factor is preferably selected from the group consisting of bFGF, VEGF, and a combination thereof.

12. The CBSC for use in the treatment of a cardiovascular disease in a subject according to claim 7, wherein the use further comprises modifying the CBSCs by gene transfer, or wherein the subject is a human.

13. An *in vitro* method of obtaining a population of cortical bone-derived stem cells (CBSCs), the method comprising the step of: obtaining cells from a cortical bone tissue sample; expanding the cells obtained from the cortical bone tissue sample to form a substantially pure population of CBSCs, wherein the cells of the population of CBSCs express CD29, Sca-1, CD105, CD44, CD106, CD73, CD271, and CD90.

## Patentansprüche

1. Isolierte, aus kortikalem Knochen gewonnene Stammzelle (CBSC), die in eine Kardiomyocyte differenzieren kann, wobei die Zelle CD29, Sca-1, CD105, CD44, CD106, CD73, CD271 und CD90 exprimiert.

2. Zelle nach Anspruch 1, wobei die Zelle kein hämatopoetisches Biomarkerprotein oder eine Nukleinsäure, die das Biomarkerprotein kodiert, exprimiert.

3. Zelle nach Anspruch 2, wobei der hämatopoetische Biomarker ausgewählt ist aus der Gruppe, bestehend aus CD34, CD45, CD5, CD11b, CD45R, Antigen 7-4, Gr-1, Ly6G/C, Terr-119 und einer Kombination daraus.

4. Zelle nach Anspruch 1, wobei die Zelle pluripotent ist.

5. Zelle nach Anspruch 4, wobei die Zelle die Fähigkeit behält, in ein Keimblatt zu differenzieren, ausgewählt aus der Gruppe, bestehend aus Mesoderm, Ektoderm, Endoderm und einer Kombination daraus.

6. Zelle nach Anspruch 1, wobei die Zelle in Kardiomyocyten differenziert und einen pro-angiogenetischen parakrinen Faktor sekretiert, wenn die Zellen dem Herzen verabreicht werden.

7. Aus kortikalem Knochen gewonnene Stammzelle (CBSC) zur Verwendung bei der Behandlung einer kardiovaskulären Erkrankung eines Patienten, wobei die Verwendung das Verabreichen einer wirksamen Menge von CBSCs an einen behandlungsbedürftigen Patienten umfasst, wobei die Zelle CD29, Sca-1, CD105, CD44, CD106, CD73, CD271 und CD90 exprimiert.

8. CBSC zur Verwendung bei der Behandlung einer kardiovaskulären Erkrankung eines Patienten nach Anspruch 7, wobei die kardiovaskuläre Erkrankung eine Myokardschädigung ist, wobei die Myokardschädigung vorzugsweise wenigstens eine von Folgenden umfasst: arterielle Erkrankungen, Atherom, Atherosklerose, Arteriosklerose, koronare Herzkrankheit, Arrhythmie, Angina pectoris, kongestive Herzinsuffizienz, ischämische Kardiomyopathie, Myokardinfarkt, Schlaganfall, transitorische ischämische Attacke, Aortenaneurysma, Kardioperikarditis, Infektion, Entzündung, Klappeninsuffizienz, vaskuläre Blutgerinnungsstörungen und Kombinationen davon.

9. CBSC zur Verwendung bei der Behandlung einer kardiovaskulären Erkrankung eines Patienten nach Anspruch 7, wobei die CBSCs vor der Verabreichung an den Patienten behandelt sind, oder wobei die CBSCs während oder nach der Verabreichung an den Patienten behandelt werden.

10. CBSC zur Verwendung bei der Behandlung einer kardiovaskulären Erkrankung eines Patienten nach Anspruch 7, wobei die CBSCs durch wenigstens eine der Folgenden: direkte Injektion, intravenöse Infusion und intraarterielle Infusion an den Patienten verabreicht werden, oder wobei die CBSCs bei dem Patienten in Herzmuskelzellen differenzieren.

11. CBSC zur Verwendung bei der Behandlung einer kardiovaskulären Erkrankung eines Patienten nach Anspruch 7, wobei die CBSCs bei dem Patienten einen pro-angiogenetischen parakrinen Faktor sekretieren, wobei der proangiogenetische parakrine Faktor vorzugsweise ausgewählt ist aus der Gruppe, bestehend aus bFGF, VEGF und einer Kombination daraus.

12. CBSC zur Verwendung bei der Behandlung einer kardiovaskulären Erkrankung eines Patienten nach Anspruch 7, wobei die Verwendung weiterhin das Modifizieren der CBSCs durch Gentransfer umfasst, oder wobei der Patient ein Mensch ist.

13. In vitro-Verfahren zur Gewinnung einer Population von aus kortikalem Knochen gewonnenen Stammzellen (CBSCs), wobei das Verfahren die folgenden Schritte umfasst: Gewinnen der Zellen aus einer Gewebeprobe eines kortikalen Knochens; Expandieren der aus der Gewebeprobe eines kortikalen Knochen gewonnen Zellen, um eine im Wesentlichen reine Population von CBSCs zu bilden, wobei die Zellen der Population von CBSCs: CD29, Sca-1, CD105, CD44, CD106, CD73, CD271 und CD90 exprimieren.

## Revendications

1. Cellule souche isolée dérivée d'os cortical (CBSC) capable de différencier dans un cardiomyocyte, dans laquelle la cellule exprime la CD29, le Sca-1, la CD105, la CD44, la CD106, le CD73, le CD271 et la CD90.

2. Cellule selon la revendication 1, dans laquelle la cellule n'exprime pas une protéine biomarqueur hématopoiétique ou un acide nucléique codant la protéine biomarqueur.

3. Cellule selon la revendication 2, dans laquelle le biomarqueur hématopoiétique est choisi parmi le groupe constitué de CD34, CD45, CD5, CD11b, CD45R, l'antigène 7-4, Gr-1, Ly6G/C, Terr-119 et une combinaison de ceux-ci.

4. Cellule selon la revendication 1, dans laquelle la cellule est pluripotente.

5. Cellule selon la revendication 4, dans laquelle la cellule conserve la capacité de différencier dans une cellule germinale choisie parmi le groupe constitué du mésoderme, d'ectoderme, d'endoderme, et une combinaison de ceux-ci.

6. Cellule selon la revendication 1, dans laquelle la cellule différencie dans des cardiomyocytes et sécrète un facteur paracrine pro-angiogénique lorsque les cellules sont administrées au cœur.

7. Cellule souche dérivée d'os cortical (CBSC) pour l'utilisation dans le traitement d'une maladie cardiovasculaire chez un sujet, l'utilisation comportant l'administration à un sujet en ayant besoin d'une quantité efficace de CBSCs, dans laquelle la cellule exprime la CD29, le Sca-1, la CD105, la CD44, la CD106, le CD73, le CD271 et la CD90.

8. CBSC pour l'utilisation dans le traitement d'une maladie cardiovasculaire chez un sujet selon la revendication 7, dans laquelle la maladie cardiovasculaire est une lésion myocardique, dans laquelle la lésion myocardique de préférence comporte au moins une parmi une maladie artérielle, un athérome, une athérosclérose, une artériosclérose, une maladie coronarienne, une arythmie, une angine de poitrine, une insuffisance cardiaque congestive, une cardiomyopathie ischémique, un infarctus du myocarde, une accident vasculaire cérébral, une accident ischémique transitoire, un anévrisme de l'aorte, une péricardite cardiaque, une infection, une inflammation, une insuffisance valvulaire, des défauts de coagulation vasculaires, et des combinaisons de ceux-ci.

9. CBSC pour l'utilisation dans le traitement d'une maladie cardiovasculaire chez un sujet selon la revendication 7, dans laquelle les CBSCs sont traitées avant l'administration au sujet ou dans laquelle les CBSCs sont traitées pendant ou après l'administration au sujet.

10. CBSC pour l'utilisation dans le traitement d'une maladie cardiovasculaire chez un sujet selon la revendication 7, dans laquelle les CBSCs sont administrées au sujet par au moins une parmi l'injection directe, la perfusion veineuse et la perfusion artérielle ou dans laquelle les CBSCs différencient dans des myocytes cardiaques chez le sujet.

11. CBSC pour l'utilisation dans le traitement d'une maladie cardiovasculaire chez un sujet selon la revendication 7, dans laquelle les CBSCs sécrètent un facteur paracrine pro-angiogénique dans le sujet, dans laquelle le facteur paracrine pro-angiogénique de préférence est choisi parmi le groupe constitué de bFGF, VEGF et une combinaison de ceux-ci.

12. CBSC pour l'utilisation dans le traitement d'une maladie cardiovasculaire chez un sujet selon la revendication 7, dans laquelle l'utilisation comporte en outre la modification des CBSCs par un transfert génique ou dans laquelle le sujet est un homme.

13. Procédé *in vitro* pour obtenir une population des cellules souches dérivées d'os cortical (CBSCs), dans lequel le procédé comporte les étapes suivantes: obtenir des cellules à partir d'un échantillon de tissu d'os cortical; l'expansion des cellules obtenues de l'échantillon de tissu d'os cortical pour former une population de CBSCs sensiblement pure, dans laquelle les cellules de la population de CBSCs expriment la CD29, le Sca-1, la CD105, la CD44, la CD106, le CD73, le CD271 et la CD90.
